# EUROPÄISCHE PATENTSCHRIFT

(11) **EP 3 174 977 B1**
(45) Veröffentlichungstag und Bekanntmachung des Hinweises auf die Patenterteilung: **10.04.2019**
(21) Anmeldenummer: 15741571.2
(22) Anmeldetag: 28.07.2015
(51) Int. Cl.: C12N 9/04, C12P 33/02

(54) **7BETA-HYDROXYSTEROID DEHYDROGENASE-MUTANTEN UND VERFAHREN ZUR HERSTELLUNG VON URSODESOXYCHOLSÄURE**
7BETA-HYDROXYSTEROID DEHYDROGENASE MUTANTS AND METHOD FOR PRODUCING URSODEOXYCHOLIC ACID
7BETA-HYDROXYSTÉROÏDES DÉSHYDROGÉNASES MUTANTS ET PROCÉDÉ DE FABRICATION D'ACIDE URSODÉSOXYCHOLIQUE

(30) Priorität: 29.07.2014 EP 14178912
(43) Veröffentlichungstag der Anmeldung: 07.06.2017
(60) Teilanmeldung: 18196616.9
(73) Patentinhaber: PharmaZell GmbH, 83064 Raubling (DE)
(72) Erfinder: HUMMEL, Werner, 52445 Titz (DE); BAKONYI, Daniel, 50969 Köln (DE)
(74) Vertreter: Reitstötter Kinzebach
(86) Internationale Anmeldenummer: PCT/EP2015/067212
(87) Internationale Veröffentlichungsnummer: WO 2016/016213

(56) Entgegenhaltungen:
- WO-A1-2011/064404
- WO-A1-2012/080504
- J.-Y. LEE ET AL: "Contribution of the 7 -hydroxysteroid dehydrogenase from Ruminococcus gnavus N53 to ursodeoxycholic acid formation in the human colon", THE JOURNAL OF LIPID RESEARCH, Bd. 54, Nr. 11, 1. Juni 2013 (2013-06-01), Seiten 3062-3069, XP055174301, ISSN: 0022-2275, DOI: 10.1194/jlr.M039834
- DATABASE UniProt [Online] 18. September 2013 (2013-09-18), "SubName: Full=7-beta-hydroxysteroid dehydrogenase {ECO:0000313|EMBL:AGN52919.1}; EC=1.1.1.201 {ECO:0000313|EMBL:AGN52919.1};", XP002745816, gefunden im EBI accession no. UNIPROT:R9UAM1 Database accession no. R9UAM1
- DATABASE UniProt [Online] 21. September 2011 (2011-09-21), "SubName: Full=Uncharacterized protein {ECO:0000313|EMBL:EGN47897.1};", XP002745817, gefunden im EBI accession no. UNIPROT:F7JXB4 Database accession no. F7JXB4
- DATABASE UniProt [Online] 22. Februar 2012 (2012-02-22), "SubName: Full=7-beta-hydroxysteroid dehydrogenase {ECO:0000313|EMBL:AET80684.1};", XP002745818, gefunden im EBI accession no. UNIPROT:G9FRD6 Database accession no. G9FRD6
- DATABASE UniProt [Online] 22. Januar 2014 (2014-01-22), "SubName: Full=Putative oxidoreductase {ECO:0000313|EMBL:GAD88695.1};", XP002745819, gefunden im EBI accession no. UNIPROT:V5HGY6 Database accession no. V5HGY6
- DATABASE UniProt [Online] 14. Dezember 2011 (2011-12-14), "SubName: Full=Short-chain dehydrogenase/reductase SDR {ECO:0000313|EMBL:EHB54992.1};", XP002745820, gefunden im EBI accession no. UNIPROT:G4HWC6 Database accession no. G4HWC6
- ERICA ELISA FERRANDI ET AL: "In search of sustainable chemical processes: cloning, recombinant expression, and functional characterization of the 7Î+-- and 7Î-hydroxysteroid dehydrogenases from", APPLIED MICROBIOLOGY AND BIOTECHNOLOGY, SPRINGER, BERLIN, DE, Bd. 95, Nr. 5, 25. Dezember 2011 (2011-12-25), Seiten 1221-1233, XP035098198, ISSN: 1432-0614, DOI: 10.1007/S00253-011-3798-X

## Beschreibung

Die Erfindung betrifft neuartige 7β-Hydroxysteroid Dehydrogenase-Mutanten, die für diese Enzym-Mutanten kodierenden Sequenzen, Verfahren zur Herstellung der Enzym-Mutanten und deren Verwendung bei enzymatischen Umsetzungen von Cholsäureverbindungen, und insbesondere bei der Herstellung von Ursodesoxycholsäure (UDCS); Gegenstand der Erfindung sind auch neuartige Verfahren zur Synthese von UDCS unter Verwendung der Enzym-Mutanten; sowie die Herstellung von UDCS unter Verwendung rekombinanter, mehrfach modifizierter Mikroorganismen.

### Hintergrund der Erfindung:

Gallensäuren sind Biomoleküle, die für die Verdauung und Absorption von Fetten, Fettsäuren und hydrophoben Vitaminen erforderlich sind. Eine Gallensäure, die beim Menschen nur in geringen Anteilen vorkommt, ist Ursodesoxycholsäure (UDCA). Sie hat mittlerweile große therapeutische Bedeutung bei der Auflösung von Cholesterin-haltigen Gallensteinen erlangt. Industriell wird diese Verbindung durch chemische oder enzymatische Schritte im Tonnenmaßstab hergestellt. Eine wichtige Vorstufe für die Synthese von UDCA ist 12-Ketoursodesoxycholsäure, die durch eine Wolff-Kishner-Reduktion in UDCA umgewandelt werden kann. Eine literaturbeschriebene Route zur Synthese von 12-Ketoursodesoxycholsäure startet von Cholsäure (3α,7α,12α-Trihydroxy-5β-cholansäure), die durch zwei oxidative Schritte, die durch 7α- und 12α-HSDHs katalysiert werden, und einen reduktiven Schritt, katalysiert durch eine 7β-HSDH, hergestellt werden kann (Bovara R et al. (1996) A new enzymatic route to the synthesis of 12-ketoursodeoxycholic acid. Biotechnol. Lett. 18:305-308; Monti D et al. (2009) One-pot multienzymatic synthesis of 12-ketoursodeoxycholic acid: Subtle cofactor specificities rule the reaction equilibria of five biocatalysts working in a row. Adv. Synth. Catal. 351:1303-1311). Eine weitere Route startet von 7-Ketolithocholsäure, die durch stereoselektive Reduktion der 7-Keto-Gruppe zu UDCA umgesetzt werden kann, auch dieser Schritt wird vorteilhafterweise enzymatisch katalysiert durchgeführt, katalysiert durch eine 7β-HSDH (Higashi S et al. (1979) Conversion of 7-ketolithocholic acid to ursodeoxycholic acid by human intestinal anaerobic microorganisms: Interchangeability of chenodeoxycholic acid and ursodeoxycholic acid. Gastroenterologia Japonica 14:417-424; Liu L et al. (2011) Identification, cloning, heterologous expression, and characterization of a NADPH-dependent 7 beta-hydroxysteroid dehydrogenase from Collinsella aerofaciens. Appl. Microbiol. Biotechnol. 90:127-135.). Eine weitere günstige Syntheseroute geht von Dehydrocholsäure (DHCA) aus, die durch zwei reduktive Schritte zu 12-Ketoursodesoxycholsäure umgesetzt werden kann, diese beiden Schritte können durch zwei stereoselektive HSDHs (3α- and 7β-HSDHs) katalysiert werden (Carrea G et al. (1992) Enzymatic synthesis of 12-ketoursodeoxycholic acid from dehydrocholic acid in a membrane reactor. Biotechnol. Lett. 14:1131-1135; Liu L et al. (2013) One-step synthesis of 12-ketoursodeoxycholic acid from dehydrocholic acid using a multienzymatic system. Appl. Microbiol. Biotechnol. 97:633-639).

Als 7β-HSDH hat sich das Enzym aus *C. aerofaciens* als gut geeignet erwiesen. Mittlerweile ist die Gensequenz dieses Enzyms aus *C. aerofaciens* bekannt, so dass zum einen das Enzym nach Klonierung rekombinant verfügbar gemacht werden kann, zum anderen besteht die Möglichkeit, mit Methoden des Proteinengineering Mutanten dieses Enzyms zu erzeugen und damit gegebenenfalls vorteilhaftere Enzymvarianten aufzufinden.

Die WO 2011/064404 beschreibt neuartige 7β-Hydroxysteroid Dehydrogenasen aus *Collinsella aerofaciens,* für diese Enzyme kodierenden Sequenzen, sowie Verfahren zur Herstellung der Enzyme und deren Verwendung bei enzymatischen Umsetzungen von Cholsäureverbindungen, wie z.B. für die Herstellung von UDCA.

Zur medikamentösen Behandlung von Gallensteinleiden werden seit vielen Jahren u. a. die Wirkstoffe Ursodesoxycholsäure (UDCS oder UDCA) und das zugehörige Diastereomer Chenodesoxycholsäure (CDCS oder CDCA) eingesetzt. Beide Verbindungen unterscheiden sich lediglich durch die Konfiguration der Hydroxygruppe am C-Atom 7 (UDCS: β-Konfiguration, CDCS: α-Konfiguration). Zur Herstellung von UDCS sind im Stand der Technik verschieden Verfahren beschrieben, welche rein chemisch durchgeführt werden oder aus einer Kombination chemischer und enzymatischer Verfahrensschritte bestehen. Ausgangspunkt ist jeweils Cholsäure (CS oder CA) oder die aus Cholsäure hergestellte CDCS.

So kann die klassisch chemische Methode zur UDCS Herstellung wie folgt schematisch dargestellt werden:

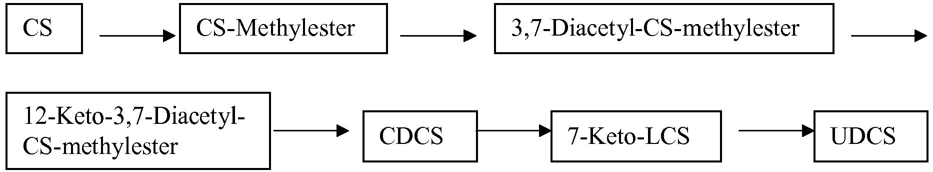

Ein gravierender Nachteil ist unter anderem folgender: da die chemische Oxidation nicht selektiv ist, müssen die Carboxy-Gruppe und die 3α und 7α-Hydroxy-Gruppe durch Veresterung geschützt werden.

Ein alternatives chemisch/enzymatisches Verfahren basierend auf der Verwendung des Enzyms 12α-Hydroxysteroid Dehydrogenase (12α-HSDH) kann wie folgt dargestellt werden und ist z.B. beschrieben in der PCT/EP2009/002190 der vorliegenden Anmelderin.

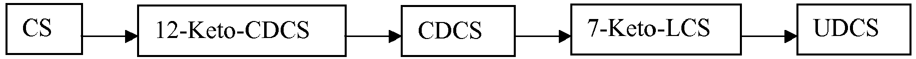

Die 12α-HSDH oxidiert dabei CS selektiv zu 12-Keto-CDCS. Die zwei nach der klassisch chemischen Methode erforderlichen Schützschritte entfallen dabei.

Weiterhin wird von Monti, D., et al., (One-Pot Multienzymatic Synthesis of 12-Ketoursodeoxycholic Acid: Subtle Cofactor Specificities Rule the Reaction Equilibria of Five Biocatalysts Working in a Row. Advanced Synthesis & Catalysis, 2009) ein alternatives enzymatisch-chemisches Verfahren beschrieben, das wie folgt schematisch darstellbar ist:

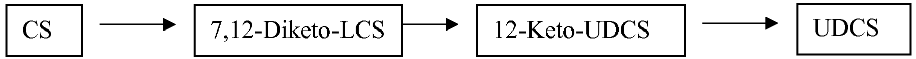

Die CS wird zuerst von 7α-HSDH aus *Bacteroides fragilis* ATCC 25285 (Zhu, D., et al., Enzymatic enantioselective reduction of -ketoesters by a thermostable 7 -hydroxysteroid dehydrogenase from Bacteroides fragilis. Tetrahedron, 2006. 62(18): p. 4535-4539) und 12α-HSDH zu 7,12-Diketo-LCS oxidiert. Diese beiden Enzyme sind jeweils NADH abhängig. Nach der Reduktion durch 7β-HSDH (NADPH abhängig) aus *Clostridium absonum* ATCC 27555 (DSM 599) (MacDonald, I.A. and P.D. Roach, Bile induction of 7 alpha- and 7 beta-hydroxysteroid dehydrogenases in Clostridium absonum. Biochim Biophys Acta, 1981. 665(2): p. 262-9) entsteht 12-Keto-UDCS. Durch Wolff-Kishner-Reduktion wird das Endprodukt erhalten. Nachteilig bei diesem Verfahren ist, dass wegen der Gleichgewichtslage der katalysieren Reaktion eine komplette Umsetzung nicht möglich ist, und dass für die erste Stufe der Umsetzung zwei unterschiedliche Enzyme eingesetzt werden müssen, was das Verfahren verteuert. Zur Kofaktor-Regenerierung werden Lactat Dehydrogenase (LDH; zur Regenerierung von NAD⁺) und Glucose Dehydrogenase (GlcDH oder GDH, zur Regenerierung von NADPH) eingesetzt. Nachteilig bei der dort verwendeten Kofaktor-Regenerierung ist, dass das entstehende Ko-Produkt nur sehr schwer aus dem Reaktionsgemisch zu entfernen ist, so dass das Reaktionsgleichgewicht nicht positiv beeinflusst werden kann, was eine unvollständige Umsetzung des Edukts bedingt.

Eine 7β-HSDH aus Stamm *Collinsella aerofaciens* ATCC 25986 (DSM 3979; ehemalige *Eubacterium aerofaciens*) wurde im Jahr 1982 von Hirano und Masuda beschrieben (Hirano, S. and N. Masuda, Characterization of NADP-dependent 7 beta-hydroxysteroid dehydrogenases from Peptostreptococcus productus and Eubacterium aerofaciens. Appl Environ Microbiol, 1982. 43(5): p. 1057-63). Sequenzinformationen wurden zu diesem Enzym nicht offenbart. Das durch Gelfiltration bestimmte Molekulargewicht betrug 45,000 Da (vgl. Hirano, Seite 1059, linke Spalte). Weiterhin konnte für das dortige Enzym die Reduktion der 7-Oxo-Gruppe zur 7β-Hydroxygruppe nicht beobachtet werden (vgl. Hirano, Seite 1061, Diskussion 1. Absatz). Der Fachmann erkennt somit, dass das von Hirano *et al.* beschriebene Enzym nicht zur Katalyse der Reduktion von Dehydrocholsäure (DHCS oder DHCA) in 7-Position zu 3,12-Diketo-7β-CS geeignet ist.

In der älteren internationalen Patentanmeldung PCT/EP2010/068576 der Anmelderin wird eine neuartige 7β-HSDH aus *Collinsella aerofaciens* ATCC 25986 beschrieben, welche unter anderem ein Molekulargewicht (bei SDS-Gelelektrophorese) von etwa 28-32 kDa, ein Molekulargewicht (bei Gelfiltration, unter nicht denaturierenden Bedingungen, wie insbesondere ohne SDS): von etwa 53 bis 60 kDa, und die Befähigung zur stereoselektiven Reduktion der 7-Carbonyl-Gruppe von 7-Keto-LCS in eine 7β-Hydroxy-Gruppe aufweist.

Außerdem wird in der PCT/EP2010/068576 ein Verfahren zur UDCS Herstellung bereitgestellt, welches schematisch wie folgt darstellbar ist:

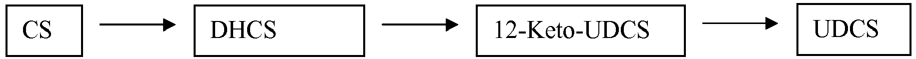

Dabei erfolgt die Oxidation von CS auf klassisch chemischem Weg in einfacher Weise. Die DHCS wird von Enzympaar 7β-HSDH und 3α-HSDH einzeln nacheinander oder in einem Topf zu 12-Keto-UDCS reduziert. Kombiniert mit der Wolff-Kishner-Reduktion kann UDCS somit in nur drei Schritten aus CS synthetisiert werden. Während die 7β-HSDH vom Kofaktor NADPH abhängig ist, benötigt die 3α-HSDH den Kofaktor NADH. Die Verfügbarkeit von Enzympaaren mit Abhängigkeit vom gleichen Kofaktor oder erweiterter Anhängigkeit, (z.B. von den Kofaktoren NADH und NADPH) wäre von Vorteil, weil damit die Kofaktor-Regenerierung vereinfacht werden könnte.

Die WO 2012/080504 beschreibt neuartige Mutanten der 7β-HSDH aus *C. aerofaciens* im Sequenzbereich der Aminosäurereste 36 bis 42 der *C. aerofaciens* Sequenz, sowie biokatalytische Verfahren zur Herstellung von UDCS, und insbesondere auch neuartige Ganzzell-Verfahren.

Die WO 2011/147957 beschreibt neuartige knock-out Stämme, die zur Herstellung von UDCS besonders geeignet sind, da die unerwünschte 7alpha -HSDH Enzymaktivität gezielt ausgeschalten werden konnte.

Aufgabe der Erfindung ist die Bereitstellung weiter verbesserter 7β-HSDHs. Insbesondere sollten Enzym-Mutanten bereitgestellt werden, welche noch vorteilhafter zur enzymatischen oder mikrobiellen Herstellung von UDCS über die stereospezifische Reduktion von DHCS in 7-Position zu 3,12-Diketo-7β-CS eingesetzt werden können, und insbesondere eine verbesserte Aktivität für Substrat und/oder Kofaktor, und/oder eine geringere Substratinhibition und/oder veränderte Kofaktor-Nutzung (erhöhte, veränderte Spezifität oder erweiterte Abhängigkeit) aufweisen.

### Kurzfassung der Erfindung:

Obige Aufgaben konnten überraschenderweise gelöst werden durch die Erzeugung und Charakterisierung von verbesserten Mutanten der 7β-HSDH aus aeroben Bakterien der Gattung *Collinsella,* insbesondere des Stammes *Collinsella aerofaciens* und deren Einsatz bei der Umsetzung von Cholsäureverbindungen, insbesondere bei der Herstellung von UDCS.

Mittlerweile ist die Gensequenz dieses Enzyms aus *C. aerofaciens* bekannt, so dass zum einen das Enzym nach Klonierung rekombinant verfügbar gemacht werden kann, und zum anderen die Möglichkeit besteht, mit Methoden des Proteinengineering Mutanten dieses Enzyms zu erzeugen und damit ggf. vorteilhaftere Enzymvarianten aufzufinden.

Auf Grund von Struktur- und Homologiebetrachtungen wurde erfindungsgemäß versucht, Sequenzbereiche zu definieren, die für die Coenzymbindung oder auch die Substraterkennung verantwortlich sein könnten. Damit ergeben sich Möglichkeiten, in diesen Bereichen durch Mutagenese gezielt Aminosäuren zu verändern, um durch diese StrukturVeränderungen Enzymeigenschaften zu verändern. So liegt bei der 7β-HSDH aus *C. aerofaciens* im Bereich der Aminosäuren um ca. 10 bis 64 der sog. "Rossmann-Fold", der für die Coenzymbindung zuständig ist. Erfindungsgemäß wurde nun insbesondere versucht, Aminosäuren in diesem Bereich der Coenzymbindung derart zu verändern, dass das Enzym statt NADPH das kostengünstigere NADH akzeptiert. Beim Versuch, die Aminosäure Arginin in Position 64 durch Asparaginsäure zu ersetzen, wurde dabei überraschenderweise gefunden, dass diese 7β-HSDH-Mutante eine deutlich höhere Aktivität aufweist. Das hat sich in der Folge auch bestätigt, als mehrere weitere Mutanten, die alle in Position 64 Mutationen aufwiesen, höhere Aktivitäten als das Wildtyp-Enzym zeigten. Auch Enzym-Mutanten, die bis zur Homogenität aufgereinigt und mit dem entsprechend gereinigten Wildtyp-Enzym verglichen wurden, zeigten überraschenderweise deutlich höhere spezifische Enzymaktivitäten.

Besonders eindeutig ist die Verbesserung der Aktivität an der Erhöhung der spezifischen Aktivität, also der auf die Proteinmenge bezogene Aktivitätswert, bei den Mutanten 7β-HSDH-R64E, sowie 7β-HSDH-R64D und 7β-HSDH-R64T zu erkennen. Die Schreibweise 7β-HSDH-R64E bedeutet, dass bei der betrachteten 7β-HSDH das Arginin (R) in Position 64 der Proteinsequenz durch Glutaminsäure (E) ersetzt wurde. Analog ist der Terminus 7β-HSDH-R64D, wo Arginin in Position 64 gegen Asparaginsäure (D) ausgetauscht wurde, zu verstehen. Als Wildtyp-Enzym wird die 7β-HSDH, die aus *C. aerofaciens* gewonnen werden kann, bezeichnet.

Weiterhin wurde obige Aufgabe gelöst durch Bereitstellung eines biokatalytischen (mikrobiellen bzw. enzymatischen) Prozesses, umfassend die enzymatische Umsetzung von DHCS über zwei durch die hierin beschriebenen 7β-HSDH-Mutanten bzw. 3α-HSDH katalysierte reduktive Teilschritte, die gleichzeitig oder zeitlich versetzt in beliebiger Reihenfolge ablaufen können, zu 12-Keto-UDCS und Kofaktorregenerierung durch Verwendung von Dehydrogenasen, welche den verbrauchen Kofaktor aus beiden reduktiven Teilschritten regenerieren.

### Figurenbeschreibung:

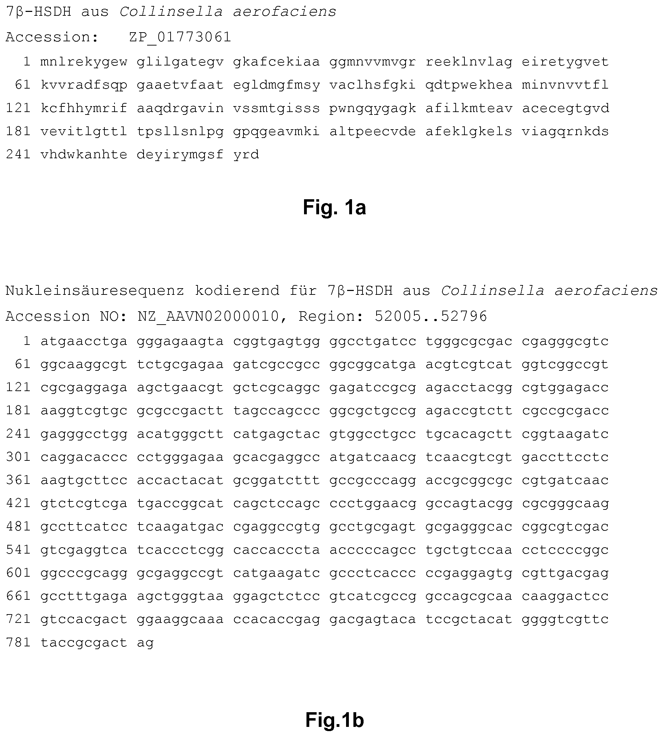

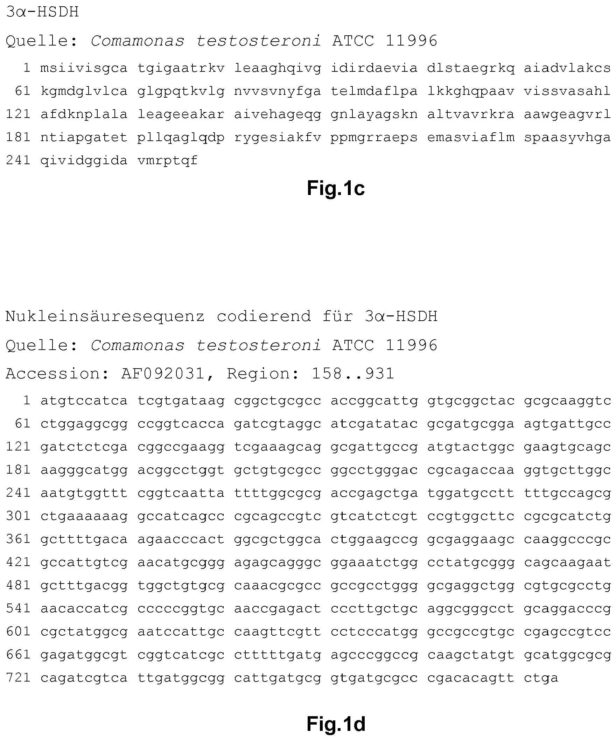

Figur 1a zeigt die Aminosäuresequenz der 7β-HSDH aus *Collinsella aerofaciens* (SEQ ID NO:2) und Figur 1b die kodierende Nukleinsäuresequenz zur Aminosäuresequenz von Figur 1a; Figur 1c zeigt die Aminosäuresequenz der 3α-HSDH aus *Comanomonas testosteroni* und Figur 1d die kodierende Nukleinsäuresequenz zur Aminosäuresequenz von Figur 1c;
Figur 2a zeigt die Aminosäuresequenz der 7β-HSDH aus *Collinsella aerofaciens;* C-terminal verlängert um die His-Tag Sequenz LEHHHHHH; Figur 2b die davon abgeleitete Mutante 7β-HSDH [R64E]; Figur 2c die davon abgeleitete Mutante 7β-HSDH [G39S]; Figur 2d die davon abgeleitete Mutante 7β-HSDH [G39S/R64E];
Figur 3 zeigt die Auftragung der spezifischen Enzymaktivität der Glutaminsäuremutante [R64E] für das Substrat DHCA (linkes Bild) und für das Coenzym NADPH (rechtes Bild).
Figur 4 zeigt die Auftragung der spezifischen Enzymaktivität der Serinmutante für das Substrat DHCA (linkes Bild) und für das Coenzym NADPH (rechtes Bild).
Figur 5 zeigt das: SDS-Gel der 7β-HSDH [G39S/R64E] Mutante nach Expression in Schüttelkolben-Fermentation in LB-Medium. Aufgetragen sind der zellfreie Rohextrakt und das Enzym nach Reinigung. Die 7β-HSDH-Mutante hat eine Größe von ca. 29,9 kDa. Aufgetragen wurden ca. 10 µg Protein
Figur 6 zeigt die Auftragung der spezifischen Enzymaktivität der Doppelmutante [G39S/R64E] für das Substrat DHCA (linkes Bild) und für das Coenzym NADPH (rechtes Bild).

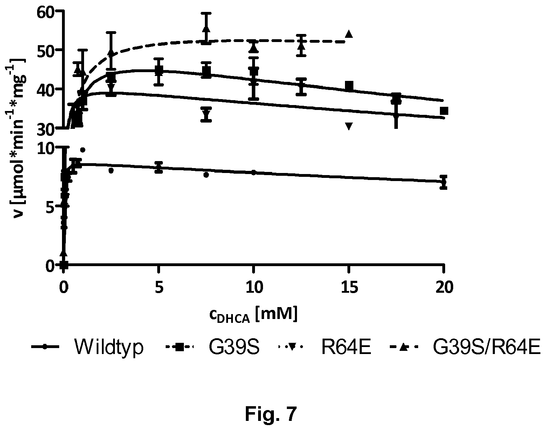

Figur 7 zeigt den Vergleich der Kinetiken einiger erfindungsgemäßer Mutanten (invertiertes Dreieck ( 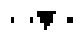 ): 7β-HSDH [R64E]; Quadrat ( 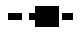 ) 7β-HSDH [G39S]; Dreieck ( 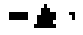 ) 7β-HSDH [G39S /R64E]) mit dem Wildtyp (Punkt ( 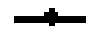 ).

### Spezielle Ausführungsformen der Erfindung

Die Erfindung betrifft insbesondere folgende spezielle Ausführungsformen:
1. 7β-Hydroxysteroiddehydrogenase (7β-HSDH), 7β-HSDH, welche wenigstens die stereospezifische enzymatische Reduktion eines 7-Ketosteroids zum korrespondierenden 7-Hydroxysteroid katalysiert, wobei das Enzym abgeleitet ist von einem Enzym mit der SEQ ID NO:2, oder einem diese Sequenz umfassenden Enzyms, wie z.B. einem Enzym umfassend die SEQ ID NO:3 (d.h. SEQ ID NO:2 N-terminal verlängert um einen Histidin-Tag oder Histidin-Anker-Sequenz), und wobei das Enzym eine Mutation in Position 64 von SEQ ID NO:2 (oder z.B. von SEQ ID NO:3) oder in den korrespondierenden Sequenzpositionen einer davon abgeleiteten Aminosäuresequenz mit wenigstens 90%, wie z. B. wenigstens 91, 92, 93, 94, 95, 96, 97, 98, 99 oder 99,5% Sequenzidentität zu SEQ ID NO:2 (oder z.B. von SEQ ID NO:3) umfasst,
   wobei die Mutation in Position 64 die Mutation R64X₁ ist,
   worin X₁ für E, D, T, L, S, P, V, K, C, A, G, Q, F, W, I, oder Y steht;
   und
   wobei das Enzym die im Vergleich zur 7β-HSDH mit SEQ ID NO:2 folgendes Eigenschaftsprofil zeigt:
   a) eine erhöhte spezifische Aktivität (Vmax [U/mg]) für NAD(P)H, insbesondere NADPH, bei der enzymatischen Reduktion von DHCA mit NAD(P)H, insbesondere NADPH, als Cofaktor; wobei z.B. die spezifische Aktivität (U/mg) in Gegenwart des Kofaktors NAD(P)H, insbesondere NADPH, im Vergleich zum nichtmutierten Enzym um wenigsten 1, 5 oder 10 % , insbesondere aber wenigstens um das 1-fache, insbesondere um das 2- bis 10-fache erhöht ist; und zusätzlich gegebenenfalls:
   b) eine erhöhte spezifische Aktivität (Vmax [U/mg]) für Dehydrocholsäure (DHCA) bei der enzymatischen Reduktion von DHCA mit NAD(P)H, insbesondere NADPH, als Cofaktor; wobei z.B. die spezifische Aktivität (U/mg) in Gegenwart des Kofaktors NAD(P)H, insbesondere NADPH, im Vergleich zum nichtmutierten Enzym um wenigsten 1, 5, 10, 50 oder 100 % , insbesondere aber wenigstens um das 1-fache, insbesondere um das 2- bis 100-fache oder 3- bis 20-fache oder 5- bis 10-fache erhöht ist;
   c) eine verringerte Substratinhibition durch DHCA, wie z.B. mit Ki-Werten im Bereich von >1 mM, wie z.B. bei 1 bis 200 mM, 2 bis 150 mM, 2,5 bis 100 mM;
   d) eine veränderte Cofaktorspezifität bezüglich NADH und NADPH, wie z.B. eine erweitere Spezifität, das heißt Nutzung eines zusätzlichen, bisher nicht genutzten Cofaktors, insbesondere NADPH;
   e) wobei diese Eigenschaften b), c) und d) einzeln oder in beliebiger Kombination vorliegen können.
2. 7β-Hydroxysteroiddehydrogenase (7β-HSDH), 7β-HSDH, welche wenigstens die stereospezifische enzymatische Reduktion eines 7-Ketosteroids zum korrespondierenden 7-Hydroxysteroid katalysiert, wobei das Enzym eine Mutation in Position 64 von SEQ ID NO:2 oder in den korrespondierenden Sequenzpositionen einer davon abgeleiteten Aminosäuresequenz mit wenigstens 80% Sequenzidentität zu SEQ ID NO:2, und zusätzlich wenigstens eine Mutation im Sequenzmotiv VMVGRRE gemäß Position 36 bis 42, insbesondere Position 39 von SEQ ID NO:2 oder in dem korrespondierenden Sequenzmotiv einer davon abgeleiteten Aminosäuresequenz mit wenigstens 80% Sequenzidentität, wie z.B. 85, 90, 91, 92, 93, 94, 95, 96, 97, 98, 99 oder 99,5% Sequenzidentität, zu SEQ ID NO:2 aufweist;
   wobei die Mutation in Position 64 die Mutation R64X₁ ist,
   worin X₁ für E, D, T, L, S, P, V, K, C, A, G, Q, F, W, I, oder Y steht;
   und
   wobei das Enzym die im Vergleich zur 7β-HSDH mit SEQ ID NO:2 (oder z.B. mit SEQ ID NO:3) folgendes Eigenschaftsprofil zeigt:
   a) eine erhöhte spezifische Aktivität (Vmax [U/mg]) für NAD(P)H, insbesondere NADPH, bei der enzymatischen Reduktion von DHCA mit NAD(P)H, insbesondere NADPH, als Cofaktor; wobei z.B. die spezifische Aktivität (U/mg) in Gegenwart des Kofaktors NAD(P)H, insbesondere NADPH, im Vergleich zum nichtmutierten Enzym um wenigsten 1, 5 oder 10 % , insbesondere aber wenigstens um das 1-fache, insbesondere um das 2- bis 10-fache erhöht ist; und zusätzlich gegebenenfalls:
   b) eine erhöhte spezifische Aktivität (Vmax [U/mg]) für Dehydrocholsäure (DHCA) bei der enzymatischen Reduktion von DHCA mit NAD(P)H, insbesondere NADPH, als Cofaktor; wobei z.B. die spezifische Aktivität (U/mg) in Gegenwart des Kofaktors NAD(P)H, insbesondere NADPH, im Vergleich zum nichtmutierten Enzym um wenigsten 1, 5, 10, 50 oder 100 % , insbesondere aber wenigstens um das 1-fache, insbesondere um das 2- bis 100-fache oder 3- bis 20-fache oder 5- bis 10-fache erhöht ist;
   c) eine verringerte Substratinhibition durch DHCA, wie z.B. mit Ki-Werten im Bereich von >1 mM, wie z.B. bei 1 bis 200 mM, 2 bis 150 mM, 2,5 bis 100 mM;
   d) eine veränderte Cofaktorspezifität bezüglich NADH und NADPH, wie z.B. eine erweitere Spezifität, das heißt Nutzung eines zusätzlichen, bisher nicht genutzten Cofaktors, insbesondere NADPH;
   e) wobei diese Eigenschaften b), c) und d) einzeln oder in beliebiger Kombination vorliegen können.
3. 7β-HSDH nach Ausführungsform 2, die zusätzlich die Aminosäuresequenz-Mutation G39X₃ aufweist,
   wobei X₃ für einen von Glycin (G) verschiedenen, insbesondere proteinogenen Aminosäurerest, insbesondere eine beliebige, spezifische Aktivität erhöhende und/oder die Substratinhibition verringernde und/oder die Kofaktornutzung oder Kofaktor-Abhängigkeit modifizierende, insbesondere natürliche Aminosäure steht.
4. 7β-HSDH nach einer der Ausführungsformen 2 und 3, ausgewählt unter den Doppelmutanten
   R64X₁/G39X₃
   worin
   X₁ für E, D, T, L, S, P, V, K, C, A, G, Q, F, W, I, Y, H oder N, insbesondere E, D, T, L, S, P oder V steht; und
   X₃ für S, A, V, I, L, C, K, Y F oder R, insbesondere S oder A, steht.
5. 7β-HSDH nach Ausführungsform 4, wobei die Doppelmutante ausgewählt ist unter: (G39S/R64E); (G39S/R64D); (G39S/R64T); (G39S/R64L); (G39S/R64S); (G39S/R64P); (G39S/R64V); (G39A/R64E); (G39A/R64D); (G39A/R64T); (G39A/R64S); (G39A/R64L); (G39A/R64P); (G39A/R64V);
   Obige Ausführungen zu Doppelmutanten gelten insbesondere für SEQ ID NO:2 und 3. Weitere spezielle Ausführungsformen betreffen Mutanten der 7β-HSDH mit SEQ ID NO:2 oder mit SEQ ID NO:3 oder einer davon abgeleiteten Sequenz mit einem Identitätsgrad von wenigsten 80% oder wenigstens 90% zur Wildtypsequenz mit wenigstens einer der obigen Eigenschaften a), b), c), d) oder e).
   Als weitere Beispiele sind zu nennen:
   (1) Beispielsweise sind zu nennen die Einfachmutanten R64X₁ worin X₁ für E, D, T, L, S, P, V, K, C, A, G, Q, F, W, I oder Y, insbesondere E, D, T, L, S, P oder V, besonders bevorzugt E steht, welche wenigstens obige Eigenschaft a) besitzen.
   (2) Beispielsweise sind zu nennen die Einfachmutanten R64X₁ worin X₁ für E, D, T, L, S, P, V, K, C, A, G, Q, F, W, I oder Y, insbesondere E, D, T, L, S, P oder V, besonders bevorzugt E steht, welche wenigstens obige Eigenschaft b) besitzen.
   (3) Beispielsweise sind zu nennen die Einfachmutanten R64X₁ worin X₁ für E, D, T, L, S, P, V, K, C, A, G, Q, F, W, I oder Y, insbesondere E, D, T, L, S, P oder V, besonders bevorzugt E steht, welche wenigstens obige Eigenschaft c) besitzen.
   (4) Beispielsweise sind zu nennen die Einfachmutanten R64X₁ worin X₁ für E, D, T, L, S, P, V, K, C, A, G, Q, F, W, I oder Y, insbesondere E, D, T, L, S, P oder V, besonders bevorzugt E steht, welche wenigstens obige Eigenschaft d) besitzen
   (5) Beispielsweise sind zu nennen die Einfachmutanten R64X₁ worin X₁ für E, D, T, L, S, P, V, K, C, A, G, Q, F, W, I oder Y, insbesondere E, D, T, L, S, P oder V, besonders bevorzugt E steht, welche wenigstens obige Eigenschaften a) und b) besitzen.
   (6) Beispielsweise sind zu nennen die Einfachmutanten R64X₁ worin X₁ für E, D, T, L, S, P, V, K, C, A, G, Q, F, W, I oder Y, insbesondere E, D, T, L, S, P oder V, besonders bevorzugt E steht, welche wenigstens obige Eigenschaften a) und c) besitzen.
   (7) Beispielsweise sind zu nennen die Einfachmutanten R64X₁ worin X₁ für E, D, T, L, S, P, V, K, C, A, G, Q, F, W, I oder Y, insbesondere E, D, T, L, S, P oder V, besonders bevorzugt E steht, welche wenigstens obige Eigenschaften a) und d) besitzen.
   (8) Beispielsweise sind zu nennen die Einfachmutanten R64X₁ worin X₁ für E, D, T, L, S, P, V, K, C, A, G, Q, F, W, I oder Y, insbesondere E, D, T, L, S, P oder V, besonders bevorzugt E steht, welche wenigstens obige Eigenschaften b) und c) besitzen.
   (9) Beispielsweise sind zu nennen die Einfachmutanten R64X₁ worin X₁ für E, D, T, L, S, P, V, K, C, A, G, Q, F, W, I oder Y, insbesondere E, D, T, L, S, P oder V, besonders bevorzugt E steht, welche wenigstens obige Eigenschaften b) und d) besitzen.
   (10) Beispielsweise sind zu nennen die Einfachmutanten R64X₁ worin X₁ für E, D, T, L, S, P, V, K, C, A, G, Q, F, W, I oder Y, insbesondere E, D, T, L, S, P oder V, besonders bevorzugt E steht, welche wenigstens obige Eigenschaften a) bis d) besitzen.
   (11) Beispielsweise sind zu nennen die Doppelmutanten R64X₁/G39X₃ worin X₁ für E, D, T, L, S, P, V, K, C, A, G, Q, F, W, I oder Y, insbesondere E, D, T, L, S, P oder V, bevorzugt E steht, und X₃ für S, A, V, I, L, C, K, Y, F oder R, bevorzugt S oder A, steht, welche wenigstens obige Eigenschaft a) besitzen.
   (12) Beispielsweise sind zu nennen die Doppelmutanten R64X₁/G39X₃ worin X₁ für E, D, T, L, S, P, V, K, C, A, G, Q, F, W, I oder Y, insbesondere E, D, T, L, S, P oder V, bevorzugt E steht, und X₃ für S, A, V, I, L, C, K, Y, F oder R, bevorzugt S oder A, steht, welche wenigstens obige Eigenschaft b) besitzen.
   (13) Beispielsweise sind zu nennen die Doppelmutanten R64X₁/G39X₃ worin X₁ für E, D, T, L, S, P, V, K, C, A, G, Q, F, W, I oder Y, insbesondere E, D, T, L, S, P oder V, bevorzugt E steht, und X₃ für S, A, V, I, L, C, K, Y, F oder R, bevorzugt S oder A, steht, welche wenigstens obige Eigenschaft c) besitzen.
   (14) Beispielsweise sind zu nennen die Doppelmutanten R64X₁/G39X₃ worin X₁ für E, D, T, L, S, P, V, K, C, A, G, Q, F, W, I oder Y, insbesondere E, D, T, L, S, P oder V, bevorzugt E steht, und X₃ für S, A, V, I, L, C, K, Y, F oder R, bevorzugt S oder A, steht, welche wenigstens obige Eigenschaft d) besitzen.
   (15) Beispielsweise sind zu nennen die Doppelmutanten R64X₁/G39X₃ worin X₁ für E, D, T, L, S, P, V, K, C, A, G, Q, F, W, I oder Y, insbesondere E, D, T, L, S, P oder V, bevorzugt E steht, und X₃ für S, A, V, I, L, C, K, Y, F oder R, bevorzugt S oder A, steht, welche wenigstens obige Eigenschaften a) und b) besitzen.
   (16) Beispielsweise sind zu nennen die Doppelmutanten R64X₁/G39X₃ worin X₁ für E, D, T, L, S, P, V, K, C, A, G, Q, F, W, I oder Y, insbesondere E, D, T, L, S, P oder V, bevorzugt E steht, und X₃ für S, A, V, I, L, C, K, Y, F oder R, bevorzugt S oder A, steht, welche wenigstens obige Eigenschaften a) und c) besitzen.
   (17) Beispielsweise sind zu nennen die Doppelmutanten R64X₁/G39X₃ worin X₁ für E, D, T, L, S, P, V, K, C, A, G, Q, F, W, I oder Y, insbesondere E, D, T, L, S, P oder V, bevorzugt E steht, und X₃ für S, A, V, I, L, C, K, Y, F oder R, bevorzugt S oder A, steht, welche wenigstens obige Eigenschaften a) und d) besitzen.
   (18) Beispielsweise sind zu nennen die Doppelmutanten R64X₁/G39X₃ worin X₁ für E, D, T, L, S, P, V, K, C, A, G, Q, F, W, I oder Y, insbesondere E, D, T, L, S, P oder V, bevorzugt E steht, und X₃ für S, A, V, I, L, C, K, Y, F oder R, bevorzugt S oder A, steht, welche wenigstens obige Eigenschaften b) und c) besitzen.
   (19) Beispielsweise sind zu nennen die Doppelmutanten R64X₁/G39X₃ worin X₁ für E, D, T, L, S, P, V, K, C, A, G, Q, F, W, I oder Y, insbesondere E, D, T, L, S, P oder V, bevorzugt E steht, und X₃ für S, A, V, I, L, C, K, Y, F oder R, bevorzugt S oder A, steht, welche wenigstens obige Eigenschaften b) und d) besitzen.
   (20) Beispielsweise sind zu nennen die Doppelmutanten R64X₁/G39X₃ worin X₁ für E, D, T, L, S, P, V, K, C, A, G, Q, F, W, I oder Y, insbesondere E, D, T, L, S, P oder V, bevorzugt E steht, und X₃ für S, A, V, I, L, C, K, Y, F oder R, bevorzugt S oder A, steht, welche wenigstens obige Eigenschaften a) bis d) besitzen.
   Die oben aufgelisteten beispielhaften Ausführungsformen (1) bis (20) betreffen insbesondere Mutanten der 7β-HSDH mit SEQ ID NO:2 oder mit SEQ ID NO:3 und weisen dazu einen Identitätsgrad von wenigsten 80 % oder wenigstens 90% auf.
6. Nukleotidsequenz kodierend für eine 7β-HSDH nach einer der vorhergehenden Ausführungsformen.
   Beispielhaft zu nennen sind Nukleinsäuresequenzen, ausgewählt unter Nukleinsäuresequenzen
   a) gleichzeitig kodierend für eine GDH und eine 7β-HSDH Mutante nach einer der Ausführungsformen 1 bis 5 und gegebenenfalls eine 3α-HSDH;
   b) kodierend für ein Fusionsprotein umfassend eine GDH und eine 7β-HSDH Mutante nach einer der Ausführungsformen 1 bis 5 und gegebenenfalls eine 3α-HSDH;
      wobei die kodierenden Sequenzen unabhängig voneinander ein- oder mehrfach in dem Konstrukt, wie z.B. in 2, 3, 4, 5, oder 6 bis 10 Kopien, enthalten sein können. Durch Wahl der geeigneten Kopienzahl können so eventuell gegebene Aktivitätsunterschiede in den einzelnen Expressionsprodukten kompensiert werden.
7. Expressionskassette, umfassend unter der Kontrolle wenigstens einer regulativer Sequenz wenigstens eine Nukleotidsequenz nach Ausführungsform 67; sowie ggf. kodierende Sequenzen für wenigstens ein (wie z.B. 1 ,2 oder 3) weiteres Enzym, ausgewählt unter Hydroxysteroiddehydrogenasen, insbesondere 3α-HSDH, und zur Kofaktorregenerierung geeignete Dehydrogenasen, wie z.B. FDH, GDH, ADH, G-6-PDH, PDH. Insbesondere können die in einer Expressions-Kassette enthaltenen Enzyme verschiedene, bevorzugt aber gleiche Kofaktorenpaare nutzen, wie z.B. das Kofaktorenpaar NAD⁺/NADH oder NADP⁺/NADPH.
8. Expressionsvektor, umfassend wenigstens eine Expressionskassette nach Ausführungsform 7.
9. Rekombinanter Mikroorganismus, der wenigstens eine Nukleotidsequenz gemäß Ausführungsform 6 oder wenigstens eine Expressionskassette nach Ausführungsform 7 oder wenigstens einen Expressionsvektor nach Ausführungsform 8 trägt.
10. Rekombinanter Mikroorganismus nach Ausführungsform 9, der zusätzlich gegebenenfalls die kodierende Sequenz für wenigstens ein weiteres Enzym, ausgewählt unter Hydroxysteroiddehydrogenasen (HSDH) und zur Kofaktorregenerierung geeignete Dehydrogenasen, trägt.
11. Rekombinanter Mikroorganismus nach Ausführungsform 10, wobei
   die weitere HSHD ausgewählt ist unter 3α-HSDHs; und
   die Dehydrogenase ausgewählt ist unter NADPH-regenerierenden Enzymen, wie NADPH Dehydrogenasen, Alkoholdehydrogenasen (ADH), und NADPH regenerierenden Formiatdehydrogenasen (FDH), sowie Glucosedehydrogenase (GDH)-, Glucose-6-Phosphat-Dehydrogenase (G-6-PDH), oder Phosphit-Dehydrogenasen (PtDH), oder
   NADH-regenerierenden Enzymen, wie NADH Dehydrogenasen, NADH regenerierenden Formiatdehydrogenasen (FDH), NADH regenerierenden Alkoholdehydrogenasen (ADH), NADH regenerierenden Glucose-6-Phosphat-Dehydrogenasen (G6PDH), NADH regenerierenden Phosphitdehydrogenasen (PtDH) sowie NADH regenerierenden Glucosedehydrogenasen (GDH).
   Beispielsweise ist zu nennen ein rekombinanter Mikroorganismus, welcher zur gleichzeitigen Expression einer erfindungsgemäßen 7β-HSDH Mutante, einer hierin beschriebenen GDH und ggf. einer hierin beschriebenen 3α-HSDH befähigt ist.
   Gegenstand der Erfindung sind auch rekombinante Mikroorganismen nach einer der Ausführungsformen 9 bis 11, welche die kodierenden Sequenzen für 7β-HSDH Mutante, GDH bzw. Mutanten davon und 3α-HSDH auf einem oder mehreren (verschiedenen) Expressionskonstrukten tragen. Gegenstand der Erfindung sind somit rekombinante Mikroorganismen, welche mit einem Einplasmidsystem modifiziert (wie z.B. transformiert) sind, das die kodierenden Sequenzen für 7β-HSDH Mutante, GDH bzw. Mutanten davon und 3α-HSDH bzw. Mutanten davon in einer oder mehreren Kopien, wie z.B. in 2, 3, 4, 5, oder 6 bis 10 Kopien, tragen. Gegenstand der Erfindung sind somit auch rekombinante Mikroorganismen, welche mit einem Einplasmidsystem modifiziert (wie z.B. transformiert) sind, das die kodierenden Sequenzen für 7β-HSDH bzw. Mutanten davon, GDH bzw. Mutanten davon und 3α-HSDH bzw. Mutanten davon in einer oder mehreren Kopien, wie z.B. in 2, 3, 4, 5 oder 6 bis 10 Kopien tragen. Die Enzyme (7β-HSDH, GDH und 3α-HSDH bzw. deren Mutanten) können aber auch auf 2 oder 3 getrennten, miteinander kompatiblen Plasmiden in einer oder mehreren Kopien enthalten sein. Geeignete Basisvektoren zur Herstellung von Einplasmidsystemen und Multicopy-Plasmiden sind dem Fachmann bekannt. Als Beispiele können genannt werden für Einplasmidsystem, z.B. pET21a und für Multicopyplasmide z.B. die von der Firma Novagen vertriebenen Duet-Vektoren, wie pACYCDuet-1, pETDuet-1, pCDFDuet-1, pRSFDuet-1 und pCOLADuet-1. Derartige Vektoren, deren Kompatibilität mit anderen Vektoren und mikrobiellen Wirtsstämmen sind z.B. dem "User Protocol TB340 Rev. E0305 der Firma Novagen zu entnehmen.
   Die optimale Kombination von Enzymen für die Generierung von Plasmidsystemen kann der Fachmann unter Berücksichtigung der Lehre der vorliegenden Erfindung ohne unzumutbaren Aufwand vornehmen. So kann der Fachmann beispielsweise in Abhängigkeit von der Kofaktorspezifität des jeweils verwendeten 7β-HSDH-Enzyms das zur Kofaktorregenerierung am besten geeignete Enzym, ausgewählt unter den oben genannten Dehydrogenasen, insbesondere GDH und den jeweiligen Mutanten davon, auswählen.
   Weiterhin besteht die Möglichkeit, die zur Umsetzung gewählten Enzyme auf zwei oder mehrere Plasmide zu verteilen und mit den so hergestellten Plasmiden zwei oder mehrere verschiedene rekombinante Mikroorganismen herzustellen, die dann gemeinsam für die erfindungsgemäße biokatalytische Umsetzung eingesetzt werden. Die jeweilige zur Herstellung des Plasmids verwendete Enzymkombination kann dabei insbesondere auch unter der Vorgabe einer vergleichbaren Kofaktor-Nutzung erfolgen. So kann beispielsweise ein erster Mikroorganismus mit einem Plasmid modifiziert werden, welches die kodierende Sequenz für eine 7β-HSDH-Mutante und eine GDH tragen. Ein zweiter Mikroorganismus kann dagegen mit einem Plasmid modifiziert sein, das die kodierende Sequenz für eine 3α-HSDH und die kodierende Sequenz für eine GDH trägt. Beide Enzympaare können so gewählt sein, dass sie gleiche Kofaktoren-Paare regenerieren können. Beide Mikroorganismen können dann gleichzeitig zur erfindungsgemäßen biokatalytischen Umsetzung eingesetzt werden.
   Die Verwendung von zwei getrennten Biokatalysatoren (rekombinanter Mikroorganismen) kann gegenüber der Verwendung nur eines Biokatalysators, in dem alle Syntheseenzyme exprimiert werden, zwei wesentliche Vorteile bieten:
   a) Beide Biokatalysatoren können separat voneinander gentechnisch modifiziert und optimiert werden. Insbesondere ist der Einsatz von verschiedenen Kofaktorregenerierungsenzymen möglich, die entweder auf NADH- oder auf NADPH-Regenerierung optimiert sind.
   b) Für die Biokatalyse können die Biokatalysatoren in unterschiedlichen Anteilen eingesetzt werden. Dies ermöglicht ein Eingreifen in die einzelnen Reaktionsgeschwindigkeiten des Multienzymprozesses während der Biokatalyse, selbst nachdem sämtliche Biokatalysatoren bereits hergestellt sind.
12. Rekombinanter Mikroorganismus nach einer der Ausführungsformen 9 bis 11 welcher ein 7α-HSDH knock-out Stamm ist, wobei der Stamm, wie z.B. beschrieben in der WO2011/147957 ist.
13. Verfahren zur enzymatischen oder mikrobiellen Synthese von 7β-Hydroxysteroiden, wobei man das korrespondierende 7-Ketosteroid in Gegenwart einer 7β-HSDH gemäß der Definition in einer der Ausführungsformen 1 bis 5 oder in Gegenwart eines diese 7β-HSDH exprimierenden rekombinanten Mikroorganismus nach einer der Ausführungsformen 9 bis 12 reduziert, und gegebenenfalls wenigstens ein gebildetes Reduktionsprodukt aus dem Reaktionsansatz isoliert.
14. Verfahren nach Ausführungsform 13, wobei das 7- Ketosteroid ausgewählt ist unter Dehydrocholsäure (DHCA),
   7-Keto-lithocholsäure (7-Keto-LCS),
   7,12-Diketo-lithocholsäure (7,12-Diketo-LCS) und
   den Derivaten davon, wie insbesondere einem Salz, Amid oder Alkylester der Säure.
15. Verfahren nach Ausführungsform 13 oder 14, wobei die Reduktion in Gegenwart und insbesondere unter Verbrauch von NADPH und/oder NADH erfolgt.
16. Verfahren nach Ausführungsform 15, wobei verbrauchtes NADPH durch Kopplung mit einem NADPH-regenerierenden Enzym regeneriert wird, wobei dieses insbesondere ausgewählt ist unter NADPH Dehydrogenasen, Alkoholdehydrogenasen (ADH), und NADPH regenerierenden Formiatdehydrogenasen (FDH) und einer NADPH-regenerierenden Glucosedehydrogenase (GDH), wobei das NADPH-regenerierende Enzym gegebenenfalls von einem rekombinanten Mikroorganismus exprimiert wird; und/oder wobei verbrauchtes NADH durch Kopplung mit einem NADH-regenerierenden Enzym regeneriert wird, wobei dieses insbesondere ausgewählt ist unter NADH-Dehydrogenasen, NADH-regenerierenden Formiatdehydrogenasen (FDH), NADH-regenerierenden Alkoholdehydrogenasen (ADH), NADH-regenerierenden Glucose-6-Phosphat-Dehydrogenasen (G6PDH), NADH-regenerierenden Phosphitdehydrogenasen (PtDH) sowie NADH-regenerierenden Glucosedehydrogenasen (GDH), wobei das NADH-regenerierende Enzym ggf. in einem rekombinanten Mikroorganismus exprimiert wird.
17. Verfahren nach Ausführungsform 16, wobei das NADPH-regenerierende Enzym ausgewählt ist unter
   a) FDHs, einschließlich Mutanten einer NAD⁺-abhängigen FDH, welche wenigstens die enzymatische Oxidation von Ameisensäure zu CO₂ katalysiert, wobei die Mutante im Vergleich zum nicht-mutierten Enzym zusätzlich NADP⁺ als Kofaktor akzeptiert; und
   b) GDHs.
18. Verfahren zur Herstellung von Ursodesoxycholsäure (UDCS) der Formel (1) 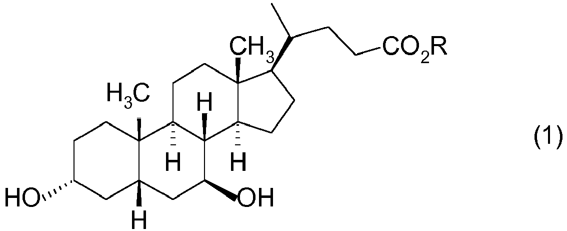 worin
   R für Alkyl, H, ein Alkalimetallion oder N(R³)₄⁺steht, worin die Reste R³ gleich oder verschieden sind und für H oder Alkyl stehen, oder die Gruppe -CO₂R durch die Säureamid-Gruppe -CONR¹R², ersetzt ist, worin R¹ und R² unabhängig voneinander für einen Alkylrest stehen;
   wobei man
   a) gegebenenfalls eine Cholsäure (CS) der Formel (2) 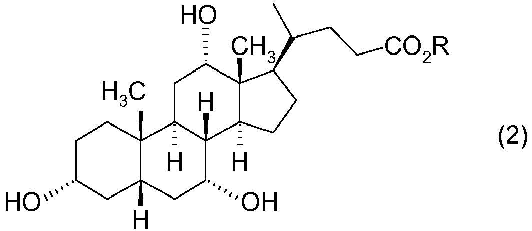 worin R die oben angegebenen Bedeutungen besitzt oder die Gruppe -CO₂R durch die Säureamid-Gruppe -CONR¹R², wie oben definiert ersetzt ist,
      zur Dehydrocholsäure (DHCS) der Formel (3) 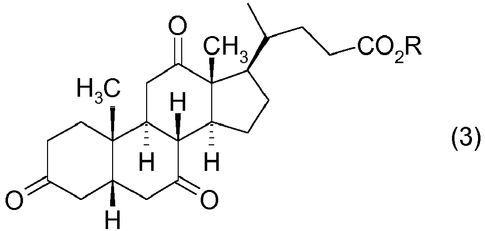 worin R die oben angegebenen Bedeutungen besitzt, oder die Gruppe -CO₂R durch die Säureamid-Gruppe -CONR¹R², wie oben definiert ersetzt ist, chemisch oxidiert;
   b) DHCS in Gegenwart wenigstens einer 7β-HSDH-Mutante gemäß der Definition in einer der Ausführungsformen 1 bis 5 (vorliegend als isoliertes Enzym oder exprimiert von einer entsprechenden rekombinanten Mikroorganismus) und in Gegenwart wenigstens einer 3α-HSDH (vorliegend als isoliertes Enzym oder exprimiert von einer entsprechenden rekombinanten Mikroorganismus) zur korrespondierenden 12-Keto-ursodesoxychiolsäure (12-Keto UDCS) der Formel (5) 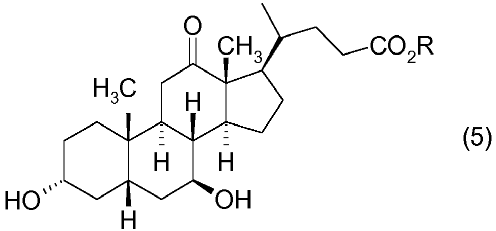 worin R die oben angegebenen Bedeutungen besitzt, oder die Gruppe -CO₂R durch die Säureamid-Gruppe -CONR¹R², wie oben definiert ersetzt ist, insbesondere in Gegenwart und unter Verbrauch von NADH und /oder NADPH
      reduziert und anschließend
   c) 12-Keto-UDCS der Formel (5) chemisch zu UDCS reduziert; und
   d) das Reaktionsprodukt gegebenenfalls weiter aufreinigt.
19. Verfahren nach Ausführungsform 18, wobei zumindest Schritt b) in Gegenwart eines rekombinanten Mikroorganismus nach einem der Ausführungsformen 9 bis 12 durchgeführt wird.
20. Verfahren nach Ausführungsform 18 oder 19, wobei Schritt b) mit gleichen oder verschiedenen Kofaktorregenerierungssystemen gekoppelt ist.
21. Verfahren zur Herstellung von UDCS der Formel (1) 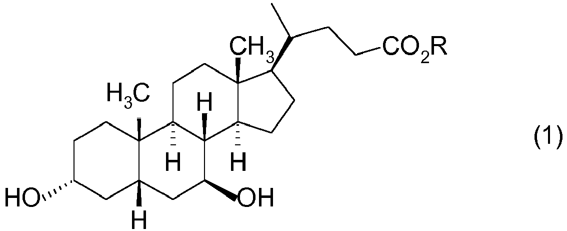 worin
   R für Alkyl, NR¹R², H, ein Alkalimetallion oder N(R³)₄⁺steht, worin die Reste R³ gleich oder verschieden sind und für H oder Alkyl stehen oder die Gruppe -CO₂R durch die Säureamid-Gruppe -CONR¹R², wie oben definiert ersetzt ist
   wobei man
   a) gegebenenfalls eine CS der Formel (2) 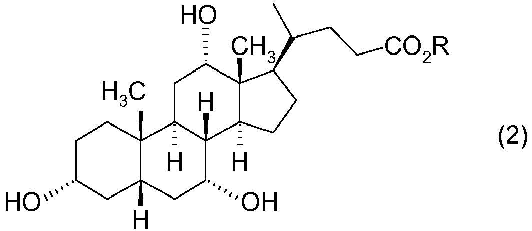 worin R die oben angegebenen Bedeutungen besitzt, oder die Gruppe -CO₂R durch die Säureamid-Gruppe -CONR¹R², wie oben definiert ersetzt ist, zur DHCS der Formel (3) 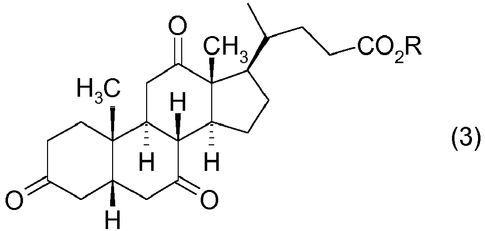 worin R die oben angegebenen Bedeutungen besitzt, oder die Gruppe -CO₂R durch die Säureamid-Gruppe -CONR¹R² wie oben definiert ersetzt ist, chemisch oxidiert;
   b) DHCS in Gegenwart wenigstens einer 7β-HSDH und in Gegenwart wenigstens einer 3α-HSDH zur korrespondierenden 12-Keto UDCS der Formel (5) 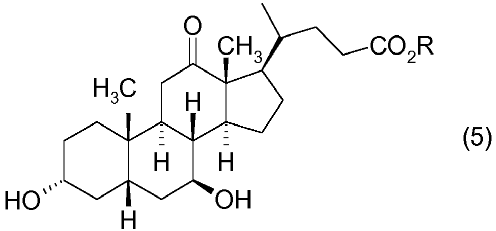 worin R die oben angegebenen Bedeutungen besitzt, oder die Gruppe -CO₂R durch die Säureamid-Gruppe -CONR¹R² wie oben definiert ersetzt ist, insbesondere in Gegenwart und unter Verbrauch von NADH und/oder NADPH, reduziert und anschließend
   c) 12-Keto-UDCS der Formel (5) chemisch zu UDCS reduziert; und
   d) das Reaktionsprodukt gegebenenfalls weiter aufreinigt;
   wobei die Umsetzungen des Schritts b) in Gegenwart eines rekombinanten Mikroorganismus nach einer der Ausführungsformen 9 bis 12 erfolgen, wie z.B. in Gegenwart ganzer Zellen eines oder mehrerer verschiedener rekombinanter Mikroorganismen nach einer der Ausführungsformen 9 bis 12 erfolgen, wobei der/die Mikroorganismen die zur Umsetzung und Kofaktorregenerierung erforderlichen Enzyme in einer hierin näher beschriebenen Weise tragen.
   Der Verfahrensschritt b) kann dabei unterschiedlich ausgestaltet sein. Entweder können beide Enzyme (7β-HSDH-Mutante und 3α-HSDH) gleichzeitig zugegen sein (z.B. Ein-Topf-Reaktion mit beiden isolierten Enzymen oder ein oder mehrere entsprechende rekombinante Mikroorganismen sind zugegen, der beide Enzyme exprimiert), oder die Teilreaktionen können in beliebiger Reihenfolge (erst die 7β-HSDH-Mutante-katalysierte Reduktion und dann die 3α-HSDH-katalysierte Reduktion; oder erst die 3α-HSDH -katalysierte Reduktion und dann die 7β-HSDH-Mutante -katalysierte Reduktion) ablaufen.
   Weiterhin kann Schritt b) mit einem Kofaktorregenerierungssystem gekoppelt sein, bei welchem NADPH durch eine NADPH regenerierende GDH unter Verbrauch von Glucose regeneriert wird; oder mit einem Kofaktorregenerierungssystem gekoppelt ist, bei welchem verbrauchtes NADH durch eine NADH regenerierende GDH, ADH oder FDH regeneriert wird.
23. Ebenfalls hierin beschrieben ist ein Bioreaktor zur Durchführung eines Verfahrens nach einer der Ausführungsformen 13 bis 21, insbesondere enthaltend wenigstens eines der Enzyme 7β-HSDH, FDH, und/oder 3α-HSDH bzw. deren Mutanten; oder 7β-HSDH, GDH und/oder 3α-HSDH bzw. deren Mutanten.
   Vorliegende Erfindung ist nicht auf die hierin beschriebenen konkreten Ausführungsformen beschränkt. Der Fachmann wird durch die Lehre der vorliegenden Erfindung vielmehr in die Lage versetzt, ohne unzumutbaren Aufwand weitere Ausgestaltungen der Erfindung bereitzustellen. So kann er beispielsweise auch weitere Enzymmutanten gezielt generieren und diese auf das gewünschte Eigenschaftsprofil (verbesserte Kofaktor-Abhängigkeit und/oder Stabilität, verringerte Substratinhibition) screenen und optimieren; oder weitere geeignete Wildtypenzyme (7β- und 3α-HSDHs, FDHs, GDHs ADHs usw.) isolieren und erfindungsgemäß verwenden. Weiterhin kann er beispielsweise je nach Eigenschaftsprofil (insbesondere Kofaktor-Abhängigkeit) der verwendeten HSDHs, wie insbesondere 7β-HSDH und 3α-HSDH oder Mutanten davon, geeignete zur Kofaktorregenerierung brauchbare Dehydrogenasen (GDH, FHD, ADH usw.) und Mutanten davon auswählen, und die ausgewählten Enzyme auf einen oder mehrere Expressionskonstrukte oder Vektoren verteilen und damit erforderlichenfalls einen oder mehrere rekombinante Mikroorganismen erzeugen, die dann ein optimiertes ganzzellbasiertes Herstellungsverfahren ermöglichen.

### Weitere Ausgestaltungen der Erfindung

### 1. Allgemeine Definitionen und verwendete Abkürzungen

Werden keine anderen Angaben gemacht, so bezeichnet der Begriff "7β-HSDH" ein Dehydrogenase-Enzym, welches wenigstens die stereospezifische und/oder regiospezifische Reduktion von DHCS oder 7,12-Diketo-3α-CS (7,12-Diketo-LCS) zu 3,12-Diketo-7β-CS oder 12-Keto-UDCS insbesondere unter stöchiometrischem Verbrauch von NADPH, und gegebenenfalls die entsprechende Umkehrreaktion katalysiert. Das Enzym kann dabei ein natives bzw. rekombinant hergestelltes Enzym sein. Das Enzym kann grundsätzlich im Gemisch mit zellulären, wie z.B. Proteinverunreinigungen, vorzugsweise aber in Reinform vorliegen. Geeignete Nachweisverfahren sind z.B. im folgenden experimentellen Teil beschrieben oder aus der Literatur bekannt (z.B. Characterization of NADP-dependent 7 beta-hydroxysteroid dehydrogenases from Peptostreptococcus productus and Eubacterium aerofaciens. S Hirano and N Masuda. Appl Environ Microbiol. 1982). Enzyme dieser Aktivität sind unter der EC Nummer 1.1.1.201 klassifiziert.

Werden keine anderen Angaben gemacht, so bezeichnet der Begriff "3α-HSDH" ein Dehydrogenase-Enzym, welches wenigstens die stereospezifische und/oder regiospezifische Reduktion von 3,12-Diketo-7β-CS oder DHCS zu 12-Keto-UDCS oder 7,12-Diketo-3α-CS (7,12-Diketo-LCS), insbesondere unter stöchiometrischem Verbrauch von NADH und/oder NADPH, und gegebenenfalls die entsprechende Umkehrreaktion katalysiert. Geeignete Nachweisverfahren sind z.B. im folgenden experimentellen Teil beschrieben oder aus der Literatur bekannt. Geeignete Enzyme sind z.B. aus *Comanomonas testosteroni* (z.B. ATCC11996) erhältlich. Eine NADPH abhängige 3α-HSDH ist z.B. aus der Nagern bekannt und ebenfalls einsetzbar. (Cloning and sequencing of the cDNA for rat liver 3 alphahydroxysteroid/dihydrodiol dehydrogenase, J E Pawlowski, M Huizinga and T M Penning, May 15, 1991 The Journal of Biological Chemistry, 266, 8820-8825). Enzyme dieser Aktivität sind unter der EC Nummer 1.1.1.50 klassifiziert.

Werden keine anderen Angaben gemacht, so bezeichnet der Begriff "GDH" ein Dehydrogenase-Enzym, welches wenigstens die Oxidation von β-D-Glucose zu D-Glucono-1,5-Lacton unter stöchiometrischem Verbrauch von NAD⁺ und/oder NADP⁺ sowie ggf. die entsprechende Umkehrreaktion katalysiert. Geeignete Enzyme sind z.B. aus *Bacillus subtili* oder *Bacillus megaterium* erhältlich. Enzyme dieser Aktivität sind unter der EC Nummer 1.1.1.47 klassifiziert.

Werden keine anderen Angaben gemacht, so bezeichnet der Begriff "FDH" ein Dehydrogenase-Enzym, welches wenigstens die Oxidation von Ameisensäure (oder korrespondierenden Formiat-Salzen) zu Kohlendioxid unter stöchiometrischem Verbrauch von NAD⁺ und/oder NADP⁺, und gegebenenfalls die entsprechende Umkehrreaktion katalysiert. Geeignete Nachweisverfahren sind z.B. im folgenden experimentellen Teil beschrieben oder aus der Literatur bekannt. Geeignete Enzyme sind z.B. aus *Candida boidinii, Pseudomonas* sp, oder *Mycobacterium vaccae* erhältlich. Enzyme dieser Aktivität sind unter der EC Nummer 1.2.1.2 klassifiziert.

Unter einer "Reinform" oder einem "reinen" oder "im wesentlichen reinen" Enzym versteht man ein Enzym mit einem Reinheitsgrad von mehr als 80, vorzugsweise mehr als 90, insbesondere mehr als 95, und vor allem mehr als 99 Gew.-%, bezogen auf den Gesamtproteingehalt, bestimmt mit Hilfe üblicher Proteinnachweismethoden, wie .z.B. der Biuret-Methode oder dem Proteinnachweis nach Lowry.et al.(vgl Beschreibung in R.K. Scopes, Protein Purification, Springer Verlag, New York, Heidelberg, Berlin (1982)).

Unter einem "Redoxäquivalent" versteht man eine als Elektronendonor bzw. Elektronenakzeptor brauchbare, niedermolekulare organische Verbindung, wie beispielsweise Nikotinamidderivate wie NAD⁺ und NADH⁺ bzw. deren reduzierte Formen NADH bzw. NADPH. "Redoxäquivalent" und "Kofaktor" werden im Kontext der vorliegenden Erfindung als Synonym verwendet. So kann ein "Kofaktor" im Sinne der Erfindung auch als "redoxfähiger Kofaktor", d.h. als Kofaktor, der in reduzierter und einer oxidierter Form vorliegen kann, umschrieben werden.

Unter einem "verbrauchten" Kofaktor versteht man diejenige reduzierte bzw. oxidierte Form des Kofaktors, die im Verlauf einer vorgegebene Reduktions- bzw. Oxidationsreaktion eines Substrats in die korrespondierende oxidierte bzw. reduzierte Form überführt wird. Durch Regenerierung wird die bei der Reaktion gebildete oxidierte bzw. reduzierte Kofaktor-Form wieder in die reduzierte bzw. oxidierte Ausgangsform zurück überführt, so dass diese für die Umsetzung des Substrats wieder zur Verfügung steht.

Unter einer "veränderten Kofaktor-Nutzung" versteht man im Rahmen der vorliegenden Erfindung eine qualitative oder quantitative Veränderung im Vergleich zu einer Referenz. Insbesondere ist eine veränderte Kofaktor-Nutzung durch Vornahme von Aminosäuresequenzmutationen zu beobachten. Diese Veränderung ist dann im Vergleich zum nicht-mutierten Ausgangsenzym feststellbar. Dabei kann die Aktivität in Bezug auf einen bestimmten Kofaktor durch Vornahme einer Mutation erhöht oder erniedrigt, oder vollständig unterbunden werden. Eine veränderte Kofaktor-Nutzung umfasst aber auch Änderungen dergestalt, dass anstelle einer Spezifität für einen einzelnen Kofaktor nunmehr wenigstens ein weiterer, vom ersten Kofaktor verschiedener, zweiter Kofaktor nutzbar ist (d.h. es liegt eine erweiterte Kofaktor-Nutzung vor) Umgekehrt kann aber auch eine ursprünglich vorhandene Befähigung zur Nutzung zweier verschiedener Kofaktoren so abgeändert werden, dass Spezifität nur für einen dieser Kofaktoren erhöht bzw. für einen dieser Kofaktoren verringert oder vollständig aufgehoben wird. So kann beispielsweise ein Enzym, das vom Kofaktor NAD (NADH) abhängig ist, aufgrund einer Veränderung der Kofaktor-Nutzung nunmehr sowohl von NAD (NADH) als auch vom Kofaktor NADP (NADPH) abhängig sein oder die ursprüngliche Abhängigkeit von NAD (NADH) kann vollständig in eine Abhängigkeit von NADP (NADPH) überführt werden und umgekehrt.

Die Begriffe "NAD⁺/NADH-Abhängigkeit" bzw. "NADP⁺/NADPH-Abhängigkeit" sind erfindungsgemäß, wenn nicht anders definiert, weit auszulegen. Diese Begriffe umfassen sowohl "spezifische" Abhängigkeiten, d.h. ausschließlich Abhängigkeit von NAD⁺/NADH bzw. NADP⁺/NADPH, als auch die Abhängigkeit der erfindungsgemäß verwendeten Enzyme von beiden Kofaktoren, d.h. Abhängigkeit von NAD⁺/NADH und NADP⁺/NADPH.

Entsprechendes gilt für die verwendeten Begriffe "NAD⁺/NADH-akzeptierend" bzw. "NADP⁺/NADPH-akzeptierend".

Die Begriffe "NAD⁺/NADH-regenerierend" bzw. "NADP⁺/NADPH-regenerierend" sind erfindungsgemäß, wenn nicht anders definiert, weit auszulegen. Diese Begriffe umfassen sowohl "spezifische" d.h. ausschließliche Befähigung verbrauchten Kofaktor NAD⁺/NADH bzw. NADP⁺/NADPH zu regenerieren, als auch die Befähigung beide Kofaktoren, d.h. NAD⁺/NADH und NADP⁺/NADPH, zu regenerieren.

"Proteinogene" Aminosäuren umfassen insbesondere (Einbuchstabencode): G, A, V, L, I, F, P, M, W, S, T, C, Y, N, Q, D, E, K, R und H.

Unter einer "Immobilisierung" versteht man die kovalente oder nicht-kovalente Bindung eines erfindungsgemäß verwendeten Biokatalysators, wie z.B. einer 7β-HSDH an einem festen, d.h. in dem umgebenden flüssigen Medium im Wesentlichen unlöslichen, Trägermaterial. Es können entsprechend auch ganze Zellen, wie die erfindungsgemäß verwendeten, rekombinanten Mikroorganismen mit Hilfe derartiger Träger immobilisiert sein.

Eine "im Vergleich zum nicht-mutierten Enzym verringerte Substratinhibition" bedeutet, dass das die beim nicht-mutierten Enzym für ein bestimmtes Substrat beobachtete Substratinhibition nicht mehr zu beobachten ist, d.h. im Wesentlichen nicht mehr messbar ist, oder erst bei höherer Substratkonzentration einsetzt, d.h. der Kᵢ-Wert erhöht ist.

Unter einer "Cholsäure-Verbindung" versteht man erfindungsgemäß Verbindungen mit dem Kohlenstoffgrundgerüst, insbesondere der Steroidstruktur der Cholsäure und dem Vorhandensein von Keto- und/oder Hydroxy- oder Acyloxy-Gruppen in der Ringposition 7 und gegebenenfalls den Ringpositionen 3 und/oder 12.

Unter einer Verbindung eines speziellen Typs, wie z.B. einer "Cholsäure-Verbindung" oder einer "Ursodesoxycholsäure-Verbindung" versteht man insbesondere auch Derivate der zugrunde liegenden Ausgangsverbindung (wie z.B. Cholsäure oder Ursodesoxycholsäure).

Derartige Derivate umfassen "Salze", wie z.B. Alkalimetallsalze wie Lithium-, Natrium- und Kaliumsalze der Verbindungen; sowie Ammoniumsalze, wobei man unter einem Ammoniumsalz das NH₄⁺-Salz bzw. solche Ammoniumsalze umfasst, worin wenigstens ein Wasserstoffatom durch einen C₁-C₆-Alkylrest ersetzt sein kann. Typische Alkylreste sind insbesondere C₁-C₄-Alkylreste, wie Methyl, Ethyl, n- oder i-Propyl-, n-, sec- oder tert-Butyl, sowie n-Pentyl und n-Hexyl und die ein- oder mehrfach verzweigten Analoga davon.

"Alkylester" erfindungsgemäß eingesetzter Verbindungen sind insbesondere Niedrigalkyl-Ester, wie z.B. C₁-C₆-Alkylester. Als nicht limitierende Beispiele sind zu nennen Methyl-, Ethyl-, n- oder i-Propyl-, n-, sec- oder tert-Butylester, oder längerkettige Ester, wie z.B. n-Pentyl- und n-Hexylester sowie die ein- oder mehrfach verzweigten Analoga davon.

"Amide" sind insbesondere Umsetzungsprodukte erfindungsgemäß eingesetzter Säuren mit Ammoniak oder primären oder sekundären Monoaminen. Derartige Amine sind beispielsweise Mono- oder Di-C₁-C₆-Alkyl-monoamine, wobei die Alkylreste unabhängig voneinander gegebenenfalls weiter substituiert sein können, wie z.B. durch Carboxy-, Hydroxy-, Halogen (wie F, Cl, Br, J)-, Nitro- und Sulfonatgruppen.

"Acylgruppen" sind insbesondere nichtaromatische Gruppen mit 2 bis 4 Kohlenstoffatomen, wie z.B. Acetyl, Propionyl und Butyryl, sowie aromatische Gruppen mit gegebenenfalls substituiertem einkernigen aromatischem Ring, wobei geeignete Substituenten z.B. ausgewählt sind unter Hydroxy-, Halogen (wie F, Cl, Br, J)-, Nitro- und C₁-C₆-Alkylgruppen, wie z.B. Benzoyl oder Toluoyl.

Die erfindungsgemäß eingesetzten bzw. hergestellten Hydroxysteroidverbindungen, wie z.B. Cholsäure, Ursodesoxycholsäure, 12-Keto-Chenodesoxycholsäure, Chenodesoxycholsäure und 7-Keto-Lithocholsäure können in stereoisomerenreiner Reinform oder im Gemisch mit anderen Stereoisomeren im erfindungsgemäßen Verfahren eingesetzt oder daraus erhalten werden. Vorzugsweise werden jedoch die eingesetzten bzw. die hergestellten Verbindungen in im Wesentlichen stereoisomerenreiner Form eingesetzt bzw. isoliert.

In folgender Tabelle sind die Strukturformeln, deren chemischen Namen und die verwendeten Abkürzungen wesentlicher chemischer Verbindungen tabellarisch zusammengefasst:

| Formel | Abkürzung | Chemischer Name |
|---|---|---|
| 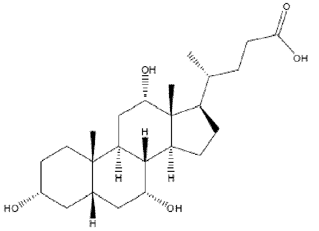 | CS | Cholsäure |
| Cholsäure | | |
| 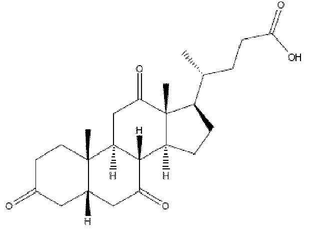 | DHCS | Dehydrocholsäure |
| Dehydrocholsäure | | |
| 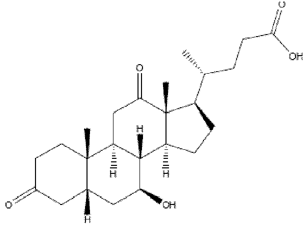 | 3,12-Diketo-7β-CS | 3,12-Diketo-7β-Hydroxycholansäure |
| 3,12-Diketo-7β-Hydroxycholansäure | | |
| 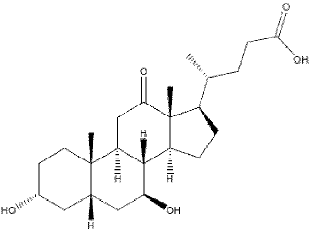 | 12Keto-UDCS | 12Keto-Ursodeoxycholsäure |
| 12keto-Ursodeoxycholsäure | | |
| 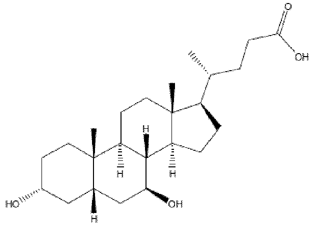 | UDCS | Ursodeoxycholsäure |
| Ursodeoxycholsäure | | |
| 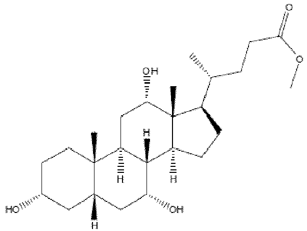 | CS-Methylester | Cholsäure-methylester |
| Cholsäure-methylester | | |
| 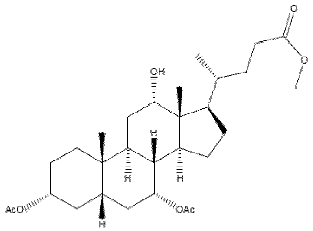 | 3,7-Diacetyl-CS-methylester | 3,7-Di-O-Acetylcholsäuremethylester* |
| 3,7-Di-O-Acetylcholsäuremethylester | | |
| 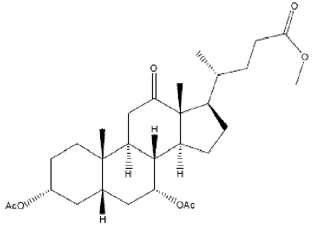 | 12-Keto-3,7-Diacetyl-CS-methylester | 12-Keto-3,7-Di-O-Acetylcholsäuremethylester* |
| 12-Keto-3,7-Di-O-Acetylcholsäuremethylester | | |
| 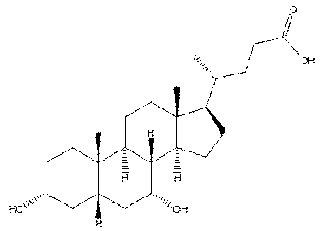 | CDCS | Chenodeoxycholsäure |
| Chenodeoxycholsäure | | |
| 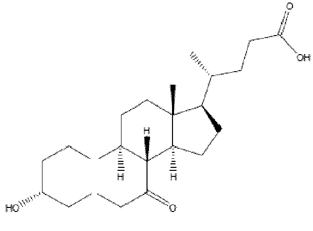 | 7-Keto-LCS | 7-Keto-Lithocholsäure |
| 7-Keto-Lithocholsäure | | |
| 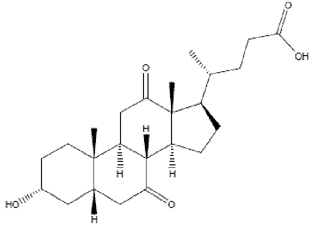 | 7,12-Diketo-LCS | 7,12-Diketo-Lithocholsäure |
| 7,12-Diketo-Lithocholsäure | | |
| 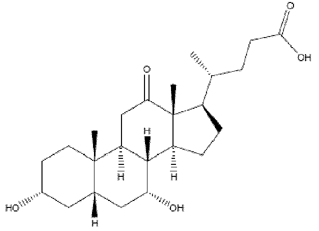 | 12-Keto-CDCS | 12-Keto-Chenodeoxycholsäure |
| 12-Keto-Chenodeoxycholsäure | | |

### 2. Proteine

Die vorliegende Erfindung ist nicht auf die konkret offenbarten Proteine bzw. Enzyme mit 7β-HSDH-, FDH-, GDH- oder 3α-HSDH Aktivität bzw. deren Mutanten beschränkt, sondern erstreckt sich vielmehr auch auf funktionale Äquivalente davon.

"Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. 7β HSDH-Aktivität, besitzen.

So versteht man beispielsweise unter "funktionalen Äquivalenten" Enzyme, die im verwendeten Test auf 7β-HSDH-, FDH-, GDH- oder 3α-HSDH -Aktivität eine um mindestens 1 % , wie z.B. mindestens 10 % oder 20 %, wie z.B. mindestens 50 % oder 75 % oder 90 % höhere oder niedrigere Aktivität eines Ausgangs-Enzyms, umfassend eine hierin definierte Aminosäuresequenz aufweisen.

Funktionale Äquivalente sind außerdem vorzugsweise zwischen pH 4 bis 11 stabil und besitzen vorteilhaft ein pH-Optimum in einem Bereich von pH 6 bis 10, wie insbesondere 8,5 bis 9,5, sowie ein Temperaturoptimum im Bereich von 15°C bis 80°C oder 20°C bis 70°C, wie z.B. etwa 45 bis 60°C oder etwa 50 bis 55°C.

Die 7β-HSDH-Aktivität kann mit Hilfe verschiedener bekannter Tests nachgewiesen werden. Ohne darauf beschränkt zu sein, sei ein Test unter Verwendung eines Referenzsubstrates, wie z. B. CS oder DHCS, unter standardisierten Bedingungen wie im experimentellen Teil definiert, zu nennen.

Tests zur Bestimmung der FDH-, GDH- oder 3α-HSDH -Aktivität sind ebenfalls an sich bekannt.

Unter "funktionalen Äquivalenten" versteht man erfindungsgemäß insbesondere auch "Mutanten", welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere, wie z.B. 1, 2, 3, 4, 5, 6, 7, 8, 9, 10, 11, 12, 13, 14 oder 15, Aminosäure-Additionen, -Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden. Beispiele für geeignete Aminosäuresubstitutionen sind in folgender Tabelle zusammengefasst:

| **Ursprünglicher Rest** | **Beispiele der Substitution** |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktivität.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls hierin beschrieben.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxylgruppen, hergestellt durch Umsetzung mit Acylgruppen.

"Funktionale Äquivalente" umfassen natürlich auch Polypeptide, welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide, Histidin-Anker, wie z.B. eines Hexahistidin-Anker umfassendes Peptid, wie z.B. "LEHHHHHH", oder Enzyme.

Erfindungsgemäß mit umfasste "funktionale Äquivalente" sind Homologe zu den konkret offenbarten Proteinen. Diese besitzen wenigstens 80 % beziehungsweise wenigsten 90 %, wie z.B. 91, 92, 93, 94, 95, 96, 97,98 oder 99%, Homologie (bzw. Identität) zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie bzw. Identität eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Die prozentualen Identitätswerte können auch anhand von BLAST Alignments, Algorithmus blastp (protein-protein BLAST), oder durch Anwendung der unten angegebenen Clustal Einstellungen ermittelt werden.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Proteine oder Polypeptide können durch Mutagenese erzeugt werden, z.B. durch Punktmutation, Verlängerung oder Verkürzung des Proteins.

Homologe der erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

Der 7β-HSDH-Wildtyp aus *Collinsella aerofaciens* ATCC 25986 ist in der WO2011/064404 der Anmelderin beschrieben.

Dieser aus *Collinsella aerofaciens* DSM 3979 erhältliche 7β-HSDH ist insbesondere durch wenigstens eine weitere der folgenden Eigenschaften, wie z.B. 2, 3, 4, 5, 6 oder 7 oder aller solcher Eigenschaften, gekennzeichnet:
a) Molekulargewicht (SDS-Gelelektrophorese): etwa 28-32 kDa, insbesondere etwa 29 bis 31 kDa oder etwa 30 kDa;
b) Molekulargewicht (Gelfiltration, unter nicht denaturierenden Bedingungen, wie insbesondere ohne SDS): etwa 53 bis 60 kDa, insbesondere etwa 55 bis 57 kDa , wie 56.1 kDa. Dies belegt die dimere Beschaffenheit der 7β-HSDH aus *Collinsella aerofaciens* DSM 3979;
c) Stereoselektiven Reduktion der 7-Carbonyl-Gruppe von 7-Keto-LCS in eine 7β-Hydroxy-Gruppe;
d) pH Optimum für die Oxidation von UDCS im Bereich von pH 8,5 bis 10,5, insbesondere 9 bis 10;
e) pH Optimum für die Reduktion von DHCS und 7-Keto-LCS im Bereich von pH 3,5 bis 6,5, insbesondere bei pH 4 bis 6;
f) wenigstens einen kinetischen Parameter aus folgender Tabelle für wenigstens eines der dort genannten Substrate/Kofaktoren; im Bereich von ±20%, insbesondere ±10%, ±5%, ±3% ±2% oder ±1% um den in der folgenden Tabelle jeweils konkret genannten Wert.

| | K_{M} (µM) | Vₘₐₓ (U/mg Protein)^{b)} | k_{cat} (1 µmol/(µmol×min)) |
|---|---|---|---|
| NADP⁺ | 5.32 | 30.58 | 944.95 |
| NADPH | 4.50 | 33.44 | 1033.44 |
| UDCS | 6.23 | 38.17 | 1179.39 |
| 7-Keto-LCS | 5.20 | 30.77 | 950.77 |
| DHCS | 9.23 | 28.33 | 875.35 |
| NAD⁺ | -^{a)} | - | Spuren |
| NADH | - | - | Spuren |

| | | | |
|---|---|---|---|
| ^{a)} konnten aufgrund der sehr geringen Aktivität nicht bestimmt werden ^{b)} 1 U = 1 µmol/min | | | |

g) Phylogenetische Sequenzverwandtschaft der prokaryotischen 7β-HSDH aus *Collinsella aerofaciens* DSM 3979 mit der tierischen 11β-HSDH-Untergruppe, umfassend *Cavia porcellus, Homo sapiens* und *Mus musulus,* verwandt.

Beispielsweise zeigt diese 7β-HSDH folgende Eigenschaften oder Kombinationen von Eigenschaften; a); b); a) und b); a) und/oder b) und c); a) und/oder b) und c) und d); a) und/oder b) und c) und d) und e); a) und/oder b) und c) und d) und e) und f).

Eine derartige 7β-HSDH oder davon abgeleitetes funktionales Äquivalent ist zudem gekennzeichnet durch
a) die stereospezifische Reduktion eines 7-Ketosteroids zum korrespondierenden 7β-Hydroxysteroid, und/oder
b) die regiospezifische Hydroxylierung eines Ketosteroids umfassend eine Ketogruppe in 7-Position und wenigstens eine weitere Ketogruppe am Steroidgerüst zum korrespondierenden 7β-Hydroxysteroid, wie insbesondere von Dehydrocholsäure (DHCS) in 7-Position zur korrespondierenden 3,12-Diketo-7β-Cholansäure, und die z.B. NADPH abhängig ist.

Eine derartige 7β-HSDH hat insbesondere eine Aminosäuresequenz gemäß SEQ ID NO:2 (Accession NO: ZP_01773061) oder eine davon abgeleiteten Sequenz mit einem Identitätsgrad von wenigstens 60%, wie z.B. wenigstens 65, 70, 75, 80, 85, oder 90, wie z.B. wenigstens 91, 92, 93, 94, 95, 96, 97, 98, 99 oder 99,5% zu dieser Sequenz; gegebenenfalls zusätzlich gekennzeichnet durch eine der folgende Eigenschaften oder Kombinationen von Eigenschaften; a); b); a) und b); a) und/oder b) und c); a) und/oder b) und c) und d); a) und/oder b) und c) und d) und e); a) und/oder b) und c) und d) und e) und f) gemäß obiger Definition.

### 3. Nukleinsäuren und Konstrukte

### 3.1 Nukleinsäuren

Gegenstand der Erfindung sind auch Nukleinsäuresequenzen, die für ein Enzym mit 7β-HSDH-, FDH-, GDH- und/oder 3α-HSDH Aktivität und deren Mutanten kodieren.

Die vorliegende Erfindung betrifft auch Nukleinsäuren mit einem bestimmten Identitätsgrad zu den hierin beschriebenen konkreten Sequenzen.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:

### Multiple alignment parameters:

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

### Pairwise alignment parameter:

| | |
|---|---|
| FAST algorithm | on |
| K-tuplesize | 1 |
| Gap penalty | 3 |
| Window size | 5 |
| Number of best diagonals | 5 |

Alternativ dazu kann die Identität auch nach Chenna, Ramu, Sugawara, Hideaki, Koike,Tadashi, Lopez, Rodrigo, Gibson, Toby J, Higgins, Desmond G, Thompson, Julie D. Multiple sequence alignment with the Clustal series of programs. (2003) Nucleic Acids Res 31 (13):3497-500, gemäß Internetadresse: http://www.ebi.ac.uk/Tools/clustalw/index.html# und mit den folgenden Parametern bestimmt werden:

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Gegenstand der Erfindung sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalente, welche z.B. unter Verwendung künstlicher Nukleotidanaloga zugänglich sind.

Die Erfindung betrifft sowohl isolierte Nukleinsäuremoleküle, welche für erfindungsgemäße Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von erfindungsgemäßer kodierenden Nukleinsäuren verwendet werden können.

Die erfindungsgemäßen Nukleinsäuremoleküle können zudem untranslatierte Sequenzen vom 3'- und/oder 5'-Ende des kodierenden Genbereichs enthalten
Die Erfindung umfasst weiterhin die zu den konkret beschriebenen Nukleotidsequenzen komplementären Nukleinsäuremoleküle oder einen Abschnitt davon.

Die erfindungsgemäßen Nukleotidsequenzen ermöglichen die Erzeugung von Sonden und Primern, die zur Identifizierung und/oder Klonierung von homologen Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Erfindungsgemäße Nukleinsäuresequenzen oder Derivate davon, Homologe oder Teile dieser Sequenzen, lassen sich beispielsweise mit üblichen Hybridisierungsverfahren oder der PCR-Technik aus anderen Bakterien, z.B. über genomische oder cDNA-Banken, isolieren. Diese DNA-Sequenzen hybridisieren unter Standardbedingungen mit den erfindungsgemäßen Sequenzen.

Unter "hybridisieren" versteht man die Fähigkeit eines Poly- oder Oligonukleotids an eine nahezu komplementäre Sequenz unter Standardbedingungen zu binden, während unter diesen Bedingungen unspezifische Bindungen zwischen nicht-komplementären Partnern unterbleiben. Dazu können die Sequenzen zu 90-100% komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze.

Zur Hybridisierung werden vorteilhaft kurze Oligonukleotide der konservierten Bereiche verwendet. Es können aber auch längere Fragmente der erfindungsgemäßen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca. 10 °C niedriger als die von DNA:RNA-Hybriden gleicher Länge.

Unter Standardbedingungen sind beispielsweise je nach Nukleinsäure Temperaturen zwischen 42 und 58 °C in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Die "Hybridisierung" kann insbesondere unter stringenten Bedingungen erfolgen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben.

Unter "stringenten" Hybridisierungs-Bedingungen werden insbesondere verstanden: Die Inkubation bei 42°C über Nacht in einer Lösung bestehend aus 50 % Formamid, 5 x SSC (750 mM NaCl, 75 mM Tri-Natrium-citrat), 50 mM Natrium Phosphat (pH7,6), 5x Denhardt Lösung, 10% Dextransulfat und 20 g/ml denaturierte, gescheerte Lachsspermien-DNA, gefolgt von einem Waschschritt der Filter mit 0,1x SSC bei 65°C.

Gegenstand der Erfindung sind auch Derivate der konkret offenbarten oder ableitbaren Nukleinsäuresequenzen.

So können weitere erfindungsgemäße Nukleinsäuresequenzen z.B. von SEQ ID NO:1, 7, oder 9 (oder von den für die Aminosäuresequenzen 2 bis 6, 8, 10 oder 11 kodierenden Nukleinsäuresequenzen) abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Gegenstand sind ebenso durch konservative Nukleotidsubstitutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten der erfindungsgemäßen Nukleinsäuresequenz mit der Sequenz SEQ ID NO:1, 7, oder 9 (oder von den für die Aminosäuresequenzen 2 bis 6, 8, 10 oder 11 kodierenden Nukleinsäuresequenzen) sind beispielsweise Allelvarianten zu verstehen, die mindestens 60 % Homologie auf der abgeleiteten Aminosäureebene, bevorzugt mindestens 80 % Homologie, ganz besonders bevorzugt mindestens 90 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivaten auch Homologe der erfindungsgemäßen Nukleinsäuresequenzen, insbesondere der SEQ ID NO:1, 7, oder 9 (oder von den für die Aminosäuresequenzen 2 bis 6, 8, 10 oder 11 kodierenden Nukleinsäuresequenzen) beispielsweise pilzliche oder bakterielle Homologe, verkürzte Sequenzen, Einzelstrang-DNA oder RNA der kodierenden und nichtkodierenden DNA-Sequenz, zu verstehen. So besitzen z.B. Homologe zur der SEQ ID NO:1, 7, oder 9 (oder von den für die Aminosäuresequenzen 2 bis 6, 8, 10 oder 11 kodierenden Nukleinsäuresequenzen) auf DNA-Ebene eine Homologie von mindestens 40 %, bevorzugt von mindestens 60 %, besonders bevorzugt von mindestens 70 %, ganz besonders bevorzugt von mindestens 80 % über den gesamten SEQ ID NO:1, 7, oder 9 (oder von den für die Aminosäuresequenzen 2 bis 6, 8, 10 oder 11 kodierenden Nukleinsäuresequenzen) angegebenen DNA-Bereich.

Außerdem sind unter Derivaten beispielsweise Fusionen mit Promotoren zu verstehen. Die Promotoren, die den angegebenen Nukleotidsequenzen vorgeschalten sind, können durch wenigstens einen Nukleotidaustausch, wenigstens eine Insertionen, Inversionen und/oder Deletionen verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Dem Fachmann sind darüber hinaus Verfahren zur Erzeugung funktionaler Mutanten bekannt.

Je nach verwendeter Technik kann der Fachmann völlig zufällige oder auch gezieltere Mutationen in Gene oder auch nicht codierende Nukleinsäurebereiche (die beispielsweise für die Regulation der Expression wichtig sind) einbringen und anschließend Genbanken erstellen. Die dazu erforderlichen molekularbiologischen Methoden sind dem Fachmann bekannt und beispielsweise beschrieben in Sambrook und Russell, Molecular Cloning. 3. Edition, Cold Spring Harbor Laboratory Press 2001.

Methoden zur Veränderung von Genen und somit zur Veränderung der durch diese codierten Proteine sind dem Fachmann seit langem geläufig, wie beispielsweise
- die ortsspezifische Mutagenese, bei der gezielt einzelne oder mehrere Nukleotide eines Gens ausgetauscht werden (Trower MK (Hrsg.) 1996; In vitro mutagenesis protocols. Humana Press, New Jersey),
- die Sättigungsmutagenese, bei der an jeder beliebigen Stelle eines Gens ein Codon für eine beliebige Aminosäure ausgetauscht oder hinzugefügt werden kann (Kegler-Ebo DM, Docktor CM, DiMaio D (1994) Nucleic Acids Res 22:1593; Barettino D, Feigenbutz M, Valcárel R, Stunnenberg HG (1994) Nucleic Acids Res 22:541; Barik S (1995) Mol Biotechnol 3:1),
- die fehleranfällige Polymerase-Kettenreaktion (error-prone PCR), bei der Nukleotidsequenzen durch fehlerhaft arbeitende DNA-Polymerasen mutiert werden (Eckert KA, Kunkel TA (1990) Nucleic Acids Res 18:3739);
- das Passagieren von Genen in Mutator-Stämmen, in denen beispielsweise aufgrund defekter DNA-Reperaturmechanismen eine erhöhte Mutationsrate von Nukleotidsequenzen auftritt (Greener A, Callahan M, Jerpseth B (1996) An efficient random mutagenesis technique using an E.coli mutator strain. In: Trower MK (Hrsg.) In vitro mutagenesis protocols. Humana Press, New Jersey), oder
- das DNA-Shuffling, bei dem ein Pool aus nahe verwandten Genen gebildet und verdaut wird und die Bruchstücke als Templates für eine Polymerase-Kettenreaktion verwendet werden, bei der durch wiederholte Strangtrennung und Wiederannäherung letztendlich Mosaikgene voller Länge erzeugt werden (Stemmer WPC (1994) Nature 370:389; Stemmer WPC (1994) Proc Natl Acad Sci USA 91:10747).

Unter Anwendung der sogenannten gerichteten Evolution ("directed evolution"; beschrieben unter anderem in Reetz MT und Jaeger K-E (1999), Topics Curr Chem 200:31; Zhao H, Moore JC, Volkov AA, Arnold FH (1999), Methods for optimizing industrial enzymes by directed evolution, In: Demain AL, Davies JE (Hrsg.) Manual of industrial microbiology and biotechnology. American Society for Microbiology) kann der Fachmann auch in gezielter Weise und auch in großem Maßstab funktionale Mutanten erzeugen. Dabei werden in einem ersten Schritt zunächst Genbanken der jeweiligen Proteine erzeugt, wobei beispielsweise die oben angegebenen Methoden zur Anwendung kommen können. Die Genbanken werden auf geeignete Weise exprimiert, beispielsweise durch Bakterien oder durch Phagen-Display-Systeme.

Die betreffenden Gene von Wirtsorganismen, die funktionale Mutanten mit Eigenschaften exprimieren, welche den gewünschten Eigenschaften weitgehend entsprechen, können einer weiteren Mutationsrunde unterworfen werden. Die Schritte der Mutation und der Selektion oder des Screening können iterativ solange wiederholt werden, bis die vorliegenden funktionalen Mutanten die gewünschten Eigenschaften in ausreichendem Maße aufweisen. Durch diese iterative Arbeitsweise können stufenweise eine begrenzte Anzahl von Mutationen , wie z.B. 1 bis 5 Mutationen, vorgenommen und auf deren Einfluss auf die betreffende Enzymeigenschaft bewertet und selektiert werden. Die selektierte Mutante kann dann in gleicher Weise einem weiteren Mutationsschritt unterworfen werden. Dadurch lässt sich die Anzahl der zu untersuchenden Einzelmutanten signifikant verringern.

Die erfindungsgemäßen Ergebnisse liefern wichtige Informationen in Bezug auf Struktur und Sequenz der betreffenden Enzyme, die erforderlich sind, um gezielt weitere Enzyme mit gewünschten modifizierten Eigenschaften zu generieren. Insbesondere können sogenannte "hot spots" definiert werden, d.h. Sequenzabschnitte, die sich potentiell eignen, um über die Einführung gezielter Mutationen eine Enzymeigenschaft zu modifizieren.

### 3.2 Konstrukte

Gegenstand der Erfindung sind außerdem Expressionskonstrukte, enthaltend unter der genetischen Kontrolle regulativer Nukleinsäuresequenzen eine für wenigstens ein erfindungsgemäßes Polypeptid kodierende Nukleinsäuresequenz; sowie Vektoren, umfassend wenigstens eines dieser Expressionskonstrukte.

Unter einer "Expressionseinheit" wird eine Nukleinsäure mit Expressionsaktivität verstanden, die einen Promotor, wie hierein definiert umfasst, und nach funktioneller Verknüpfung mit einer zu exprimierenden Nukleinsäure oder einem Gen, die Expression, also die Transkription und die Translation dieser Nukleinsäure oder dieses Gens reguliert. Man spricht deshalb auch in diesem Zusammenhang von einer "regulativen Nukleinsäuresequenz". Zusätzlich zum Promotor können weitere, regulative Elemente, wie z.B. Enhancer, enthalten sein.

Unter einer "Expressionskassette" oder "Expressionskonstrukt" wird erfindungsgemäß eine Expressionseinheit verstanden, die mit der zu exprimierenden Nukleinsäure oder dem zu exprimierenden Gen funktionell verknüpft ist. Im Gegensatz zu einer Expressionseinheit umfasst eine Expressionskassette somit nicht nur Nukleinsäuresequenzen, welche Transkription und Translation regulieren, sondern auch die Nukleinsäuresequenzen, welche als Folge der Transkription und Translation als Protein exprimiert werden sollen.

Die Begriffe "Expression" oder "Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einem "Promotor", einer "Nukleinsäure mit Promotoraktivität" oder einer "Promotorsequenz" wird eine Nukleinsäure verstanden, die in funktioneller Verknüpfung mit einer zu transkribierenden Nukleinsäure die Transkription dieser Nukleinsäure reguliert.

Unter einer "funktionellen" oder "operativen" Verknüpfung versteht man in diesem Zusammenhang beispielsweise die sequentielle Anordnung einer der Nukleinsäuren mit Promotoraktivität und einer zu transkribierenden Nukleinsäuresequenz und gegebenenfalls weiterer regulativer Elemente, wie zum Beispiel Nukleinsäuresequenzen, die die Transkription von Nukleinsäuren gewährleisten, sowie zum Beispiel einen Terminator, derart, dass jedes der regulativen Elemente seine Funktion bei der Transkription der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu transkribierende Nukleinsäuresequenz hinter (d.h. am 3'-Ende) der Promotorsequenz positioniert wird, so dass beide Sequenzen kovalent miteinander verbunden sind. Dabei kann der Abstand zwischen der Promotorsequenz und der transgen zu exprimierende Nukleinsäuresequenz geringer als 200 Basenpaare, oder kleiner als 100 Basenpaare oder kleiner als 50 Basenpaare sein.

Neben Promotoren und Terminator sind als Beispiele weiterer regulativer Elemente zu nennen Targeting-Sequenzen, Enhancer, Polyadenylierungssignale, selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Erfindungsgemäße Nukleinsäurekonstrukte umfassen insbesondere Sequenz SEQ ID NO:1, 7, oder 9 (oder von den für die Aminosäuresequenzen 2 bis 6, 8, 10 oder 11 kodierenden Nukleinsäuresequenzen) oder Derivate und Homologe davon, sowie die davon ableitbaren Nukleinsäuresequenzen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die erfindungsgemäßen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Beispiele geeigneter Regulationssequenzen sind in Promotoren wie cos-, tac-, trp-, tet-, trp-tet-, lpp-, lac-, lpp-lac-, lacl^{q-,} T7-, T5-, T3-, gal-, trc-, ara-, rhaP (rhaP_{BAD})SP6-, lambda-P_{R}- oder im lambda-P_{L}-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha , AC, P-60, CYC1, GAPDH, TEF, rp28, ADH enthalten. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar.

Geeignete Plasmide sind beispielsweise in *E. coli* pLG338, pACYC184, pBR322, pUC18, pUC19, pKC30, pRep4, pHS1, pKK223-3, pDHE19.2, pHS2, pPLc236, pMBL24, pLG200, pUR290, pIN-III¹¹³-B1, Agt11 oder pBdCl, in Streptomyces plJ101, pIJ364, plJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, pGHlac⁺, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985 , ISBN 0 444 904018) entnommen werden.

In einer weiteren Ausgestaltungsform des Vektors kann der das erfindungsgemäße Nukleinsäurekonstrukt oder die erfindungsgemäße Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäßen Nukleinsäure bestehen.

Für eine optimale Expression heterologer Gene in Organismen ist es vorteilhaft die Nukleinsäuresequenzen entsprechend des im Organismus verwendeten spezifischen "Codonnutzung" zu verändern. Der "Codonnutzung" lässt sich anhand von Computerauswertungen anderer, bekannter Gene des betreffenden Organismus leicht ermitteln.

Die Herstellung einer erfindungsgemäßen Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

### 4. Mikroorganismen

Je nach Zusammenhang kann unter dem Begriff "Mikroorganismus" der Ausgangsmikroorganismus (Wildtyp) oder ein genetisch veränderter, rekombinanter Mikroorganismus oder beides verstanden werden.

Mit Hilfe der erfindungsgemäßen Vektoren sind rekombinante Mikroorganismen herstellbar, welche beispielsweise mit wenigstens einem erfindungsgemäßen Vektor transformiert sind und zur Produktion der erfindungsgemäßen Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben. Einen Überblick über bakterielle Expressionssysteme für die heterologe Expression von Proteinen liefertz.B. auch Terpe, K. Appl. Microbiol. Biotechnol. (2006) 72: 211-222.

Als rekombinante Wirtsorganismen für die erfindungsgemäße Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gramnegative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium, Clostridium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden

Der Wirtsorganismus oder die Wirtsorganismen gemäß der Erfindung enthalten dabei vorzugsweise mindestens eine der in dieser Erfindung beschriebenen Nukleinsäuresequenzen, Nukleinsäurekonstrukte oder Vektoren, die für ein Enzym mit 7β-HSDH-Aktivität gemäß obiger Definition kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu Beginn der Fermentation vorgelegt oder semikontinuierlich oder kontinuierlich nachgefüttert werden.

### 5. Herstellung der UDCS

### 1. Schritt: Chemische Umsetzung von CS zu DHCS

Die Hydroxygruppen von CS werden mit Chromsäure bzw. Chromaten in saurer Lösung (z.B. H₂SO₄) zu Carbonylgruppe in an sich bekannter Weise auf klassischchemischem Weg oxidiert. Dadurch entsteht DHCS.

### 2. Schritt: Enzymatische oder mikrobielle Umsetzung von DHCS zu 12-Keto-UDCS

In wässriger Lösung wird DHCS durch 3α-HSDH und 7β-HSDH bzw. Mutanten davon spezifisch zu 12-Keto-UDCS in Anwesenheit von NADPH bzw. NADH reduziert. Der Kofaktor NADPH bzw. NADH kann durch eine ADH bzw. FDH bzw. GDH bzw. Mutanten davon von Isopropanol bzw. Natium-formiat bzw. Glucose regeneriert werden. Die Reaktion läuft unter milder Bedingung. Beispielsweise kann die Reaktion bei pH = 6 bis 9, insbesondere etwa pH = 8 und bei etwa 10 bis 30, 15 bis 25 oder etwa 23°C durchgeführt werden. Im falle eines mikrobiellen Umsetzungsschrittes können rekombinante Mikroorganismen, welche die erforderliche(n) Enzymaktivität(en) exprimieren in Gegenwart des umzusetzenden Substrates (DHCS) anaerob oder aerob in geeigneten Flüssigmedien kultiviert werden. Geeignete Kultivierungsbedingungen sind dem Fachmann an sich bekannt. Sie umfassen Umsetzungen im pH Bereich von beispielsweise 5 bis 10 oder 6 bis 9, bei Temperaturen im Bereich von 10 bis 60 oder 15 bis 45 oder 25 bis 40 oder 37 °C. Geeignete Medien umfassen z.B. die unten beschriebenen LB und TB Medien. Die Umsetzungsdauer kann dabei z.B. batchweise oder kontinuierlich oder in sonstigen üblichen Verfahrensvarianten erfolgen (wie oben beschrieben). Die Umsetzungsdauer kann dabei z.B. im Bereich von Minuten bis mehreren Stunden oder tagen Liegen, und z.B. 1h bis 48 h betragen. Gegebenenfalls kann, wenn Enzymaktivität nicht kontinuierlich exprimiert wird, diese durch Zugabe eines geeigneten Induktors, nach Erreichen einer Zielzelldichte, z.B. von etwa OD₆₀₀ = 0,5 bis 1,0, eingeleitet werden.

Weitere mögliche geeignete Abwandlungen des mikrobiellen Herstellungsverfahrens hinsichtlich Fahrweise der Fermentation, Zusätze zum Medium, Enzymimmobilisierung und Isolierung der Wertstoffe sind auch dem folgenden Abschnitt betreffend "Herstellung der Enzyme bzw. Mutanten" zu entnehmen

### 3. Schritt Chemische Umsetzung von 12-Keto-UDCS zu UDCS

Die 12-Carbonylgruppe von 12-Keto-UDCS wird mittels Wolff-Kishner-Reduktion in an sich bekannter Weise entfernt, dadurch entsteht UDCS aus 12-Keto-UDCS. In der Reaktion wird zuerst die Carbonylgruppe mit Hydrazin zum Hydrazon umgesetzt. Anschließend wird das Hydrazon in Gegenwart einer Base (z.B. KOH) auf 200°C erhitzt, hierbei wird Stickstoff abgespaltet und UDCS entsteht.

### 6. Rekombinante Herstellung der Enzyme und Mutanten

Hierin beschrieben sind weiterhin Verfahren zur rekombinanten Herstellung erfindungsgemäßer Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Die hergestellten Mikroorganismen können kontinuierlich oder diskontinuierlich im batch- Verfahren (Satzkultivierung) oder im fed batch (Zulaufverfahren) oder repeated fed batch Verfahren (repetitives Zulaufverfahren) kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basische Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff-haltige Gasmischungen, wie z. B. Umgebungsluft, in die Kultur eingetragen. Die Temperatur der Kultur liegt normalerweise bei 20°C bis 45°C. Die Kultur wird so lange fortgesetzt, bis sich ein Maximum des gewünschten Produktes gebildet hat. Dieses Ziel wird normalerweise innerhalb von 10 Stunden bis 160 Stunden erreicht.

Die Fermentationsbrühe wird anschließend weiterverarbeitet. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Die Zellen können auch, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, lonenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

### 7. Enzymimmobilisierung

Die erfindungsgemäßen Enzyme können in den hierin beschriebenen Verfahren frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes" " in K. Drauz und H. Waldmann, Enzyme Catalysis in Organic Synthesis 2002, Vol.III, 991-1032, Wiley-VCH, Weinheim beschrieben.

### Experimenteller Teil:

Werden keine anderen Angaben gemacht, so können die im Rahmen der vorliegenden Erfindung durchgeführten Klonierungsschritte, wie z.B. Restriktionsspaltungen, Agarose Gelelektrophorese, Reinigung von DNA-Fragmenten, Transfer von Nukleinsäuren auf Nitrozellulose und Nylonmembranen, Verknüpfen von DNA-Fragmenten, Transformation von Mikroorganismen, Anzucht von Mikroorganismen, Vermehrung von Phagen und Sequenzanalyse rekombinanter DNA wie bei Sambrook et al. (1989) a.a.O. beschrieben durchgeführt werden.

### A. Allgemeine Angaben

### Materialien:

Die genomische DNA von *Collinsella aerofaciens* DSM 3979 (ATCC 25986, frühere Bezeichnung *Eubacterium aerofaciens*) wurde von der Deutschen Sammlung für Mikroorganismen und Zellkulturen (DSMZ) bezogen. DHCA, UDCS und 7-Keto-LCS sind an sich bekannte und in der Literatur beschriebene Ausgangsverbindungen. Alle übrigen Chemikalien wurden von Sigma-Aldrich und Fluka (Deutschland) bezogen. Sämtliche Restriktionsendonukleasen, die T4 DNA Ligase, die *Taq* DNA Polymerase, die *Phusion* DNA Polymerase und Isopropyl-β-D-1-thiogalactopyranosid (IPTG) wurden von Thermo Scientific (Deutschland) bezogen.

### Medien:

LB-Medium, enthaltend Trypton 10 g, Hefeextrakt 5 g, NaCl 10 g pro Liter Medium
Al-Medium (Auto-Induktions-Medium), enthaltend 24 g Hefeextrakt, 12 g Casein hydrolysat, 5 g Glycerin, 50 g Glucose, 20 g Lactose in 100 mM KPi pH7.0 pro Liter Medium.

### Sequenzen:

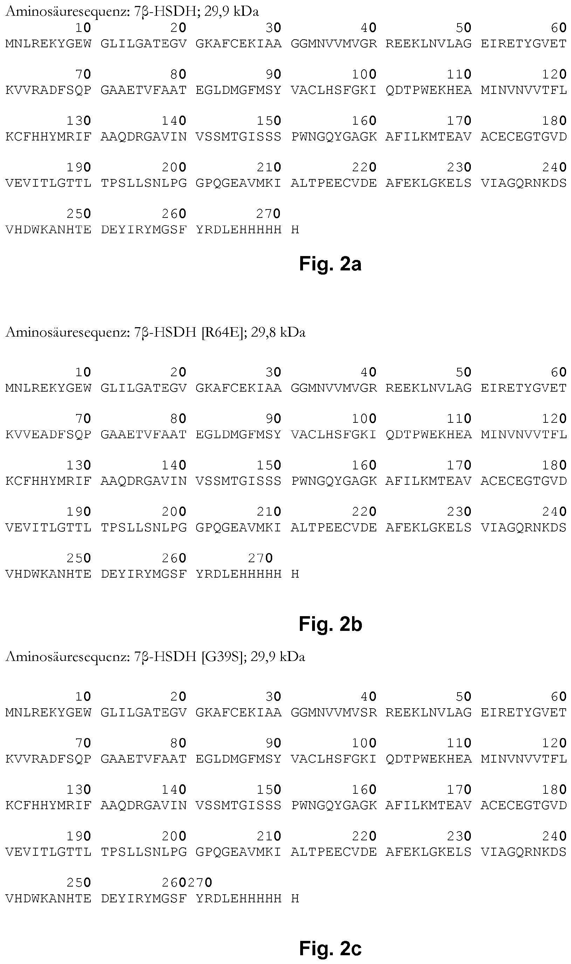

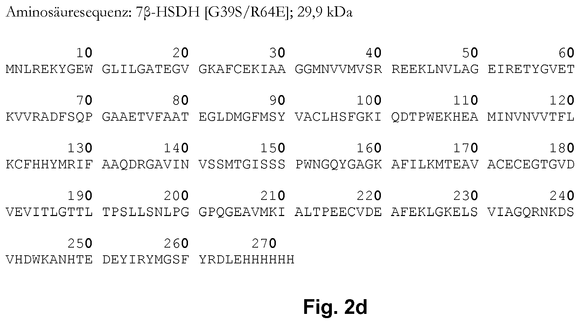

In Figur 2 Aufgeführt sind die Aminosäure-Sequenzen des Wildtyp-Enzyms der 7β-HSDH und von Mutanten, allerdings alle jeweils mit einem C-terminalen Hexa-Histidin-Tag versehen. In dieser Art sind alle Enzyme isoliert und charakterisiert worden.

### Beispiel 1: Herstellung der rekombinanten 7β-HSDH

### A) Plasmidtransformation:

Zur Expression rekombinanter 7β-HSDH-Enzyme (Wildtyp- und Mutantenenzyme) wurde der kommerziell verfügbare Expressionsvektor pET28a(+)verwendet, in den das jeweilige 7β-HSDH-Gen über die Schnittstellen der Restriktionsenzyme *Nco*I und *Xho*I in üblicher Weise einkloniert wurde. Der Expressionsvektor pET28a(+) ermöglicht es, dass direkt bei der Klonierung des HSDH-Gens eine Gensequenz an das HSDH-Gen angehängt wird, die für eine Abfolge von 6 Histidin-Molekülen kodiert. Diese Sequenz (als Hexahistidin-Tag oder His-Tag bezeichnet) erscheint dann nach der Expression C-terminal am HSDH-Protein. Das Ursprungs- bzw. Wildtyp-HSDH-Genstammt aus dem Bakterium *Collinsella aerofaciens* ATCC 25986, das Plasmid, das das jeweilige 7β-HSDH-Gen enthält, wird als pET28a(+)_7β-HSDH bezeichnet. Zur Transformation des pET28a(+)_7β-HSDH-Plasmids wurden 5 µl Ligationsansatz mit 100 µl kompetenten *E. coli*-Zellen BL21(DE3)Δ7α-HSDH versetzt und die Transformation nach Hanahan (J. Mol. Biol. (1983), Vol. 166, pp. 557) durchgeführt, wobei dieselben Schritte mit dem Plasmid, das das Wildtyp-Gen und mit dem Plasmid, das mutierte HSDH-Gene enthält, durchgeführt wurden. Der *E. coli*-Stamm BL21(DE3)Δ7α-HSDH, der hier regelmäßig verwendet wurde, zeichnet sich dadurch aus, dass in diesem Stamm das Gen für die 7α-HSDH deletiert wurde. Die genaue Charakterisierung der für diese Arbeiten verwendeten *E. coli*-Stämme ist in Tabelle 1 zusammengefasst.

**Tabelle 1: Verwendete Escherichia coli Stämme**

| **Stamm** | **Genotyp** |
|---|---|
| *Escherichia coli* DH5α | F-endA1 glnV44 thi-1 recA1 relA1 gyrA96 deoR nupG Φ80dlacZΔM15 Δ(lacZYA-argF)U169, hsdR17(rK - mK+),λ- |
| *Escherichia coli* BL21 (DE3) Δ7α-HSDH (= *E. coli* mit deletierter 7α-HSDH) | F-ompT gal dcm Ion hsdSB(rB-mB-) λ(DE3 [lacl lacUV5-T7 gene 1 ind1 sam7 nin5]) hshA- KanR+ |

### B) Expression und Zellvermehrung:

Zur Expression wurde der das Expressionskonstrukt (pET28a(+)_7β-HSDH-Plasmid) enthaltende *E. coli*-Stamm in LB-Medium (Trypton 10 g, Hefeextrakt 5 g, NaCl 10 g pro Liter) für 20Std. bei 25°C vermehrt, wobei das Medium 50 µg/ml Kanamycin enthält. Die Zellen wurden durch Zentrifugation (5,000×g, 30 min, 4°C) geerntet.

### C) Rohextrakt-Gewinnung:

Das Pellet wurde im Aufschlusspuffer (10 mM Imidazol, 50 mM Natriumphosphat, 300 mM NaCl, pH 8) resuspendiert, wobei pro 1 g Zellen (Feuchtmasse) 4 ml Puffer zugesetzt wurden. Die Zellen wurden dann unter konstanter Kühlung durch einminütige Ultraschallbehandlung (30 W Leistung, 50 % Arbeitsintervall und 1 min Pause) unter Verwendung eines Ultraschallgeräts Sonopuls HD2070 (Fa. Bandelin, Berlin, Deutschland) aufgeschlossen. Der Aufschluss wurde dreimal wiederholt. Die Zellsuspension wurde zentrifugiert (18,000×g, 30 min, 4°C), wobei der Überstand als zellfreier Rohextrakt bezeichnet wird.

### D) Reinigung des Enzyms:

Der His-Tag ermöglicht eine sehr einfache Reinigung des HSDH-Proteins, da diese Sequenz von speziellem Chromatographiematerial (NTA (Ni-Nitrilotriacetat)-modifiziertes Trägermaterial, beladen mit 2-wertigen Nickelionen; z.B. "His Pur Ni-NTA" (Fa. Nimagen B. V., Nijmegen, Niederlande) hoch-spezifisch gebunden wird. Dazu wird der zellfreie Rohextrakt auf dieses Material enthaltende Tropfsäule aufgetragen, die vorher mit dem Aufschlusspuffer (3 bis 5 Säulenvolumina) äquilibriert wurde. Schwach bindendes Protein wurde durch Waschen mit 3 bis 5 Säulenvolumina Waschpuffer (20 mM Imidazol, 50 mM Natriumphosphat, 300 mM NaCl, pH 8) entfernt. Das His-Tag-7β-HSDH-Protein wurde mit Imidazol-haltigem Elutionspuffer (250 mM Imidazol, 50 mM Natriumphosphat, 300 mM NaCl, pH 8) eluiert. Das Verfahren wurde bei Raumtemperatur durchgeführt. Durch Umpufferung wurde das enthaltende Imidazol entfernt.

Die Proteinkonzentration wurde nach der von Bradford beschriebenen Methode bestimmt (100 µl Probe mit 900 µl Bradford Reagenz mischen, für mind. 15 min im Dunkeln inkubieren, dann bei 595 nm gegen eine mit Rinderserum-Albumin erstellte Kalibriergerade bestimmt). Für eine Analyse mittels SDS-PAGE (SDS-Polyacrylamid-Gelelektrophorese) wurde das Gel mit Coomassie Brilliant Blue angefärbt.

### E) Aktivitätsbestimmung:

Die Aktivität der 7β-HSDH und der Mutanten wurde mit einem photometrischen Assay bestimmt, wobei die Abnahme der Extinktion wird bei 340 nm über einen Zeitraum von 30 Sekunden gemessen wurde. Die Reaktionslösung enthielt in einem Gesamtvolumen von 1 ml: 874 µl 50 mM Kaliumphosphat (KPi) Puffer, pH 8.0; 100 µl 100 mM einer DHCA-Lösung (DHCA gelöst in 50 mM KPi, pH 8); 10 µl der Enzymlösung (gfls. verdünnt); 16 µl einer 12,5 mM NADPH-Lösung (gelöst in dest. H₂O). Die Aktivität wird im Folgenden in Units (U) angegeben, wobei 1 U der Abnahme von 1 µMol NADPH / min entspricht.

### Beispiel 2: Herstellung von 7β-HSDH Mutanten in Aminosäure-Position 64 und deren Charakterisierung

### A) Primer:

Für die positionsgerichtete Mutagenese der 7β-HSDH wurden die unten angegebenen Mutageneseprimer verwendet (siehe Tabelle 2). Die Primer wurden auf Grundlage der 7β-HSDH-Gensequenz dahingehend ausgewählt, dass sie den gewünschten Aminosäurenaustausch bewirken. Zur Erzeugung der Mutanten wurden folgende Primerpaare verwendet:

**Tabelle 2: Primer für die positionsgerichtete Mutagenese der 7β-HSDH an Position 64**

| **Bezeichnung** | **Austausch** | **Primer** | **SEQ ID NO** | **5' → 3' Sequenz** |
|---|---|---|---|---|
| R64E_ for | R64E | forward | 12 | ACCAAGGTCGTGGAGGCCGACTTTAGC |
| R64E rev | | reverse | 13 | GCTAAAGTCGGCCTCCACGACCTTGGT |
| R64D_ for | R64D | forward | 14 | ACCAAGGTCGTGGACGCCGACTTTAGC |
| R64D_rev | | reverse | 15 | GCTAAAGTCGGCGTCCACGACCTTGGT |
| R64T_ for | R64T | forward | 16 | ACCAAGGTCGTGACGGCCGACTTTAGC |
| R64T_rev | | reverse | 17 | GCTAAAGTCGGCCGTCACGACCTTGGT |
| R64W_ for | R64W | forward | 18 | ACCAAGGTCGTGTGGGCCGACTTTAGC |
| R64W_rev | | reverse | 19 | GCTAAAGTCGGCCCACACGACCTTGGT |
| R64Y_ for | R64Y | forward | 20 | ACCAAGGTCGTGTACGCCGACTTTAGC |
| R64Y_rev | | reverse | 21 | GCTAAAGTCGGCGTACACGACCTTGGT |
| R64F_ for | R64F | forward | 22 | ACCAAGGTCGTGTTCGCCGACTTTAGC |
| R64F_rev | | reverse | 23 | GCTAAAGTCGGCGAACACGACCTTGGT |
| R64C_ for | R64C | forward | 24 | ACCAAGGTCGTGTGCGCCGACTTTAGC |
| R64C_rev | | reverse | 25 | GCTAAAGTCGGCGCACACGACCTTGGT |
| R64N_ for | R64N | forward | 26 | ACCAAGGTCGTGAACGCCGACTTTAGC |
| R64N_rev | | reverse | 27 | GCTAAAGTCGGCGTTCACGACCTTGGT |
| R64Q_ for | R64Q | forward | 28 | ACCAAGGTCGTGCAGGCCGACTTTAGC |
| R64Q_rev | | reverse | 29 | GCTAAAGTCGGCCTGCACGACCTTGGT |
| R64H_ for | R64H | forward | 30 | ACCAAGGTCGTGCACGCCGACTTTAGC |
| R64H_rev | | reverse | 31 | GCTAAAGTCGGCGTGCACGACCTTGGT |
| R64K_for | R64K | forward | 32 | ACCAAGGTCGTGAAGGCCGACTTTAGC |
| R64K_rev | | reverse | 33 | GCTAAAGTCGGCCTTCACGACCTTGGT |
| R64I_ for | R64I | forward | 34 | ACCAAGGTCGTGATCGCCGACTTTAGC |
| R64I_rev | | reverse | 35 | GCTAAAGTCGGCGATCACGACCTTGGT |
| R64G_ for | R64G | forward | 36 | ACCAAGGTCGTGGGCGCCGACTTTAGC |
| R64G_rev | | reverse | 37 | GCTAAAGTCGGCGCCCACGACCTTGGT |
| R64A_ for | R64A | forward | 38 | ACCAAGGTCGTGGCCGCCGACTTTAGC |
| R64A_rev | | reverse | 39 | GCTAAAGTCGGCGGCCACGACCTTGGT |
| R64V_ for | R64V | forward | 40 | ACCAAGGTCGTGGTCGCCGACTTTAGC |
| R64V_rev | | reverse | 41 | GCTAAAGTCGGCGACCACGACCTTGGT |
| R64L for | R64L | forward | 42 | ACCAAGGTCGTGCTCGCCGACTTTAGC |
| R64L_rev | | reverse | 43 | GCTAAAGTCGGCGAGCACGACCTTGGT |
| R64S_ for | R64S | forward | 44 | ACCAAGGTCGTGAGCGCCGACTTTAGC |
| R64S_rev | | reverse | 45 | GCTAAAGTCGGCGCTCACGACCTTGGT |

### B) QuikChange®-PCR:

Nachdem eine erste Mutante, in der die Aminosäure Arginin in Position 64 durch Asparaginsäure ersetzt worden war, eine deutlich höhere Aktivität als das Wildtyp-Enzym zeigte, sollte in dieser Position weitere proteinogen Aminosäuren eingebaut und diese verschiedenen Mutanten bezüglich ihrer Aktivität getestet werden. Ein gezielter Austausch einer Aminosäure kann mit Hilfe der "QuikChange®-PCR"-Methode erreicht werden. Dazu wurde folgende PCR-Reaktion (Reaktionsansatz s. Tab 3) durchgeführt: Zuerst erfolgte ein 2 min langer initialer Denaturierungsschritt bei 95°C, danach wurden 20 Zyklen von Denaturierung (30 s bei 95°C), Primerhybridisierung (1 min bei 60 - 68°C) und Elongation (13 min bei 68°C) durchgeführt. Als letzter Schritt erfolgte eine finale Elongation von 10 min bei 68°C bevor die Polymerasekettenreaktion durch Kühlen auf 4°C beendet wurde. Als Templat wurde ein pET28a-Vektor mit dem Gen der 7β-HSDH (Wildtyp) verwendet.

**Tabelle 3: PCR-Ansatz für die Erzeugung der verschiedenen 7β-HSDH Varianten**

| **PCR-Reaktionsansatz** | |
|---|---|
| Puffer (10x) | 5,0 µl |
| dNTP-Mix (10 mM) | 1,5µl |
| Forward-Primer (10 pmol/µl) | 5,0 µl |
| Revers-Primer (10 pmol/µl) | 5,0 µl |
| Template | 1,0 µl |
| Pfu Polymerase | 0,5 µl |
| DMSO | 2,0 µl |
| ddH₂O | 30,0µl |
| | 50,0 µl |

Um Aminosäuren in Proteinsequenzen gezielt austauschen zu können, wird die DNA-Sequenz des entsprechenden Gens positionsgerichtet mutiert. Hierzu werden zueinander komplementäre Primer genutzt, die die gewünschte Mutation in ihrer Sequenz tragen. Als Template dient N6-adeninmethylierte, doppelsträngige Plasmid-DNA, die das zu mutierende Gen trägt. N6-adeninmethylierte Plasmid-DNA werden aus einem dam+ *E. coli-*Stamm wie zum Beispiel *E. coli* DH5α isoliert.

Die Polymerasekettenreaktion wird wie oben beschrieben durchgeführt. Dabei werden die Primer komplementär zum Template verlängert, wodurch Plasmide mit der gewünschten Mutation entstehen, die einen Strangbruch aufweisen. Anders als bei anderen PCR-Reaktionen steigt die DNA-Ausbeute hierbei nur linear, da neu gebildete DNA-Moleküle nicht als Template für die PCR-Reaktion dienen können.

Nach Abschluss der PCR-Reaktion wurde das PCR-Produkt mittels PCR-Purification-Kit (Analytik Jena AG, Jena, Deutschland) aufgereinigt und die parentale, N6-adeninmethylierte DNA mit Hilfe des Restriktionsenzyms *dpn*I verdaut. Dieses Enzym hat die Besonderheit, dass es unspezifisch N6-adeninmethylierte DNA restringiert, jedoch nicht die neu gebildete, nichtmethylierte DNA. Die Restriktion erfolgte, indem 1 µl *dpn*I zum PCR-Reaktionsansatz hinzu gegeben und für 2 h oder über Nacht bei 37°C inkubiert wurde. Zur Transformation von 100 µl chemisch-kompetenten DH5α-Zellen wurden 7,5 µl dieses Ansatzes eingesetzt.

### C) Aktivitätswerte der Enzymmutanten:

Die Aktivitätsmessung (s. Beispiel 1) der Mutanten, die in Position 64 mutagenisiert worden sind, ergab die in der folgenden Tabellen 4 aufgeführten Werte. Dabei bedeutet die Schreibweise [R64E] in der ersten Tabellenspalte, dass bei der betrachteten Mutante das Arginin (R) in Position 64 der Proteinsequenz durch Glutaminsäure (E) ersetzt wurde. Die jeweilige Aminosäure wird dabei nach dem internationalen Ein-Buchstaben-Code abgekürzt. Analog bedeutet [R64D], dass in dieser Position eine Asparaginsäure eingefügt wurde.

**Tabelle 4: Aktivitäten der verschiedenen 7β-HSDH-Varianten, die an der Position 64 verändert wurden.**

| **Mutante** | **Volumetrische Aktivität [U/ml]** | **spezifische Aktivität [U/mg]** |
|---|---|---|
| 7β-HSDH (WT) | 96,8 | 8,7 |
| 7β-HSDH [R64E] | 892,7 | 60,2 |
| 7β-HSDH [R64D] | 641,6 | 32,1 |
| 7β-HSDH [R64T] | 450,5 | 27,4 |
| 7β-HSDH [R64L] | 334 | 20,9 |
| 7β-HSDH [R64S] | 333,7 | 20,1 |
| 7β-HSDH [R64P] | 402,0 | 19,1 |
| 7β-HSDH [R64V] | 407,7 | 15,1 |
| 7β-HSDH [R64K] | 296,5 | 15,1 |
| 7β-HSDH [R64C] | 269,7 | 14,19 |
| 7β-HSDH [R64A] | 273 | 13,77 |
| 7β-HSDH [R64G] | 223 | 12,36 |
| 7β-HSDH [R64Q] | 217 | 12,2 |
| 7β-HSDH [R64F] | 216 | 10,5 |
| 7β-HSDH [R64W] | 214 | 10,1 |
| 7β-HSDH [R64I] | 171 | 9,83 |
| 7β-HSDH [R64Y] | 220,3 | 9,7 |
| 7β-HSDH [R64H] | 97,8 | 5,34 |
| 7β-HSDH [R64N] | 3,4 | 0,6 |

### D) Michaelis-Menten-Kinetiken

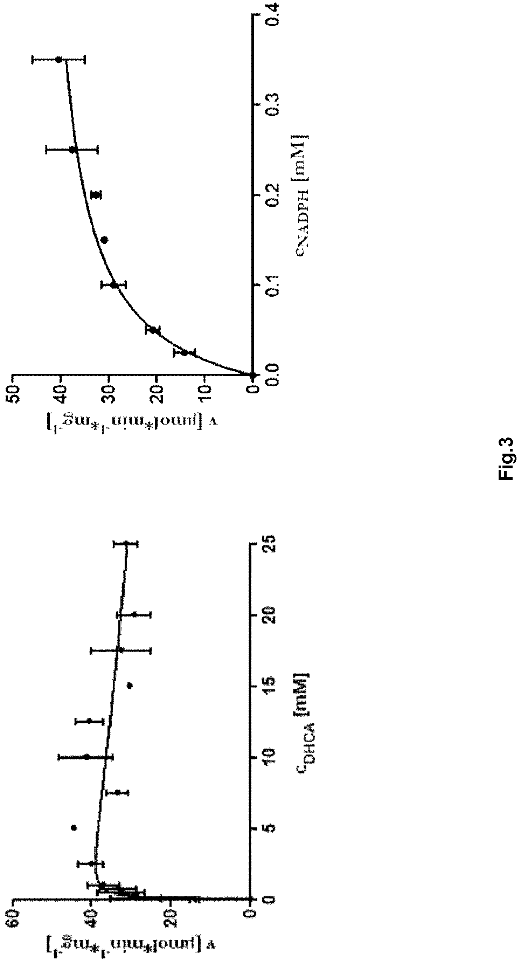

Die besten Mutanten der jeweiligen Position wurden entsprechend Beispiel 1D gereinigt und die kinetische Konstanten vₘₐₓ und K_{M} für das Substrat DHCA und das Coenzym NADPH ermittelt. In Figur 3 sind die Verlaufsdiagramme der Kinetiken abgebildet und in Tabelle 5 die kinetischen Konstanten aufgelistet.

**Tabelle 5: Kinetische Konstanten für das aufgereinigte Enzym 7β-HSDH [R64E] für das Substrat DHCA und den Kofaktor NADPH. (x = keine Inhibierung)**

| ***Enzym*** | ***Substrat*** | ***vₘₐₓ(U*/*mg)*** | ***K_{M}(µM)*** | ***K_{I}(mM)*** |
|---|---|---|---|---|
| 7β-HSDH [R64E] | DHCA | 41,56 ± 1,9 | 81,01 ± 18,9 | 74,23 ± 24,9 |
| | NADPH | 60,31 ± 15,6 | 45,48 ± 3,5 | x |
| 7β-HSDH (WT) | DHCA | 8,7 ± 0,2 | 20,5 ± 2,9 | 79,8 ± 16,4 |
| | NADPH | 8,8 ± 0,2 | 15,2 ± 2,2 | x |

Aus Tabelle 5 geht hervor, dass die maximale Geschwindigkeit gegenüber dem Wildtypenzym um ca. das 5-fache gesteigert werden konnte. Der K_{M}-Wert ist gleich geblieben. Die Substratinhibierung ist deutlich schwächer geworden.

### Beispiel 3: Herstellung von 7β-HSDH Mutanten in Aminosäure-Position 39 und deren Charakterisierung

### A) Primer:

Für die positionsgerichtete Mutagenese der 7β-HSDH wurden die in Tabelle 6 aufgeführten Mutageneseprimer verwendet. Die Primer wurden auf Grundlage der 7β-HSDH-Gensequenz dahingehend ausgewählt, dass sie den gewünschten Aminosäurenaustausch bewirken. Zur Erzeugung der Mutanten wurden folgende Primerpaare verwendet:

**Tabelle 6: Primer für die positionsgerichtete Mutagenese der 7β-HSDH an Position 39**

| ***Bezeichnung*** | ***Austausch*** | ***Primer*** | ***SEQ ID NO*** | ***5'* -> *3' Sequenz*** |
|---|---|---|---|---|
| G39S_for | G39S | forward | 54 | GTCGTCATGGTCAGCCGTCGCGAGGAG |
| G39S_rev | | reverse | 55 | CTCCTCGCGACGGCTGACCATGACGAC |
| G39V_for | G39V | forward | 56 | GTCGTCATGGTCGTCCGTCGCGAGGAG |
| G39V_rev | | reverse | 57 | CTCCTCGCGACGGACGACCATGACGAC |
| G39I_for | G39I | forward | 58 | GTCGTCATGGTCATCCGTCGCGAGGAG |
| G39I_rev | | reverse | 59 | CTCCTCGCGACGGATGACCATGACGAC |
| G39C_for | G39C | forward | 60 | GTCGTCATGGTCTGCCGTCGCGAGGAG |
| G39C_rev | | reverse | 61 | CTCCTCGCGACGGCAGACCATGACGAC |
| G39K_for | G39K | forward | 62 | GTCGTCATGGTCAAGCGTCGCGAGGAG |
| G39K_rev | | reverse | 63 | CTCCTCGCGACGCTTGACCATGACGAC |
| G39Y_for | G39Y | forward | 64 | GTCGTCATGGTCTACCGTCGCGAGGAG |
| G39Y_ rev | | reverse | 65 | CTCCTCGCGACGGTAGACCATGACGAC |
| G39F_for | G39F | forward | 66 | GTCGTCATGGTCTTCCGTCGCGAGGAG |
| G39F_rev | | reverse | 67 | CTCCTCGCGACGGAAGACCATGACGAC |
| G39R_for | G39R | forward | 68 | GTCGTCATGGTCCGCCGTCGCGAGGAG |
| G39R_rev | | reverse | 69 | CTCCTCGCGACGGCGGACCATGACGAC |

### B) QuikChange®-PCR:

Der gezielte Austausch von Glycin in Position 39 gegen Serin wurde wie in Beispiel 2B beschrieben mittels QuikChange®-PCR durchgeführt.

### C) Aktivitätswerte der Enzymmutanten:

Die Aktivitätsmessung (s. Beispiel 1) der Mutante, die in Position 39 Serin an Stelle von Glycin enthält (7β-HSDH [G39S]), ergab eine volumetrische Aktivität von 735 U/ml und eine spezifische Enzymaktivität von 52,9 U/mg Protein, während das Wildtyp-Enzym dazu im Vergleich eine volumetrische Aktivität von 96,8 U/ml und eine spezifische Aktivität von 8,7 U/mg aufwies.

### D) Michaelis-Menten-Kinetiken

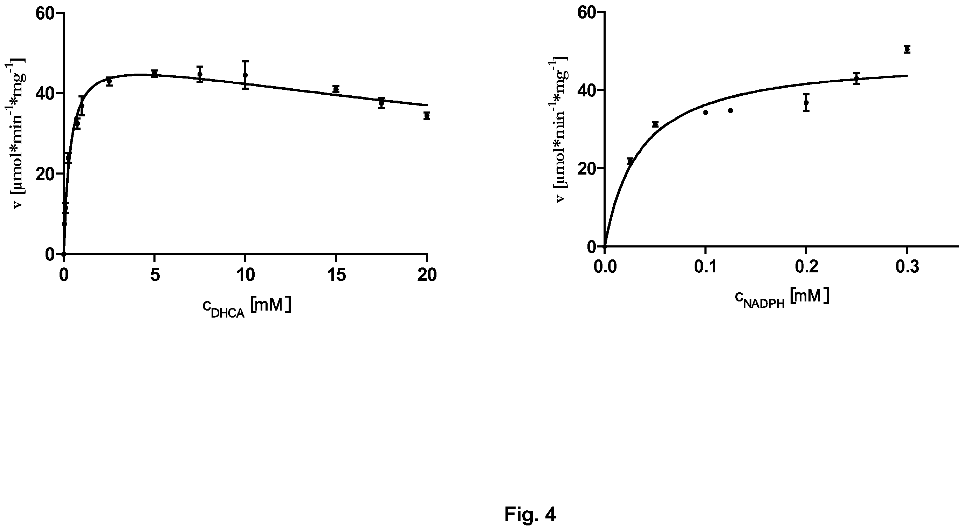

Die kinetischen Parameter Konstanten vₘₐₓ und K_{M} wurden mit aufgereinigtem Enzym aufgenommen. In Figur 4 sind die Verlaufsdiagramme der Kinetiken abgebildet und in Tabelle 7 die kinetischen Konstanten aufgelistet.

**Tabelle 7: Kinetische Konstanten für das aufgereinigte Enzym 7β-HSDH [G39S] für das Substrat DHCA und den Kofaktor NADPH. (x = keine Inhibierung)**

| ***Enzym*** | ***Substrat*** | ***vₘₐₓ(U*/*mg)*** | ***K_{M} (µM)*** | ***K_{I} (mM)*** |
|---|---|---|---|---|
| 7β-HSDH [G39S] | DHCA | 52,0 ± 1,9 | 352,4 ± 42,7 | 51,8 ± 10,1 |
| | NADPH | 48,8 ± 2,2 | 34,5 ± 6,6 | x |
| 7β-HSDH | DHCA | 8,7 ± 0,2 | 20,5 ± 2,9 | 79,8 ± 16,4 |
| | NADPH | 8,8 ± 0,2 | 15,2 ± 2,2 | x |

Aus Tabelle 7 geht hervor, dass die maximale Geschwindigkeit gegenüber dem Wildtypen um ca. das 6-fache gesteigert werden konnte. Der K_{M}-Wert ist dafür um das 4-fache schlechter geworden. Die Substratinhibierung ist genau wie bei der Mutante in Position 64 etwas schwächer geworden, wurde aber nicht eliminiert.

### Beispiel 4: Herstellung von 7β-HSDH Mutanten in Aminosäure-Position 17 und deren Charakterisierung

### A) Primer:

Für die positionsgerichtete Mutagenese der 7β-HSDH wurden die in Tabelle 8 aufgeführten Mutageneseprimer verwendet. Zur Erzeugung der Mutanten wurden folgende Primerpaare verwendet:

**Tabelle 8: Primer für die positionsgerichtete Mutagenese der 7β-HSDH an Position 17**

| ***Bezeichnung*** | ***Austausch*** | ***Primer*** | ***SEQ ID NO*** | ***5' -> 3' Sequenz*** |
|---|---|---|---|---|
| T17F_for | T17F | forward | 46 | ATCCTGGGCGCGTTCGAGGGCGTCGGC |
| T17F_rev | | reverse | 47 | GCCGACGCCCTCGAACGCGCCCAGGAT |
| T171_for | T17I | forward | 48 | ATCCTGGGCGCGATCGAGGGCGTCGGC |
| T171_rev | | reverse | 49 | GCCGACGCCCTCGATCGCGCCCAGGAT |
| T17A_for | T17A | forward | 50 | ATCCTGGGCGCGGCCGAGGGCGTCGGC |
| T17A_rev | | reverse | 51 | GCCGACGCCCTCGGCCGCGCCCAGGAT |
| T17S_for | T17S | forward | 52 | TCCTGGGCGCGAGCGAGGGCGTC |
| T17S_rev | | reverse | 53 | GACGCCCTCGCTCGCGCCCAGGA |

### B) QuikChange®-PCR:

Die gezielten Austausche von Threonin in Position 17 gegen die in Tabelle 8 aufgeführten Aminosäuren wurden wie in Beispiel 2B beschrieben mittels QuikChange®-PCR durchgeführt.

### C) Aktivitätswerte der Enzymmutanten:

Die Aktivitätsmessungen (s. Beispiel 1) der Mutanten, die in Position 17 eine andere Aminosäure als Threonin enthalten, sind in Tabelle 9 zusammengefasst.

**Tabelle 9: Aktivitäten der verschiedenen 7β-HSDH-Varianten, die an der Position 17 verändert wurden.**

| **Mutante** | **Volumetrische Aktivität [U/ml]** | **spezifische Aktivität [U/mg]** |
|---|---|---|
| 7β-HSDH (WT) | 96,8 | 8,7 |
| 7β-HSDH [T17F] | 645,2 | 46,1 |
| 7β-HSDH [T17A] | 541 | 20,3 |
| 7β-HSDH [T17I] | 299,8 | 18,4 |
| 7β-HSDH [T17S] | 580 | 17,2 |

### Beispiel 5: Herstellung von 7β-HSDH Mutanten, die in mehreren Positionen Aminosäure-Austausche enthalten und deren Charakterisierung

Neben den in den Beispielen 2 - 4 beschriebenen Einzelmutanten kann man Mutationen auch vorteilhaft kombinieren. Als Beispiel wurde eine Doppelmutante erzeugt, bei der gleichzeitig die Positionen 39 und 64 verändert wurden. Dabei wurde der beste Aminosäure-Austausch in Position 39 ([G39S]) kombiniert mit dem besten Austausch in Position 64 ([R64E]).

### A) Erzeugung und Aktivitätswerte für die Doppelmutante

Die Methode zur Gewinnung der Doppelmutante und der Aktivitätstest richtete sich dabei nach den Methoden, wie sie unter 2B und 2C beschrieben sind.

In Tab. 10 sind die Aktivitätswerte für die Doppelmutante im Vergleich zu den Werten für das Wildtypenzym zusammengestellt.

**Tabelle 10: Aktivitätswerte der 7β-HSDH-Doppelmutante [G39S/R64E] im Vergleich zum Wildtypenzym.**

| **Mutante** | **Volumetrische Ak tivität [U/ml]** | **spezifische Aktivität [U/mg]** |
|---|---|---|
| 7β-HSDH (WT) | 96,8 | 8,7 |
| 7β-HSDH [G39S/R64E] | 1115,0 | 57,5 |

### B) SDS-Polyacrylamid-Gelelektrophorese (SDS-PAGE)

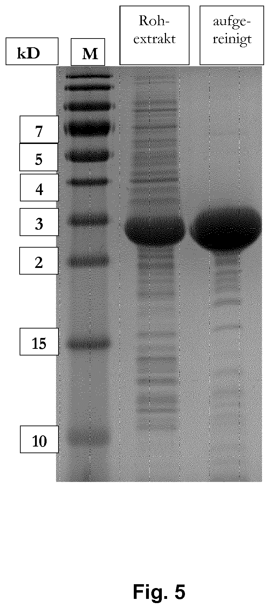

Eine SDS-PAGE wurde durchgeführt, um die Expressionsleistung der heterologen Expression bewerten zu können. Als Beispiel wurde die Doppelmutante aufgetragen. In Figur 5 ist ein solches SDS-Gel dargestellt, das durch Auftragung des Zell-Rohextrakts zeigt, dass diese Mutante eine sehr gute Überexpression zeigt; die dicke Bande, die bei ca. 30 kDa läuft, belegt, dass das intrazelluläre lösliche Protein in hohem Maß nur aus der Doppelmutante besteht. Zusätzlich belegt das Gel den Reinheitsgrad der aufgereinigten Doppelmutante. Das Molekulargewicht der Mutante beträgt ca. 29,9k Da.

### C) Michaelis-Menten-Kinetiken

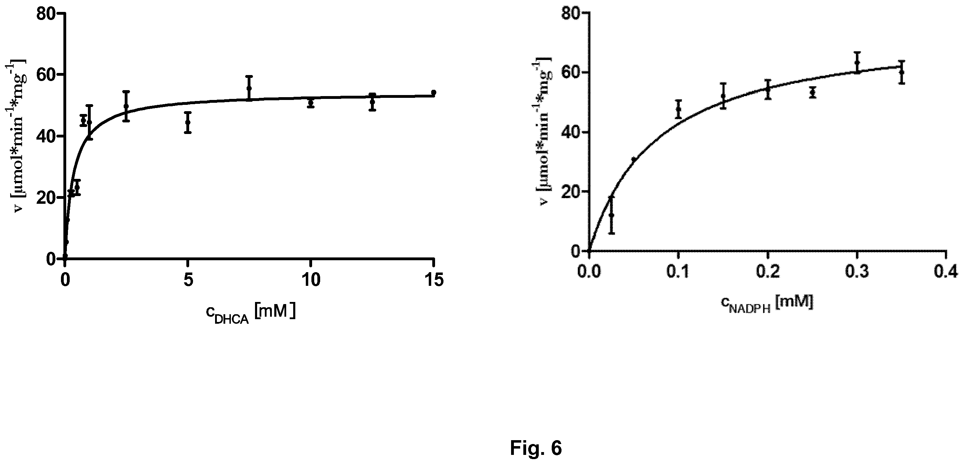

Die Mutante [G39S/R64E] wurde aufgereinigt (siehe Punkt 3) und die kinetischen Parameter für das gereinigte Enzym bestimmt. In Figur 6 sind die Verlaufsdiagramme abgebildet und in Tabelle 11 die kinetischen Konstanten aufgelistet.

**Tabelle 11: Kinetische Konstanten für das aufgereinigte Enzym 7β-HSDH [G39S/R64E] für das Substrat DHCA und den Kofaktor NADPH. (x = keine Inhibierung)**

| ***Enzym*** | ***Substrat*** | ***vₘₐₓ(U*/*mg)*** | ***K_{M} (µM)*** | ***K_{I} (mM)*** |
|---|---|---|---|---|
| 7β-HSDH [G39S/R64E] | DHCA | 54,3 ± 1,6 | 349,3 ± 5,23 | x |
| | NADPH | 75,5 ± 4,8 | 76,6 ± 15,9 | x |

Aus dem Verlaufsdiagramm (für das Substrat DHCA) in Figur 6 fällt auf, das die Kombination der Positionen 39 und 64 zur Folge hatte, dass die 7β-HSDH keine Substratinhibierung mehr aufweist. Weiterhin konnte die Aktivität um ca. 7-fache gesteigert werden.

Zusammenfassung der erfindungsgemäß beispielhaft erzielten Verbesserungen/Änderungen):

| 7β-Typ | Vmax (DHCA) | Substratinhibition Ki | Kofaktor-Spezifität |
|---|---|---|---|
| WT | | | NADPH |
| R64E | + 5-fach besser | + verringert | NADPH |
| G39S | + 6-fach besser | + verringert | NADPH |
| T17F | + 5-fach besser | n.b. | NADPH/ geringe Aktivität mit NADH |
| G39S / R64E | + 7-fach besser | ++ beseitigt | NADPH |

| | | | |
|---|---|---|---|
| n.b. = nicht bestimmt (Angaben im Vergleich zum Wildtyp) | | | |

### Zuordnung von SEQ ID NOs:

| SEQ ID NO: | Beschreibung | Typ |
|---|---|---|
| 1 | 7β-HSDH C. aerofaciens (Wildtyp) | NS |
| 2 | 7β-HSDH C. aerofaciens (Wildtyp) | AS |
| 3 | 7β-HSDH C. aerofaciens (Wildtyp) (mit His-Tag) | AS |
| 4 | 7β-HSDH R64E Mutante(mit His-Tag) | AS |
| 5 | 7β-HSDH G39S Mutante(mit His-Tag) | AS |
| 6 | 7β-HSDH G39S R64E Mutante(mit His-Tag) | AS |
| 7 | GDH (B. subtilis) | NS |
| 8 | GDH (B. subtilis) | AS |
| 9 | 3α-HSDH (C. testosteroni) | NS |
| 10 | 3α-HSDH (C. testosteroni) | AS |
| 11 | FDH (Wildtyp) (M.vaccae) | AS |
| 12 | PCR Primer | NS |
| 13 | PCR Primer | NS |
| 14 | PCR Primer | NS |
| 15 | PCR Primer | NS |
| 16 | PCR Primer | NS |
| 17 | PCR Primer | NS |
| 18 | PCR Primer | NS |
| 19 | PCR Primer | NS |
| 20 | PCR Primer | NS |
| 21 | PCR Primer | NS |
| 22 | PCR Primer | NS |
| 23 | PCR Primer | NS |
| 24 | PCR Primer | NS |
| 25 | PCR Primer | NS |
| 26 | PCR Primer | NS |
| 27 | PCR Primer | NS |
| 28 | PCR Primer | NS |
| 29 | PCR Primer | NS |
| 30 | PCR Primer | NS |
| 31 | PCR Primer | NS |
| 32 | PCR Primer | NS |
| 33 | PCR Primer | NS |
| 34 | PCR Primer | NS |
| 35 | PCR Primer | NS |
| 36 | PCR Primer | NS |
| 37 | PCR Primer | NS |
| 38 | PCR Primer | NS |
| 39 | PCR Primer | NS |
| 40 | PCR Primer | NS |
| 41 | PCR Primer | NS |
| 42 | PCR Primer | NS |
| 43 | PCR Primer | NS |
| 44 | PCR Primer | NS |
| 45 | PCR Primer | NS |
| 46 | PCR Primer | NS |
| 47 | PCR Primer | NS |
| 48 | PCR Primer | NS |
| 49 | PCR Primer | NS |
| 50 | PCR Primer | NS |
| 51 | PCR Primer | NS |
| 52 | PCR Primer | NS |
| 53 | PCR Primer | NS |
| 54 | PCR Primer | NS |
| 55 | PCR Primer | NS |
| 56 | PCR Primer | NS |
| 57 | PCR Primer | NS |
| 58 | PCR Primer | NS |
| 59 | PCR Primer | NS |
| 60 | PCR Primer | NS |
| 61 | PCR Primer | NS |
| 62 | PCR Primer | NS |
| 63 | PCR Primer | NS |
| 64 | PCR Primer | NS |
| 65 | PCR Primer | NS |
| 66 | PCR Primer | NS |
| 67 | PCR Primer | NS |
| 68 | PCR Primer | NS |
| 69 | PCR Primer | NS |

| | | |
|---|---|---|
| AS = Aminosäuresequenz NS = Nukleinsäuresequenz | | |

## Patentansprüche

1. 7β-Hydroxysteroiddehydrogenase (7β-HSDH), welche wenigstens die stereospezifische enzymatische Reduktion eines 7-Ketosteroids zum korrespondierenden 7-Hydroxysteroid katalysiert, wobei das Enzym eine Mutation in Position 64 von SEQ ID NO:2 oder in den korrespondierenden Sequenzpositionen einer davon abgeleiteten Aminosäuresequenz mit wenigstens 90 % Sequenzidentität zu SEQ ID NO:2 umfasst;
wobei die Mutation in Position 64 die Mutation R64X₁ ist,
worin X₁ für E, D, T, L, S, P, V, K, C, A, G, Q, F, W, I, oder Y steht;
und
wobei das Enzym im Vergleich zur 7β-HSDH mit SEQ ID NO:2 folgendes Eigen-schaftsprofil zeigt:
a) eine erhöhte spezifische Aktivität (Vmax [U/mg]) für NADPH bei der enzymatischen Reduktion von Dehydrocholsäure (DHCA) mit NADPH als Cofaktor; und zusätzlich gegebenenfalls:
b) eine erhöhte spezifische Aktivität (Vmax [U/mg]) für DHCA bei der enzymatischen Reduktion von DHCA mit NADPH als Cofaktor;
c) eine verringerte Substratinhibition durch DHCA,
d) eine veränderte Cofaktorspezifität bezüglich NADH und NADPH,
e) wobei diese Eigenschaften b), c) und d) einzeln oder in beliebiger Kombination vorliegen können.

2. 7β-Hydroxysteroiddehydrogenase (7β-HSDH), welche wenigstens die stereospezifische enzymatische Reduktion eines 7-Ketosteroids zum korrespondierenden 7-Hydroxysteroid katalysiert, wobei das Enzym eine Mutation in Position 64 von SEQ ID NO:2 oder in den korrespondierenden Sequenzpositionen einer davon abgeleiteten Aminosäuresequenz mit wenigstens 80 % Sequenzidentität zu SEQ ID NO:2, und zusätzlich wenigstens eine Mutation im Sequenzmotiv VMVGRRE gemäß Position 36 bis 42 von SEQ ID NO:2 oder in dem korrespondierenden Sequenzmotiv einer davon abgeleiteten Aminosäuresequenz mit wenigstens 80 % Sequenzidentität zu SEQ ID NO:2 aufweist;
wobei die Mutation in Position 64 die Mutation R64X₁ ist,
worin X₁ für E, D, T, L, S, P, V, K, C, A, G, Q, F, W, I, oder Y steht;
und
wobei das Enzym im Vergleich zur 7β-HSDH mit SEQ ID NO:2 folgendes Eigen-schaftsprofil zeigt:
a) eine erhöhte spezifische Aktivität (Vmax [U/mg]) für NADPH bei der enzymatischen Reduktion von Dehydrocholsäure (DHCA) mit NADPH als Cofaktor; und zusätzlich gegebenenfalls:
b) eine erhöhte spezifische Aktivität (Vmax [U/mg]) für DHCA bei der enzymatischen Reduktion von DHCA mit NADPH als Cofaktor;
c) eine verringerte Substratinhibition durch DHCA,
d) eine veränderte Cofaktorspezifität bezüglich NADH und NADPH,
e) wobei diese Eigenschaften b), c) und d) einzeln oder in beliebiger Kombination vorliegen können.

3. 7β-HSDH nach Anspruch 2, die zusätzlich die Aminosäuresequenz-Mutation
G39X₃
aufweist,
wobei X₃ für einen von Glycin (G) verschiedenen Aminosäurerest steht.

4. 7β-HSDH nach einem der Ansprüche 2 und 3, ausgewählt unter
den Doppelmutanten
R64X₁/G39X₃
worin
X₁ für E, D, T, L, S, P, V, K, C, A, G, Q, F, W, I, Y, H oder N steht; und
X₃ für S, A, V, I, L, C, K, Y, F oder R steht.

5. 7β-HSDH nach Anspruch 4, wobei die Doppelmutante ausgewählt ist unter: (G39S/R64E); (G39S/R64D); (G39S/R64T); (G39S/R64L); (G39S/R64S); (G39S/R64P); (G39S/R64V); (G39A/R64E); (G39A/R64D); (G39A/R64T); (G39A/R64S); (G39A/R64L); (G39A/R64P); (G39A/R64V).

6. Nukleotidsequenz kodierend für eine 7β-HSDH nach einem der vorhergehenden Ansprüche.

7. Expressionskassette, umfassend unter der Kontrolle wenigstens einer regulativer Sequenz wenigstens eine Nukleotidsequenz nach Anspruch 6.

8. Expressionsvektor, umfassend wenigstens eine Expressionskassette nach Anspruch 7.

9. Rekombinanter Mikroorganismus, der wenigstens eine Nukleotidsequenz gemäß Anspruch 6 oder wenigstens eine Expressionskassette nach Anspruch 7 oder wenigstens einen Expressionsvektor nach Anspruch 8 trägt.

10. Rekombinanter Mikroorganismus nach Anspruch 9, der zusätzlich gegebenenfalls die kodierende Sequenz für wenigstens ein weiteres Enzym, ausgewählt unter Hydroxysteroiddehydrogenasen (HSDH) und zur Cofaktorregenerierung geeignete Dehydrogenasen, trägt.

11. Rekombinanter Mikroorganismus nach Anspruch 10, wobei
die weitere HSDH ausgewählt ist unter 3α-HSDHs; und
die Dehydrogenase ausgewählt ist unter NADPH-regenerierenden Enzymen, wie NADPH Dehydrogenasen, Alkoholdehydrogenasen (ADH), und NADPH regenerierenden Formiatdehydrogenasen (FDH), sowie Glucosedehydrogenasen (GDH), Glucose-6-Phosphat-Dehydrogenasen (G-6-PDH), oder Phosphit-Dehydrogenasen (PtDH), oder NADH-regenerierenden Enzymen, wie NADH Dehydrogenasen, NADH regenerierenden Formiatdehydrogenasen (FDH), NADH regenerierenden Alkoholdehydrogenasen (ADH), NADH regenerierenden Glucose-6-Phosphat-Dehydrogenasen (G6PDH), NADH regenerierenden Phosphitdehydrogenasen (PtDH) sowie NADH regenerierenden Glucosedehydrogenasen (GDH).

12. Rekombinanter Mikroorganismus nach einem der Ansprüche 9 bis 11, welcher ein 7α-HSDH knock-out Stamm ist.

13. Verfahren zur enzymatischen oder mikrobiellen Synthese von 7β-Hydroxysteroiden, wobei man das korrespondierende 7-Ketosteroid in Gegenwart einer 7β-HSDH gemäß der Definition in einem der Ansprüche 1 bis 5 oder in Gegenwart eines diese 7β-HSDH exprimierenden rekombinanten Mikroorganismus nach einem der Ansprüche 9 bis 12 reduziert, und gegebenenfalls wenigstens ein gebildetes Reduktionsprodukt aus dem Reaktionsansatz isoliert.

14. Verfahren nach Anspruch 13, wobei das 7- Ketosteroid ausgewählt ist unter Dehydrocholsäure (DHCA),
7-Keto-lithocholsäure (7-Keto-LCS),
7,12-Diketo-lithocholsäure (7,12-Diketo-LCS) und
den Derivaten davon, wie insbesondere einem Salz, Amid oder Alkylester der Säure.

15. Verfahren nach Anspruch 13 oder 14, wobei die Reduktion in Gegenwart und insbesondere unter Verbrauch von NADPH und/oder NADH erfolgt.

16. Verfahren nach Anspruch 15, wobei verbrauchtes NADPH durch Kopplung mit einem NADPH-regenerierenden Enzym regeneriert wird, wobei dieses insbesondere ausgewählt ist unter NADPH Dehydrogenasen, Alkoholdehydrogenasen (ADH), und NADPH regenerierenden Formiatdehydrogenasen (FDH) und einer NADPH-regenerierenden Glucosedehydrogenase (GDH), wobei das NADPH-regenerierende Enzym gegebenenfalls von einem rekombinanten Mikroorganismus exprimiert wird; und/oder wobei verbrauchtes NADH durch Kopplung mit einem NADH-regenerierenden Enzym regeneriert wird, wobei dieses insbesondere ausgewählt ist unter NADH-Dehydrogenasen, NADH-regenerierenden Formiatdehydrogenasen (FDH), NADH-regenerierenden Alkoholdehydrogenasen (ADH), NADH-regenerierenden Glucose-6-Phosphat-Dehydrogenasen (G6PDH), NADH-regenerierenden Phosphitdehydrogenasen (PtDH) sowie NADH-regenerierenden Glucosedehydrogenasen (GDH), wobei das NADH-regenerierende Enzym ggf. in einem rekombinanten Mikroorganismus exprimiert wird.

17. Verfahren nach Anspruch 16, wobei das NADPH-regenerierende Enzym ausgewählt ist unter
a) FDHs, einschließlich Mutanten einer NAD⁺-abhängigen FDH, welche wenigstens die enzymatische Oxidation von Ameisensäure zu CO₂ katalysiert, wobei die Mutante im Vergleich zum nicht-mutierten Enzym zusätzlich NADP⁺ als Cofaktor akzeptiert; und
b) GDHs.

18. Verfahren zur Herstellung von Ursodesoxycholsäure (UDCS) der Formel (1) 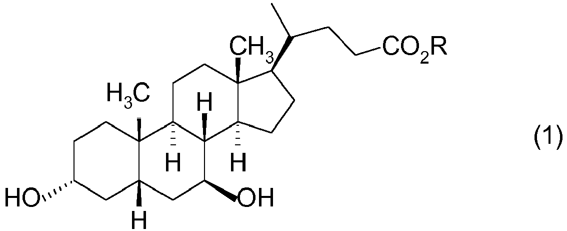 worin
R für Alkyl, H, ein Alkalimetallion oder N(R³)₄⁺steht, worin die Reste R³ gleich oder verschieden sind und für H oder Alkyl stehen, oder die Gruppe -CO₂R durch die Säureamid-Gruppe -CONR¹R², ersetzt ist, worin R¹ und R² unabhängig voneinander für einen Alkylrest stehen;
wobei man
a) gegebenenfalls eine Cholsäure (CS) der Formel (2) 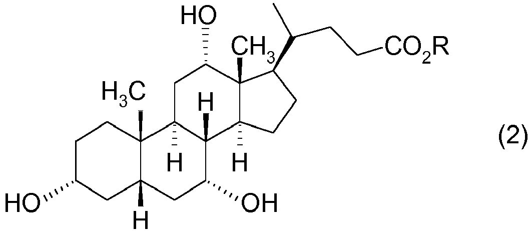 worin R die oben angegebenen Bedeutungen besitzt oder die Gruppe -CO₂R durch die Säureamid-Gruppe -CONR¹R², wie oben definiert ersetzt ist,
zur Dehydrocholsäure (DHCS) der Formel (3) 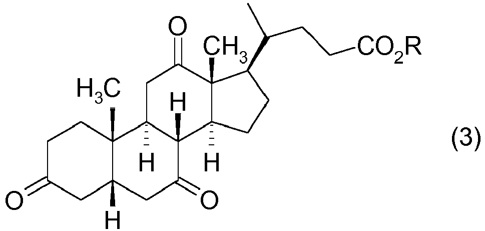 worin R die oben angegebenen Bedeutungen besitzt, oder die Gruppe -CO₂R durch die Säureamid-Gruppe -CONR¹R², wie oben definiert ersetzt ist, chemisch oxidiert;
b) DHCS in Gegenwart wenigstens einer 7β-HSDH-Mutante gemäß der Definition in einem der Ansprüche 1 bis 5 und in Gegenwart wenigstens einer 3α-HSDH zur korrespondierenden 12-Keto-ursodesoxycholsäure (12-Keto UDCS) der Formel (5) 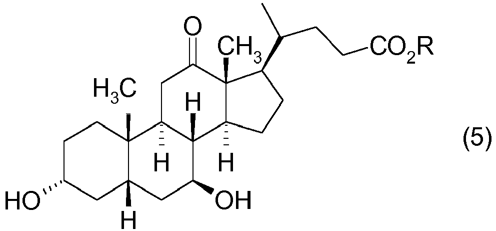 worin R die oben angegebenen Bedeutungen besitzt, oder die Gruppe -CO₂R durch die Säureamid-Gruppe -CONR¹R², wie oben definiert ersetzt ist, insbesondere in Gegenwart und unter Verbrauch von NADH und/oder NADPH
reduziert und anschließend
c) 12-Keto-UDCS der Formel (5) chemisch zu UDCS reduziert; und
d) das Reaktionsprodukt gegebenenfalls weiter aufreinigt.

19. Verfahren nach Anspruch 18, wobei zumindest Schritt b) in Gegenwart eines rekombinanten Mikroorganismus nach einem der Ansprüche 9 bis 12 durchgeführt wird.

20. Verfahren nach Anspruch 18 oder 19, wobei Schritt b) mit gleichen oder verschiedenen Kofaktorregenerierungssystemen gekoppelt ist.

21. Verfahren zur Herstellung von UDCS der Formel (1) 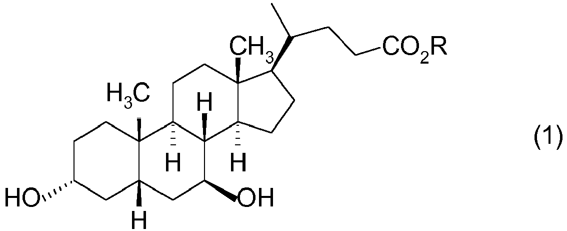 worin
R für Alkyl, H, ein Alkalimetallion oder N(R³)₄⁺ steht, worin die Reste R³ gleich oder verschieden sind und für H oder Alkyl stehen, oder die Gruppe -CO₂R durch die Säureamid-Gruppe -CONR¹R², wie oben definiert ersetzt ist;
wobei man
a) gegebenenfalls eine CS der Formel (2) 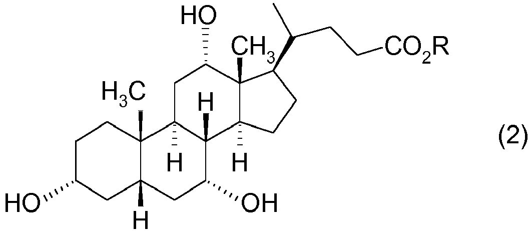 worin R die oben angegebenen Bedeutungen besitzt, oder die Gruppe -CO₂R durch die Säureamid-Gruppe -CONR¹R², wie oben definiert ersetzt ist, zur DHCS der Formel (3) 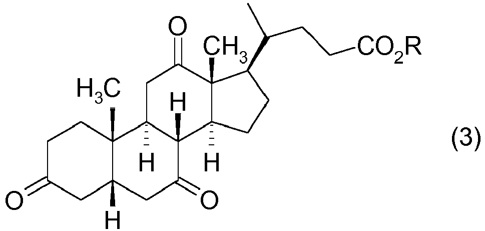 worin R die oben angegebenen Bedeutungen besitzt, oder die Gruppe -CO₂R durch die Säureamid-Gruppe -CONR¹R² wie oben definiert ersetzt ist, chemisch oxidiert;
b) DHCS in Gegenwart wenigstens einer 7β-HSDH und in Gegenwart wenigstens einer 3α-HSDH zur korrespondierenden 12-Keto UDCS der Formel (5) 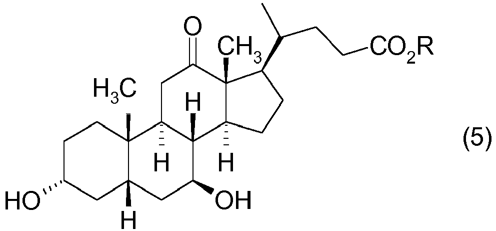 worin R die oben angegebenen Bedeutungen besitzt, oder die Gruppe -CO₂R durch die Säureamid-Gruppe -CONR¹R² wie oben definiert ersetzt ist, insbesondere in Gegenwart und unter Verbrauch von NADH und/oder NADPH, reduziert und anschließend
c) 12-Keto-UDCS der Formel (5) chemisch zu UDCS reduziert; und
d) das Reaktionsprodukt gegebenenfalls weiter aufreinigt;
wobei die Umsetzungen des Schritts b) in Gegenwart eines rekombinanten Mikroorganismus nach einem der Ansprüche 9 bis 12 erfolgen.

## Claims

1. 7β-Hydroxysteroid dehydrogenase (7β-HSDH) which catalyzes at least the stereospecific enzymatic reduction of a 7-ketosteroid to the corresponding 7-hydroxysteroid, wherein the enzyme comprises a mutation at position 64 of SEQ ID NO:2 or at the corresponding sequence positions of an amino acid sequence derived therefrom with at least 90% sequence identity to SEQ ID NO:2;
wherein the mutation at position 64 is the mutation R64X₁, wherein X₁ represents E, D, T, L, S, P, V, K, C, A, G, Q, F, W, I or Y;
and
wherein the enzyme, in comparison with the 7β-HSDH with SEQ ID NO:2, shows the following property profile:
a) an increased specific activity (Vmax [U/mg]) for NADPH in the enzymatic reduction of dehydrocholic acid (DHCA) with NADPH as cofactor; and additionally optionally:
b) an increased specific activity (Vmax [U/mg]) for DHCA in the enzymatic reduction of DHCA with NADPH as cofactor;
c) a reduced substrate inhibition by DHCA,
d) a modified cofactor specificity with respect to NADH and NADPH,
e) it being possible for these properties b), c) and d) to be present individually or in any combination.

2. 7β-Hydroxysteroid dehydrogenase (7β-HSDH) which catalyzes at least the stereospecific enzymatic reduction of a 7-ketosteroid to the corresponding 7-hydroxysteroid, wherein the enzyme comprises a mutation at position 64 of SEQ ID NO:2 or at the corresponding sequence positions of an amino acid sequence derived therefrom with at least 80% sequence identity to SEQ ID NO:2, and additionally has at least one mutation in the sequence motif VMVGRRE as per position 36 to 42 of SEQ ID NO:2 or in the corresponding sequence motif of an amino acid sequence derived therefrom with at least 80% sequence identity to SEQ ID NO:2;
wherein the mutation at position 64 is the mutation R64X₁, wherein X₁ represents E, D, T, L, S, P, V, K, C, A, G, Q, F, W, I or Y;
and
wherein the enzyme, in comparison with the 7β-HSDH with SEQ ID NO:2, shows the following property profile:
a) an increased specific activity (Vmax [U/mg]) for NADPH in the enzymatic reduction of dehydrocholic acid (DHCA) with NADPH as cofactor; and additionally optionally:
b) an increased specific activity (Vmax [U/mg]) for DHCA in the enzymatic reduction of DHCA with NADPH as cofactor;
c) a reduced substrate inhibition by DHCA,
d) a modified cofactor specificity with respect to NADH and NADPH,
e) it being possible for these properties b), c) and d) to be present individually or in any combination.

3. 7β-HSDH according to Claim 2, which has additionally the amino acid sequence mutation
G39X₃
wherein X₃ represents an amino acid radical other than glycine (G).

4. 7β-HSDH according to either of Claims 2 and 3, selected among
the dual mutants
R64X₁/G39X₃,
wherein
X₁ represents E, D, T, L, S, P, V, K, C, A, G, Q, F, W, I, Y, H or N; and
X₃ represents S, A, V, I, L, C, K, Y, F or R.

5. 7β-HSDH according to Claim 4, wherein the dual mutant is selected among:
(G39S/R64E); (G39S/R64D); (G39S/R64T); (G39S/R64L); (G39S/R64S); (G39S/R64P); (G39S/R64V); (G39A/R64E); (G39A/R64D); (G39A/R64T); (G39A/R64S); (G39A/R64L); (G39A/R64P); (G39A/R64V).

6. Nucleotide sequence which codes for a 7β-HSDH according to any of the preceding claims.

7. Expression cassette, comprising at least one nucleotide sequence according to Claim 6 under the control of at least one regulatory sequence.

8. Expression vector, comprising at least one expression cassette according to Claim 7.

9. Recombinant microorganism which bears at least one nucleotide sequence according to Claim 6 or at least one expression cassette according to Claim 7 or at least one expression vector according to Claim 8.

10. Recombinant microorganism according to Claim 9 which additionally optionally bears the coding sequence for at least one further enzyme, selected among hydroxysteroid dehydrogenases (HSDH) and dehydrogenases which are suitable for cofactor regeneration.

11. Recombinant microorganism according to Claim 10, the further HSDH being selected among 3α-HSDHs; and the dehydrogenase being selected among NADPH-regenerating enzymes, such as NADPH dehydrogenases, alcohol dehydrogenases (ADH), and NADPH regenerating formate dehydrogenases (FDH), and also glucose dehydrogenases (GDH), glucose-6-phosphate dehydrogenases (G-6-PDH) or phosphite dehydrogenases (PtDH), or NADH-regenerating enzymes, such as NADH dehydrogenases, NADH-regenerating formate dehydrogenases (FDH), NADH-regenerating alcohol dehydrogenases (ADH), NADH-regenerating glucose-6-phosphate dehydrogenases (G6PDH), NADH-regenerating phosphite dehydrogenases (PtDH) and NADH-regenerating glucose dehydrogenases (GDH).

12. Recombinant microorganism according to any of Claims 9 to 11, which is a 7α-HSDH knock-out strain.

13. Process for the enzymatic or microbial synthesis of 7β-hydroxysteroids, wherein the corresponding 7-ketosteroid is reduced in the presence of a 7β-HSDH according to the definition in any of Claims 1 to 5 or in the presence of a recombinant microorganism which expresses this 7β-HSDH according to one of Claims 9 to 12, and optionally at least one formed reduction product is isolated from the reaction mixture.

14. Process according to Claim 13, wherein the 7-ketosteroid is selected among
dehydrocholic acid (DHCA),
7-keto-lithocholic acid (7-keto-LCA), 7,12-diketo-lithocholic acid (7,12-diketo-LCA) and the derivatives thereof such as, in particular, a salt, an amide or an alkyl ester of the acid.

15. Process according to Claim 13 or 14, wherein the reduction takes place in the presence of and in particular with the consumption of NADPH and/or NADH.

16. Process according to Claim 15, wherein consumed NADPH is regenerated by coupling with an NADPH-regenerating enzyme, wherein the latter is selected in particular among NADPH dehydrogenases, alcohol dehydrogenases (ADH) and NADPH-regenerating formate dehydrogenases (FDH) and an NADPH-regenerating glucose dehydrogenase (GDH), wherein the NADPH-regenerating enzyme is optionally expressed by a recombinant microorganism; and/or where consumed NADH is regenerated by coupling with an NADH-regenerating enzyme, wherein the latter is selected in particular among NADH-dehydrogenases, NADH-regenerating formate dehydrogenases (FDH), NADH-regenerating alcohol dehydrogenases (ADH), NADH-regenerating glucose-6-phosphate dehydrogenases (G6PDH), NADH-regenerating phosphite dehydrogenases (PtDH) and NADH-regenerating glucose dehydrogenases (GDH), wherein the NADH-regenerating enzyme is optionally expressed in a recombinant microorganism.

17. Process according to Claim 16, wherein the NADPH-regenerating enzyme is selected among
a) FDHs, including mutants of a NAD⁺-dependent FDH, which catalyzes at least the enzymatic oxidation of formic acid to CO₂, wherein the mutant in comparison with the unmutated enzyme additionally accepts NADP⁺ as cofactor; and
b) GDHs.

18. Process for the preparation of ursodeoxycholic acid (UDCA) of the formula (1) 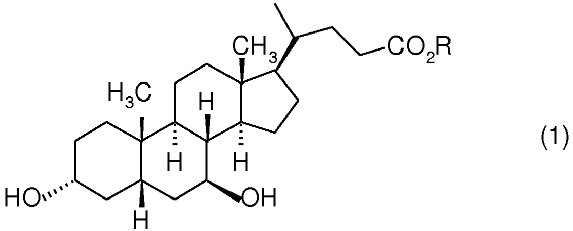 in which
R represents alkyl, H, an alkali metal ion or N(R³)₄⁺, wherein the radicals R³ are identical or different and represent H or alkyl, or the group -CO₂R is replaced by the acid amide group -CONR¹R², wherein R¹ and R² independently of one another represent an alkyl radical;
in which
a) optionally a cholic acid (CA) of the formula (2) 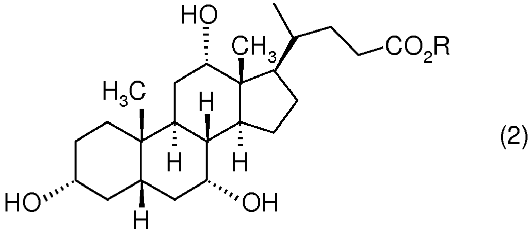 in which R has the abovementioned meanings or the group -CO₂R is replaced by the acid amide group -CONR¹R² as defined hereinabove
is oxidized chemically to dehydrocholic acid (DHCA) of the formula (3) 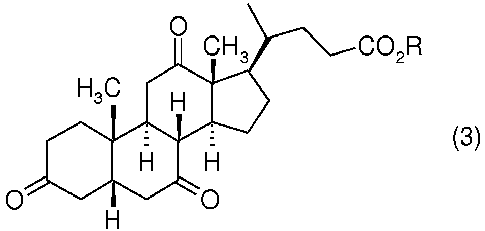 in which R has the abovementioned meanings or the group -CO₂R is replaced by the acid amide group -CONR¹R² as defined hereinabove;
b) DHCA is reduced in the presence of at least one 7β-HSDH mutant according to the definition in any of Claims 1 to 5 and in the presence of at least one 3α-HSDH to the corresponding 12-keto-ursodeoxycholic acid (12-keto UDCA) of the formula (5) 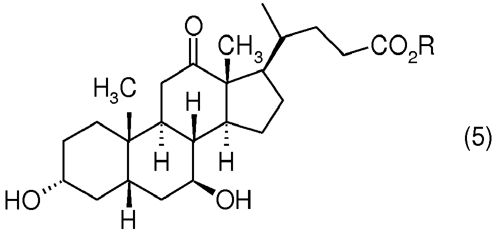 in which R has the abovementioned meanings or the group -CO₂R is replaced by the acid amide group -CONR¹R² as defined hereinabove, in particular in the presence and with the consumption of NADH and /or NADPH, and subsequently
c) 12-keto-UDCA of the formula (5) is reduced chemically to UDCA; and
d) optionally, the reaction product is purified further.

19. Process according to Claim 18, wherein at least step b) is carried out in the presence of a recombinant microorganism according to one of Claims 9 to 12.

20. Process according to Claim 18 or 19, wherein step b) is coupled with identical or different cofactor regeneration systems.

21. Process for the preparation of UDCA of the formula (1) 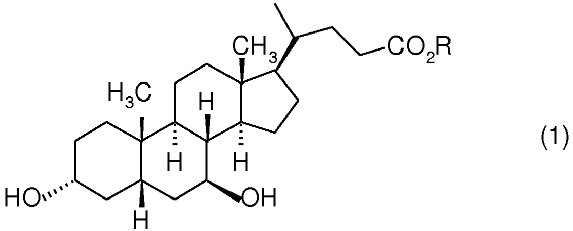 in which
R represents alkyl, H, an alkali metal ion or N(R³)₄⁺, wherein the radicals R³ are identical or different and represent H or alkyl, or the group -CO₂R is replaced by the acid amide group -CONR¹R², as defined hereinabove;
in which
a) optionally a cholic acid (CA) of the formula (2) 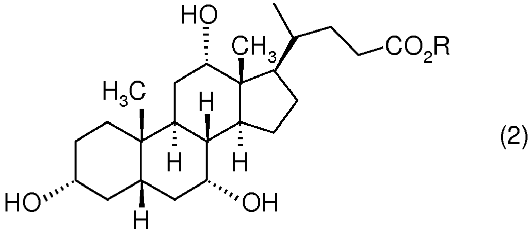 in which R has the abovementioned meanings or the group -CO₂R is replaced by the acid amide group -CONR¹R² as defined hereinabove is oxidized chemically to DHCA of the formula (3) 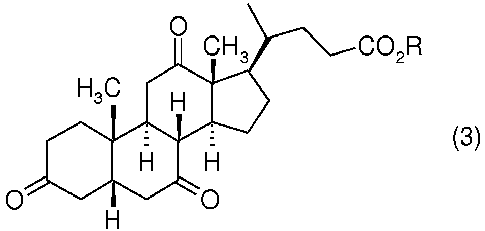 in which R has the abovementioned meanings or the group -CO₂R is replaced by the acid amide group -CONR¹R² as defined hereinabove;
b) DHCA is reduced in the presence of at least one 7β-HSDH and in the presence of at least one 3α-HSDH to the corresponding 12-keto UDCA of the formula (5) 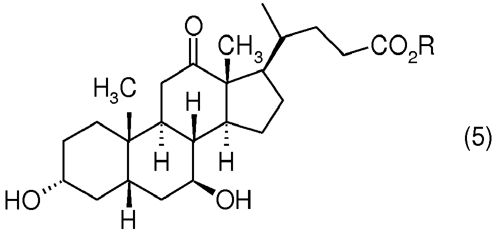 in which R has the abovementioned meanings or the group -CO₂R is replaced by the acid amide group -CONR¹R² as defined hereinabove, in particular in the presence and with the consumption of NADH and/or NADPH, and subsequently
c) 12-keto-UDCA of the formula (5) is reduced chemically to UDCA; and
d) optionally, the reaction product is purified further;
wherein the conversions of step b) are carried out in the presence of a recombinant microorganism according to any of Claims 9 to 12.

## Revendications

1. 7β-hydroxystéroïde déshydrogénase (7β-HSDH), qui catalyse au moins la réduction enzymatique stéréospécifique d'un 7-cétostéroïde en le 7-hydroxystéroïde correspondant, l'enzyme comprenant une mutation en position 64 de la séquence SEQ ID NO : 2 ou dans les positions de séquence correspondantes d'une séquence d'acides aminés dérivée de celle-ci, ayant une identité de séquence d'au moins 90 % avec la séquence SEQ ID NO : 2.
la mutation en la position 64 étant la mutation R64X₁, dans laquelle X₁ représente E, D, T, L, S, P, V, K, C, A, G, Q, F, W, I, ou Y ;
et
l'enzyme en comparaison de la 7β-HSDH à SEQ ID NO : 2 présentant le profil de propriétés suivant :
a) une activité spécifique accrue (Vₘₐₓ [U/mg]) pour NADPH dans la réduction enzymatique de l'acide déhydrocholique (DHCA) avec NADPH en tant que cofacteur ; et en outre éventuellement :
b) une activité spécifique accrue (Vₘₐₓ [U/mg]) pour le DHCA dans la réduction enzymatique du DHCA avec NADPH en tant que cofacteur ;
c) une inhibition réduite du substrat par le DHCA,
d) une spécificité de cofacteur modifiée en ce qui concerne le NADH et le NADPH,
e) ces propriétés b), c) et d) pouvant être présentes individuellement ou en une combinaison quelconque.

2. 7β-hydroxystéroïde déshydrogénase (7β-HSDH), qui catalyse au moins la réduction enzymatique stéréospécifique d'un 7-cétostéroïde en le 7-hydroxystéroïde correspondant, l'enzyme comprenant une mutation en position 64 de la séquence SEQ ID NO : 2 ou dans les positions de séquence correspondantes d'une séquence d'acides aminés dérivée de celle-ci, ayant une identité de séquence d'au moins 80 % avec la séquence SEQ ID NO : 2, et en outre au moins une mutation dans le motif de séquence VMVGRRE selon les positions 36 à 42 de la séquence SEQ ID NO : 2 ou dans le motif de séquence correspondant d'une séquence d'acides aminés dérivé de celle-ci, ayant une identité de séquence d'au moins 80 % avec la séquence SEQ ID NO : 2 ;
la mutation en la position 64 étant la mutation R64X₁, dans laquelle X₁ représente E, D, T, L, S, P, V, K, C, A, G, Q, F, W, I, ou Y ;
et
l'enzyme en comparaison de la 7β-HSDH à SEQ ID NO : 2 présentant le profil de propriétés suivant :
a) une activité spécifique accrue (Vₘₐₓ [U/mg]) pour NADPH dans la réduction enzymatique de l'acide déhydrocholique (DHCA) avec NADPH en tant que cofacteur ; et en outre éventuellement :
b) une activité spécifique accrue (Vₘₐₓ [U/mg]) pour le DHCA dans la réduction enzymatique du DHCA avec NADPH en tant que cofacteur ;
c) une inhibition réduite du substrat par le DHCA,
d) une spécificité de cofacteur modifiée en ce qui concerne le NADH et le NADPH,
e) ces propriétés b), c) et d) pouvant être présentes individuellement ou en une combinaison quelconque.

3. 7β-HSDH selon la revendication 2, qui comporte en outre la mutation de séquence d'acides aminés
G39X₃,
X₃ représentant un résidu d'acide aminé différent de la glycine (G).

4. 7β-HSDH selon l'une quelconque des revendications 2 et 3, choisie parmi les doubles mutants
R64X₁/G39X₃
dans lesquels
X₁ représente E, D, T, L, S, P, V, K, C, A, G, Q, F, W, I, Y, H ou N ; et
X₃ représente S, A, V, I, L, C, K, Y, F ou R.

5. 7β-HSDH selon la revendication 4, dans laquelle les doubles mutants sont choisis parmi :
(G39S/R64E) ; (G39S/R64D) ; (G39S/R64T) ; (G39S/R64L) ; (G39S/R64S) ; (G39S/R64P) ; (G39S/R64V) ; (G39A/R64E) ; (G39A/R64D) ; (G39A/R64T) ; (G39A/R64S) ; (G39A/R64L) ; (G39A/R64P) ; (G39A/R64V).

6. Séquence de nucléotides codant pour une 7β-HSDH selon l'une quelconque des revendications précédentes.

7. Cassette d'expression comprenant sous le contrôle d'au moins une séquence régulatrice au moine une séquence de nucléotides selon la revendication 6.

8. Vecteur d'expression, comprenant au moins une cassette d'expression selon la revendication 7.

9. Micro-organisme recombinant, qui porte au moins une séquence de nucléotides selon la revendication 6 ou au moins une cassette d'expression selon la revendication 7 ou au moins un vecteur d'expression selon la revendication 8.

10. Micro-organisme recombinant selon la revendication 9, qui porte en outre éventuellement la séquence codante pour au moins une autre enzyme, choisie parmi les hydroxystéroïde déshydrogénases (HSDH) et les déshydrogénases appropriées à la régénération de cofacteurs.

11. Micro-organisme recombinant selon la revendication 10, dans lequel
l'autre HSDH est choisie parmi les 3α-HSDH ; et
la déshydrogénase est choisie parmi les enzymes régénérant le NADPH, telles que les NADPH déshydrogénases, les alcool déshydrogénases (ADH), et les formiate déshydrogénases (FDH) régénérant le NADPH, ainsi que les glucose déshydrogénases (GDH), les glucose-6-phosphate déshydrogénases (G-6-PDH), ou les phosphite déshydrogénases (PtDH), ou
les enzymes régénérant le NADH, telles que les NADH déshydrogénases, les formiate déshydrogénases (FDH) régénérant le NADH, les alcool déshydrogénases régénérant le NADH (ADH), les glucose-6-phosphate déshydrogénases régénérant le NADH (G6PDH), les phosphite déshydrogénases régénérant le NADH (PtDH) ainsi que les glucose déshydrogénases régénérant le NADH (GDH).

12. Micro-organisme recombinant selon l'une quelconque des revendications 9 à 11, qui est une souche à 7β-HSDH invalidée.

13. Procédé pour la synthèse microbienne ou enzymatique de 7β-hydroxystéroïdes, dans lequel on réduit le 7-cétostéroïde correspondant en présente d'une 7β-HSDH selon la définition dans l'une quelconque des revendications 1 à 5 ou en présence d'un micro-organisme recombinant selon l'une quelconque des revendications 9 à 12, exprimant cette 7β-HSDH, et éventuellement on isole à partir du mélange réactionnel au moins un produit de réduction formé.

14. Procédé selon la revendication 13, dans lequel le 7-cétostéroïde est choisi parmi
l'acide déhydrocholique (DHCA)
l'acide 7-céto-lithocholique (7-céto-LCS),
l'acide 7,12-dicéto-lithocholique (7,12-dicéto-LCS) et les dérivés de ceux-ci, comme en particulier un sel, amide ou ester alkylique de l'acide.

15. Procédé selon la revendication 13 ou 14, dans lequel la réduction a lieu en présence et en particulier avec consommation de NADPH et/ou NADH.

16. Procédé selon la revendication 15, dans lequel le NADPH consommé est régénéré par couplage avec une enzyme régénérant le NADPH, celle-ci étant en particulier choisie parmi les NADPH déshydrogénases, les alcool déshydrogénases (ADH), et les formiate déshydrogénases (FDH) régénérant le NADPH, et une glucose déshydrogénase (GDH) régénérant le NADPH, l'enzyme régénérant le NADPH étant éventuellement exprimée par un micro-organisme recombinant ; et/ou dans lequel le NADH consommé est régénéré par couplage avec une enzyme régénérant le NADH, celle-ci étant en particulier choisie parmi les NADH déshydrogénases, les formiate déshydrogénases (FDH) régénérant le NADH, les alcool déshydrogénases (ADH) régénérant le NADH, les glucose-6-phosphate déshydrogénases régénérant le NADH (G6PDH), les phosphite déshydrogénases régénérant le NADH (PtDH) ainsi que les glucose déshydrogénases régénérant le NADH (GDH), l'enzyme régénérant le NADH étant éventuellement exprimée dans un micro-organisme recombinant.

17. Procédé selon la revendication 16, dans lequel l'enzyme régénérant le NADPH est choisie parmi
a) des FDH, y compris des mutants d'une FDH dépendante de NAD+, qui catalyse au moins l'oxydation enzymatique de l'acide formique en CO₂, les mutants en comparaison de l'enzyme non mutée acceptant en outre NADP⁺ en tant que cofacteur ; et
b) des GDH.

18. Procédé pour la préparation d'acide ursodésoxycholique (UDCS) de formule (1) 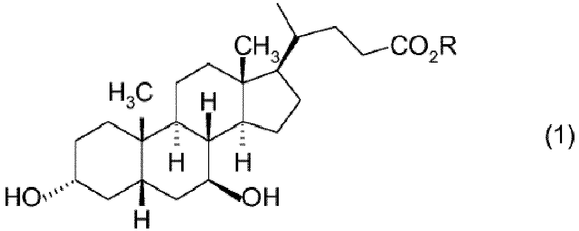 dans laquelle
R représente un groupe alkyle, H, un ion de métal alcalin ou N(R³)₄⁺, ou les radicaux R³ sont identiques ou différents et représentent H ou un groupe alkyle, ou le groupe -CO₂R est remplacé par le groupe amido -CONR¹R², dans lequel R¹ et R² représentent indépendamment l'un de l'autre un radical alkyle ;
dans lequel
a) éventuellement on oxyde chimiquement un acide cholique (CS) de formule (2) 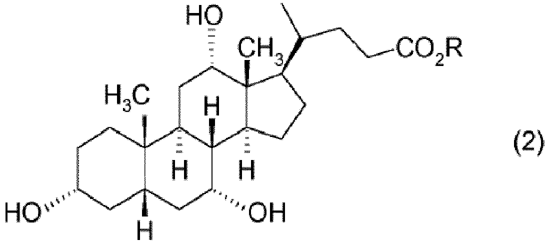 dans laquelle R a les significations indiquées ci-dessus ou le groupe -CO₂R est remplacé par le groupe amido -CONR¹R², tel que défini plus haut,
en l'acide déhydrocholique (DHCS) de formule (3) 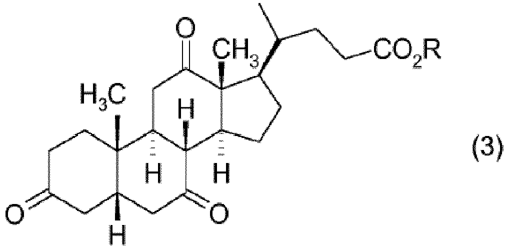 dans laquelle R a les significations indiquées plus haut, ou le groupe -CO₂R est remplacé par le groupe amido -CONR¹R², tel que défini plus haut,
b) on réduit le DHCS en présence d'au moins un mutant de 7β-HSDH selon la définition dans l'une quelconque des revendications 1 à 5 et en présence d'au moins une 3α-HSDH, en l'acide 12-céto-ursodésoxycholique (12-céto-UDCS) de formule (5) 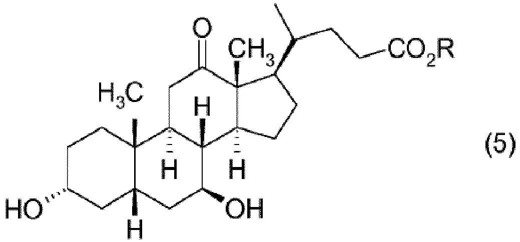 dans laquelle R a les significations indiquées plus haut, ou le groupe -CO₂R est remplacé par le groupe amido -CONR¹R², tel que défini plus haut, en particulier en présence et avec consommation de NADH et/ou NADPH et ensuite
c) on réduit chimiquement le 12-céto-UDCS de formule (5) en UDCS ; et
d) éventuellement on purifie davantage le produit de réaction.

19. Procédé selon la revendication 18, dans lequel on effectue au moins l'étape b) en présence d'un micro-organisme recombinant selon l'une quelconque des revendications 9 à 12.

20. Procédé selon la revendication 18 ou 19, dans lequel l'étape b) est couplée ave des systèmes de régénération de cofacteurs, identiques ou différents.

21. Procédé pour la préparation d'UDCS de formule (1) 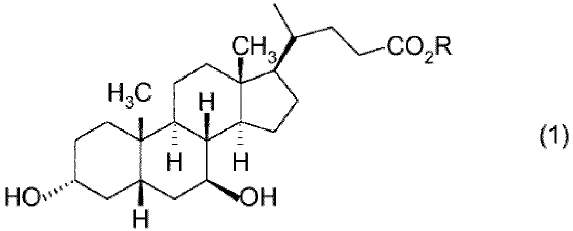 dans laquelle
R représente un groupe alkyle, H, un ion de métal alcalin ou N(R³)₄⁺, ou les radicaux R³ sont identiques ou différents et représentent H ou un groupe alkyle, ou le groupe -CO₂R est remplacé par le groupe amido -CONR¹R², tel que défini plus haut ;
dans lequel
a) éventuellement on oxyde chimiquement un CS de formule (2) 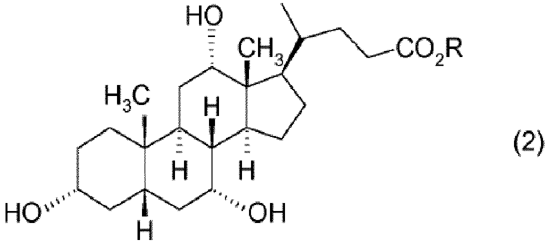 dans laquelle R a les significations indiquées ci-dessus, ou le groupe -CO₂R est remplacé par le groupe amido -CONR¹R², tel que défini plus haut, en le DHCS de formule (3) 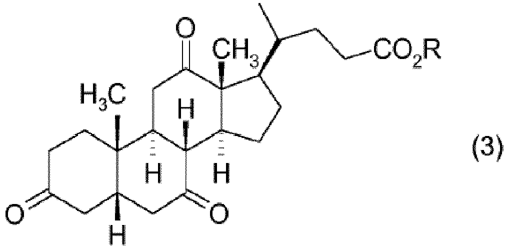 dans laquelle R a les significations indiquées plus haut, ou le groupe -CO₂R est remplacé par le groupe amido -CONR¹R², tel que défini plus haut,
b) on réduit le DHCS en présence d'au moins une 7β-HSDH et en présence d'au moins une 3α-HSDH, en l'acide 12-céto-UDCS de formule (5) 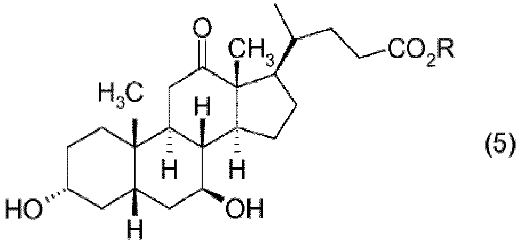 dans laquelle R a les significations indiquées plus haut, ou le groupe -CO₂R est remplacé par le groupe amido -CONR¹R², tel que défini plus haut, en particulier en présence et avec consommation de NADH et/ou NADPH et ensuite
c) on réduit chimiquement le 12-céto-UDCS de formule (5) en UDCS ; et
d) éventuellement on purifie davantage le produit de réaction,
les réactions de l'étape b) s'effectuant en présence d'un micro-organisme recombinant selon l'une quelconque des revendications 9 à 12.


### SEQUENCE LISTING

<110> Pharmazell GmbH
<120> Neue 7beta-Hydroxysteroid Dehydrogenase-Mutanten
<130> M/54192-PCT
<160> 69
<170> PatentIn version 3.5
<210> 1
   <211> 792
   <212> DNA
   <213> Collinsella aerofaciens
<220>
   <221> CDS
   <222> (1)..(792)
<400> 1
   atg aac ctg agg gag aag tac ggt gag tgg ggc ctg atc ctg ggc gcg        48
   Met Asn Leu Arg Glu Lys Tyr Gly Glu Trp Gly Leu Ile Leu Gly Ala
   1               5                   10                  15
   acc gag ggc gtc ggc aag gcg ttc tgc gag aag atc gcc gcc ggc ggc        96
   Thr Glu Gly Val Gly Lys Ala Phe Cys Glu Lys Ile Ala Ala Gly Gly
               20                  25                  30
   atg aac gtc gtc atg gtc ggc cgt cgc gag gag aag ctg aac gtg ctc       144
   Met Asn Val Val Met Val Gly Arg Arg Glu Glu Lys Leu Asn Val Leu
           35                  40                  45
   gca ggc gag atc cgc gag acc tac ggc gtg gag acc aag gtc gtg cgc       192
   Ala Gly Glu Ile Arg Glu Thr Tyr Gly Val Glu Thr Lys Val Val Arg
       50                  55                  60
   gcc gac ttt agc cag ccc ggc gct gcc gag acc gtc ttc gcc gcg acc       240
   Ala Asp Phe Ser Gln Pro Gly Ala Ala Glu Thr Val Phe Ala Ala Thr
   65                  70                  75                  80
   gag ggc ctg gac atg ggc ttc atg agc tac gtg gcc tgc ctg cac agc       288
   Glu Gly Leu Asp Met Gly Phe Met Ser Tyr Val Ala Cys Leu His Ser
                   85                  90                  95
   ttc ggt aag atc cag gac acc ccc tgg gag aag cac gag gcc atg atc       336
   Phe Gly Lys Ile Gln Asp Thr Pro Trp Glu Lys His Glu Ala Met Ile
               100                 105                 110
   aac gtc aac gtc gtg acc ttc ctc aag tgc ttc cac cac tac atg cgg       384
   Asn Val Asn Val Val Thr Phe Leu Lys Cys Phe His His Tyr Met Arg
           115                 120                 125
   atc ttt gcc gcc cag gac cgc ggc gcc gtg atc aac gtc tcg tcg atg       432
   Ile Phe Ala Ala Gln Asp Arg Gly Ala Val Ile Asn Val Ser Ser Met
       130                 135                 140
   acc ggc atc agc tcc agc ccc tgg aac ggc cag tac ggc gcg ggc aag       480
   Thr Gly Ile Ser Ser Ser Pro Trp Asn Gly Gln Tyr Gly Ala Gly Lys
   145                 150                 155                 160
   gcc ttc atc ctc aag atg acc gag gcc gtg gcc tgc gag tgc gag ggc        528
   Ala Phe Ile Leu Lys Met Thr Glu Ala Val Ala Cys Glu Cys Glu Gly
                   165                 170                 175
   acc ggc gtc gac gtc gag gtc atc acc ctc ggc acc acc cta acc ccc        576
   Thr Gly Val Asp Val Glu Val Ile Thr Leu Gly Thr Thr Leu Thr Pro
               180                 185                 190
   agc ctg ctg tcc aac ctc ccc ggc ggc ccg cag ggc gag gcc gtc atg        624
   Ser Leu Leu Ser Asn Leu Pro Gly Gly Pro Gln Gly Glu Ala Val Met
           195                 200                 205
   aag atc gcc ctc acc ccc gag gag tgc gtt gac gag gcc ttt gag aag        672
   Lys Ile Ala Leu Thr Pro Glu Glu Cys Val Asp Glu Ala Phe Glu Lys
       210                 215                 220
   ctg ggt aag gag ctc tcc gtc atc gcc ggc cag cgc aac aag gac tcc        720
   Leu Gly Lys Glu Leu Ser Val Ile Ala Gly Gln Arg Asn Lys Asp Ser
   225                 230                 235                 240
   gtc cac gac tgg aag gca aac cac acc gag gac gag tac atc cgc tac        768
   Val His Asp Trp Lys Ala Asn His Thr Glu Asp Glu Tyr Ile Arg Tyr
                   245                 250                 255
   atg ggg tcg ttc tac cgc gac tag                                        792
   Met Gly Ser Phe Tyr Arg Asp
               260
<210> 2
   <211> 263
   <212> PRT
   <213> Collinsella aerofaciens
<400> 2
   Met Asn Leu Arg Glu Lys Tyr Gly Glu Trp Gly Leu Ile Leu Gly Ala
   1               5                   10                  15
   Thr Glu Gly Val Gly Lys Ala Phe Cys Glu Lys Ile Ala Ala Gly Gly
               20                  25                  30
   Met Asn Val Val Met Val Gly Arg Arg Glu Glu Lys Leu Asn Val Leu
           35                  40                  45
   Ala Gly Glu Ile Arg Glu Thr Tyr Gly Val Glu Thr Lys Val Val Arg
       50                  55                  60
   Ala Asp Phe Ser Gln Pro Gly Ala Ala Glu Thr Val Phe Ala Ala Thr
   65                  70                  75                  80
   Glu Gly Leu Asp Met Gly Phe Met Ser Tyr Val Ala Cys Leu His Ser
                   85                  90                  95
   Phe Gly Lys Ile Gln Asp Thr Pro Trp Glu Lys His Glu Ala Met Ile
               100                 105                 110
   Asn Val Asn Val Val Thr Phe Leu Lys Cys Phe His His Tyr Met Arg
           115                 120                 125
   Ile Phe Ala Ala Gln Asp Arg Gly Ala Val Ile Asn Val Ser Ser Met
       130                 135                 140
   Thr Gly Ile Ser Ser Ser Pro Trp Asn Gly Gln Tyr Gly Ala Gly Lys
   145                 150                 155                 160
   Ala Phe Ile Leu Lys Met Thr Glu Ala Val Ala Cys Glu Cys Glu Gly
                   165                 170                 175
   Thr Gly Val Asp Val Glu Val Ile Thr Leu Gly Thr Thr Leu Thr Pro
               180                 185                 190
   Ser Leu Leu Ser Asn Leu Pro Gly Gly Pro Gln Gly Glu Ala Val Met
           195                 200                 205
   Lys Ile Ala Leu Thr Pro Glu Glu Cys Val Asp Glu Ala Phe Glu Lys
       210                 215                 220
   Leu Gly Lys Glu Leu Ser Val Ile Ala Gly Gln Arg Asn Lys Asp Ser
   225                 230                 235                 240
   Val His Asp Trp Lys Ala Asn His Thr Glu Asp Glu Tyr Ile Arg Tyr
                   245                 250                 255
   Met Gly Ser Phe Tyr Arg Asp
               260
<210> 3
   <211> 271
   <212> PRT
   <213> Artificial Sequence
<220>
   <223> 7β HSDH (His6)
<400> 3
   Met Asn Leu Arg Glu Lys Tyr Gly Glu Trp Gly Leu Ile Leu Gly Ala
   1               5                   10                  15
   Thr Glu Gly Val Gly Lys Ala Phe Cys Glu Lys Ile Ala Ala Gly Gly
               20                  25                  30
   Met Asn Val Val Met Val Gly Arg Arg Glu Glu Lys Leu Asn Val Leu
           35                  40                  45
   Ala Gly Glu Ile Arg Glu Thr Tyr Gly Val Glu Thr Lys Val Val Arg
       50                  55                  60
   Ala Asp Phe Ser Gln Pro Gly Ala Ala Glu Thr Val Phe Ala Ala Thr
   65                  70                  75                  80
   Glu Gly Leu Asp Met Gly Phe Met Ser Tyr Val Ala Cys Leu His Ser
                   85                  90                  95
   Phe Gly Lys Ile Gln Asp Thr Pro Trp Glu Lys His Glu Ala Met Ile
               100                 105                 110
   Asn Val Asn Val Val Thr Phe Leu Lys Cys Phe His His Tyr Met Arg
           115                 120                 125
   Ile Phe Ala Ala Gln Asp Arg Gly Ala Val Ile Asn Val Ser Ser Met
       130                 135                 140
   Thr Gly Ile Ser Ser Ser Pro Trp Asn Gly Gln Tyr Gly Ala Gly Lys
   145                 150                 155                 160
   Ala Phe Ile Leu Lys Met Thr Glu Ala Val Ala Cys Glu Cys Glu Gly
                   165                 170                 175
   Thr Gly Val Asp Val Glu Val Ile Thr Leu Gly Thr Thr Leu Thr Pro
               180                 185                 190
   Ser Leu Leu Ser Asn Leu Pro Gly Gly Pro Gln Gly Glu Ala Val Met
           195                 200                 205
   Lys Ile Ala Leu Thr Pro Glu Glu Cys Val Asp Glu Ala Phe Glu Lys
       210                 215                 220
   Leu Gly Lys Glu Leu Ser Val Ile Ala Gly Gln Arg Asn Lys Asp Ser
   225                 230                 235                 240
   Val His Asp Trp Lys Ala Asn His Thr Glu Asp Glu Tyr Ile Arg Tyr
                   245                 250                 255
   Met Gly Ser Phe Tyr Arg Asp Leu Glu His His His His His His
               260                 265                 270
<210> 4
   <211> 271
   <212> PRT
   <213> Artificial Sequence
<220>
   <223> 7β-HSDH R64E Mutante
<400> 4
   Met Asn Leu Arg Glu Lys Tyr Gly Glu Trp Gly Leu Ile Leu Gly Ala
   1               5                   10                  15
   Thr Glu Gly Val Gly Lys Ala Phe Cys Glu Lys Ile Ala Ala Gly Gly
               20                  25                  30
   Met Asn Val Val Met Val Gly Arg Arg Glu Glu Lys Leu Asn Val Leu
           35                  40                  45
   Ala Gly Glu Ile Arg Glu Thr Tyr Gly Val Glu Thr Lys Val Val Glu
       50                  55                  60
   Ala Asp Phe Ser Gln Pro Gly Ala Ala Glu Thr Val Phe Ala Ala Thr
   65                  70                  75                  80
   Glu Gly Leu Asp Met Gly Phe Met Ser Tyr Val Ala Cys Leu His Ser
                   85                  90                  95
   Phe Gly Lys Ile Gln Asp Thr Pro Trp Glu Lys His Glu Ala Met Ile
               100                 105                 110
   Asn Val Asn Val Val Thr Phe Leu Lys Cys Phe His His Tyr Met Arg
           115                 120                 125
   Ile Phe Ala Ala Gln Asp Arg Gly Ala Val Ile Asn Val Ser Ser Met
       130                 135                 140
   Thr Gly Ile Ser Ser Ser Pro Trp Asn Gly Gln Tyr Gly Ala Gly Lys
   145                 150                 155                 160
   Ala Phe Ile Leu Lys Met Thr Glu Ala Val Ala Cys Glu Cys Glu Gly
                   165                 170                 175
   Thr Gly Val Asp Val Glu Val Ile Thr Leu Gly Thr Thr Leu Thr Pro
               180                 185                 190
   Ser Leu Leu Ser Asn Leu Pro Gly Gly Pro Gln Gly Glu Ala Val Met
           195                 200                 205
   Lys Ile Ala Leu Thr Pro Glu Glu Cys Val Asp Glu Ala Phe Glu Lys
       210                 215                 220
   Leu Gly Lys Glu Leu Ser Val Ile Ala Gly Gln Arg Asn Lys Asp Ser
   225                 230                 235                 240
   Val His Asp Trp Lys Ala Asn His Thr Glu Asp Glu Tyr Ile Arg Tyr
   245 250 255
   Met Gly Ser Phe Tyr Arg Asp Leu Glu His His His His His His
   260 265 270
<210> 5
   <211> 271
   <212> PRT
   <213> Artificial Sequence
<220>
   <223> 7β-HSDH G39S Mutante
<400> 5
   Met Asn Leu Arg Glu Lys Tyr Gly Glu Trp Gly Leu Ile Leu Gly Ala
   1               5                   10                  15
   Thr Glu Gly Val Gly Lys Ala Phe Cys Glu Lys Ile Ala Ala Gly Gly
               20                  25                  30
   Met Asn Val Val Met Val Ser Arg Arg Glu Glu Lys Leu Asn Val Leu
           35                  40                  45
   Ala Gly Glu Ile Arg Glu Thr Tyr Gly Val Glu Thr Lys Val Val Arg
       50                  55                  60
   Ala Asp Phe Ser Gln Pro Gly Ala Ala Glu Thr Val Phe Ala Ala Thr
   65                  70                  75                  80
   Glu Gly Leu Asp Met Gly Phe Met Ser Tyr Val Ala Cys Leu His Ser
                   85                  90                  95
   Phe Gly Lys Ile Gln Asp Thr Pro Trp Glu Lys His Glu Ala Met Ile
               100                 105                 110
   Asn Val Asn Val Val Thr Phe Leu Lys Cys Phe His His Tyr Met Arg
           115                 120                 125
   Ile Phe Ala Ala Gln Asp Arg Gly Ala Val Ile Asn Val Ser Ser Met
       130                 135                 140
   Thr Gly Ile Ser Ser Ser Pro Trp Asn Gly Gln Tyr Gly Ala Gly Lys
   145                 150                 155                 160
   Ala Phe Ile Leu Lys Met Thr Glu Ala Val Ala Cys Glu Cys Glu Gly
                   165                 170                 175
   Thr Gly Val Asp Val Glu Val Ile Thr Leu Gly Thr Thr Leu Thr Pro
                   180                 185                 190
   Ser Leu Leu Ser Asn Leu Pro Gly Gly Pro Gln Gly Glu Ala Val Met
           195                 200                 205
   Lys Ile Ala Leu Thr Pro Glu Glu Cys Val Asp Glu Ala Phe Glu Lys
       210                 215                 220
   Leu Gly Lys Glu Leu Ser Val Ile Ala Gly Gln Arg Asn Lys Asp Ser
   225                 230                 235                 240
   Val His Asp Trp Lys Ala Asn His Thr Glu Asp Glu Tyr Ile Arg Tyr
                   245                 250                 255
   Met Gly Ser Phe Tyr Arg Asp Leu Glu His His His His His His
               260                 265                 270
<210> 6
   <211> 271
   <212> PRT
   <213> Artificial Sequence
<220>
   <223> 7β-HSDH G39S/R64E Mutante
<400> 6
   Met Asn Leu Arg Glu Lys Tyr Gly Glu Trp Gly Leu Ile Leu Gly Ala
   1               5                   10                  15
   Thr Glu Gly Val Gly Lys Ala Phe Cys Glu Lys Ile Ala Ala Gly Gly
               20                  25                  30
   Met Asn Val Val Met Val Ser Arg Arg Glu Glu Lys Leu Asn Val Leu
           35                  40                  45
   Ala Gly Glu Ile Arg Glu Thr Tyr Gly Val Glu Thr Lys Val Val Arg
       50                  55                  60
   Ala Asp Phe Ser Gln Pro Gly Ala Ala Glu Thr Val Phe Ala Ala Thr
   65                  70                  75                  80
   Glu Gly Leu Asp Met Gly Phe Met Ser Tyr Val Ala Cys Leu His Ser
                   85                  90                  95
   Phe Gly Lys Ile Gln Asp Thr Pro Trp Glu Lys His Glu Ala Met Ile
               100                 105                 110
   Asn Val Asn Val Val Thr Phe Leu Lys Cys Phe His His Tyr Met Arg
           115                 120                 125
   Ile Phe Ala Ala Gln Asp Arg Gly Ala Val Ile Asn Val Ser Ser Met
       130                 135                 140
   Thr Gly Ile Ser Ser Ser Pro Trp Asn Gly Gln Tyr Gly Ala Gly Lys
   145                 150                 155                 160
   Ala Phe Ile Leu Lys Met Thr Glu Ala Val Ala Cys Glu Cys Glu Gly
                   165                 170                 175
   Thr Gly Val Asp Val Glu Val Ile Thr Leu Gly Thr Thr Leu Thr Pro
               180                 185                 190
   Ser Leu Leu Ser Asn Leu Pro Gly Gly Pro Gln Gly Glu Ala Val Met
           195                 200                 205
   Lys Ile Ala Leu Thr Pro Glu Glu Cys Val Asp Glu Ala Phe Glu Lys
       210                 215                 220
   Leu Gly Lys Glu Leu Ser Val Ile Ala Gly Gln Arg Asn Lys Asp Ser
   225                 230                 235                 240
   Val His Asp Trp Lys Ala Asn His Thr Glu Asp Glu Tyr Ile Arg Tyr
                   245                 250                 255
   Met Gly Ser Phe Tyr Arg Asp Leu Glu His His His His His His
               260                 265                 270
<210> 7
   <211> 786
   <212> DNA
   <213> Bacillus subtilis
<220>
   <221> CDS
   <222> (1)..(786)
<400> 7
   atg tat ccg gat tta aaa gga aaa gtc gtc gct att aca gga gct gct       48
   Met Tyr Pro Asp Leu Lys Gly Lys Val Val Ala Ile Thr Gly Ala Ala
   1               5                   10                  15
   tca ggg ctc gga aag gcg atg gcc att cgc ttc ggc aag gag cag gca       96
   Ser Gly Leu Gly Lys Ala Met Ala Ile Arg Phe Gly Lys Glu Gln Ala
               20                  25                  30
   aaa gtg gtt atc aac tat tat agt aat aaa caa gat ccg aac gag gta      144
   Lys Val Val Ile Asn Tyr Tyr Ser Asn Lys Gln Asp Pro Asn Glu Val
           35                  40                  45
   aaa gaa gag gtc atc aag gcg ggc ggt gaa gct gtt gtc gtc caa gga      192
   Lys Glu Glu Val Ile Lys Ala Gly Gly Glu Ala Val Val Val Gln Gly
       50                  55                  60
   gat gtc acg aaa gag gaa gat gta aaa aat atc gtg caa acg gca att       240
   Asp Val Thr Lys Glu Glu Asp Val Lys Asn Ile Val Gln Thr Ala Ile
   65                  70                  75                  80
   aag gag ttc ggc aca ctc gat att atg att aat aat gcc ggt ctt gaa       288
   Lys Glu Phe Gly Thr Leu Asp Ile Met Ile Asn Asn Ala Gly Leu Glu
                   85                  90                  95
   aat cct gtg cca tct cac gaa atg ccg ctc aag gat tgg gat aaa gtc       336
   Asn Pro Val Pro Ser His Glu Met Pro Leu Lys Asp Trp Asp Lys Val
               100                 105                 110
   atc ggc acg aac tta acg ggt gcc ttt tta gga agc cgt gaa gcg att       384
   Ile Gly Thr Asn Leu Thr Gly Ala Phe Leu Gly Ser Arg Glu Ala Ile
           115                 120                 125
   aaa tat ttc gta gaa aac gat atc aag gga aat gtc att aac atg tcc       432
   Lys Tyr Phe Val Glu Asn Asp Ile Lys Gly Asn Val Ile Asn Met Ser
       130                 135                 140
   agt gtg cac gaa gtg att cct tgg ccg tta ttt gtc cac tat gcg gca       480
   Ser Val His Glu Val Ile Pro Trp Pro Leu Phe Val His Tyr Ala Ala
   145                 150                 155                 160
   agt aaa ggc ggg ata aag ctg atg aca gaa aca tta gcg ttg gaa tac       528
   Ser Lys Gly Gly Ile Lys Leu Met Thr Glu Thr Leu Ala Leu Glu Tyr
                   165                 170                 175
   gcg ccg aag ggc att cgc gtc aat aat att ggg cca ggt gcg atc aac       576
   Ala Pro Lys Gly Ile Arg Val Asn Asn Ile Gly Pro Gly Ala Ile Asn
               180                 185                 190
   acg cca atc aat gct gaa aaa ttc gct gac cct aaa cag aaa gct gat       624
   Thr Pro Ile Asn Ala Glu Lys Phe Ala Asp Pro Lys Gln Lys Ala Asp
           195                 200                 205
   gta gaa agc atg att cca atg gga tat atc ggc gaa ccg gag gag atc       672
   Val Glu Ser Met Ile Pro Met Gly Tyr Ile Gly Glu Pro Glu Glu Ile
       210                 215                 220
   gcc gca gta gca gcc tgg ctt gct tcg aag gaa gcc agc tac gtc aca       720
   Ala Ala Val Ala Ala Trp Leu Ala Ser Lys Glu Ala Ser Tyr Val Thr
   225                 230                 235                 240
   ggc atc acg tta ttc gcg gac ggc ggt atg aca caa tat cct tca ttc       768
   Gly Ile Thr Leu Phe Ala Asp Gly Gly Met Thr Gln Tyr Pro Ser Phe
                   245                 250                 255
   cag gca ggc cgc ggt taa                                               786
   Gln Ala Gly Arg Gly
               260
<210> 8
   <211> 261
   <212> PRT
   <213> Bacillus subtilis
<400> 8
   Met Tyr Pro Asp Leu Lys Gly Lys Val Val Ala Ile Thr Gly Ala Ala
   1               5                   10                  15
   Ser Gly Leu Gly Lys Ala Met Ala Ile Arg Phe Gly Lys Glu Gln Ala
               20                  25                  30
   Lys Val Val Ile Asn Tyr Tyr Ser Asn Lys Gln Asp Pro Asn Glu Val
           35                  40                  45
   Lys Glu Glu Val Ile Lys Ala Gly Gly Glu Ala Val Val Val Gln Gly
       50                  55                  60
   Asp Val Thr Lys Glu Glu Asp Val Lys Asn Ile Val Gln Thr Ala Ile
   65                  70                  75                  80
   Lys Glu Phe Gly Thr Leu Asp Ile Met Ile Asn Asn Ala Gly Leu Glu
                   85                  90                  95
   Asn Pro Val Pro Ser His Glu Met Pro Leu Lys Asp Trp Asp Lys Val
               100                 105                 110
   Ile Gly Thr Asn Leu Thr Gly Ala Phe Leu Gly Ser Arg Glu Ala Ile
           115                 120                 125
   Lys Tyr Phe Val Glu Asn Asp Ile Lys Gly Asn Val Ile Asn Met Ser
       130                 135                 140
   Ser Val His Glu Val Ile Pro Trp Pro Leu Phe Val His Tyr Ala Ala
   145                 150                 155                 160
   Ser Lys Gly Gly Ile Lys Leu Met Thr Glu Thr Leu Ala Leu Glu Tyr
                   165                 170                 175
   Ala Pro Lys Gly Ile Arg Val Asn Asn Ile Gly Pro Gly Ala Ile Asn
               180                 185                 190
   Thr Pro Ile Asn Ala Glu Lys Phe Ala Asp Pro Lys Gln Lys Ala Asp
           195                 200                 205
   Val Glu Ser Met Ile Pro Met Gly Tyr Ile Gly Glu Pro Glu Glu Ile
       210                 215                 220
   Ala Ala Val Ala Ala Trp Leu Ala Ser Lys Glu Ala Ser Tyr Val Thr
   225                 230                 235                 240
   Gly Ile Thr Leu Phe Ala Asp Gly Gly Met Thr Gln Tyr Pro Ser Phe
                   245                 250                 255
   Gln Ala Gly Arg Gly
   260
<210> 9
   <211> 774
   <212> DNA
   <213> Comamonas testosteroni
<220>
   <221> CDS
   <222> (1)..(774)
<400> 9
   atg tcc atc atc gtg ata agc ggc tgc gcc acc ggc att ggt gcg gct        48
   acg cgc aag gtc ctg gag gcg gcc ggt cac cag atc gta ggc atc gat        96
   ata cgc gat gcg gaa gtg att gcc gat ctc tcg acg gcc gaa ggt cga       144
   aag cag gcg att gcc gat gta ctg gcg aag tgc agc aag ggc atg gac       192
   ggc ctg gtg ctg tgc gcc ggc ctg gga ccg cag acc aag gtg ctt ggc       240
   aat gtg gtt tcg gtc aat tat ttt ggc gcg acc gag ctg atg gat gcc       288
   ttt ttg cca gcg ctg aaa aaa ggc cat cag ccc gca gcc gtc gtc atc       336
   tcg tcc gtg gct tcc gcg cat ctg gct ttt gac aag aac cca ctg gcg       384
   ctg gca ctg gaa gcc ggc gag gaa gcc aag gcc cgc gcc att gtc gaa       432
   cat gcg gga gag cag ggc gga aat ctg gcc tat gcg ggc agc aag aat       480
   gct ttg acg gtg gct gtg cgc aaa cgc gcc gcc gcc tgg ggc gag gct       528
   ggc gtg cgc ctg aac acc atc gcc ccc ggt gca acc gag act ccc ttg       576
   ctg cag gcg ggc ctg cag gac ccg cgc tat ggc gaa tcc att gcc aag         624
   ttc gtt cct ccc atg ggc cgc cgt gcc gag ccg tcc gag atg gcg tcg         672
   gtc atc gcc ttt ttg atg agc ccg gcc gca agc tat gtg cat ggc gcg         720
   cag atc gtc att gat ggc ggc att gat gcg gtg atg cgc ccg aca cag         768
   ttc tga                                                                 774
<210> 10
   <211> 257
   <212> PRT
   <213> Comamonas testosteroni
<400> 10
<210> 11
   <211> 401
   <212> PRT
   <213> Mycobacterium vaccae
<400> 11
   Met Ala Lys Val Leu Cys Val Leu Tyr Asp Asp Pro Val Asp Gly Tyr
   1               5                   10                  15
   Pro Lys Thr Tyr Ala Arg Asp Asp Leu Pro Lys Ile Asp His Tyr Pro
               20                  25                  30
   Gly Gly Gln Ile Leu Pro Thr Pro Lys Ala Ile Asp Phe Thr Pro Gly
           35                  40                  45
   Gln Leu Leu Gly Ser Val Ser Gly Glu Leu Gly Leu Arg Glu Tyr Leu
       50                  55                  60
   Glu Ser Asn Gly His Thr Leu Val Val Thr Ser Asp Lys Asp Gly Pro
   65                  70                  75                  80
   Asp Ser Val Phe Glu Arg Glu Leu Val Asp Ala Asp Val Val Ile Ser
                   85                  90                  95
   Gln Pro Phe Trp Pro Ala Tyr Leu Thr Pro Glu Arg Ile Ala Lys Ala
               100                 105                 110
   Lys Asn Leu Lys Leu Ala Leu Thr Ala Gly Ile Gly Ser Asp His Val
           115                 120                 125
   Asp Leu Gln Ser Ala Ile Asp Arg Asn Val Thr Val Ala Glu Val Thr
       130                 135                 140
   Tyr Cys Asn Ser Ile Ser Val Ala Glu His Val Val Met Met Ile Leu
   145                 150                 155                 160
   Ser Leu Val Arg Asn Tyr Leu Pro Ser His Glu Trp Ala Arg Lys Gly
                   165                 170                 175
   Gly Trp Asn Ile Ala Asp Cys Val Ser His Ala Tyr Asp Leu Glu Ala
               180                 185                 190
   Met His Val Gly Thr Val Ala Ala Gly Arg Ile Gly Leu Ala Val Leu
           195                 200                 205
   Arg Arg Leu Ala Pro Phe Asp Val His Leu His Tyr Thr Asp Arg His
       210                 215                 220
   Arg Leu Pro Glu Ser Val Glu Lys Glu Leu Asn Leu Thr Trp His Ala
   225                 230                 235                 240
   Thr Arg Glu Asp Met Tyr Pro Val Cys Asp Val Val Thr Leu Asn Cys
                   245                 250                 255
   Pro Leu His Pro Glu Thr Glu His Met Ile Asn Asp Glu Thr Leu Lys
               260                 265                 270
   Leu Phe Lys Arg Gly Ala Tyr Ile Val Asn Thr Ala Arg Gly Lys Leu
           275                 280                 285
   Cys Asp Arg Asp Ala Val Ala Arg Ala Leu Glu Ser Gly Arg Leu Ala
       290                 295                 300
   Gly Tyr Ala Gly Asp Val Trp Phe Pro Gln Pro Ala Pro Lys Asp His
   305                 310                 315                 320
   Pro Trp Arg Thr Met Pro Tyr Asn Gly Met Thr Pro His Ile Ser Gly
                   325                 330                 335
   Thr Thr Leu Thr Ala Gln Ala Arg Tyr Ala Ala Gly Thr Arg Glu Ile
               340                 345                 350
   Leu Glu Cys Phe Phe Glu Gly Arg Pro Ile Arg Asp Glu Tyr Leu Ile
           355                 360                 365
   Val Gln Gly Gly Ala Leu Ala Gly Thr Gly Ala His Ser Tyr Ser Lys
       370                 375                 380
   Gly Asn Ala Thr Gly Gly Ser Glu Glu Ala Ala Lys Phe Lys Lys Ala
   385                 390                 395                 400
   Val
<210> 12
   <211> 27
   <212> DNA
   <213> artificial
<220>
   <223> PCR primer
<400> 12
   accaaggtcg tggaggccga ctttagc 27
<210> 13
   <211> 27
   <212> DNA
   <213> artificial
<220>
   <223> PCR primer
<400> 13
   gctaaagtcg gcctccacga ccttggt 27
<210> 14
   <211> 27
   <212> DNA
   <213> artificial
<220>
   <223> PCR primer
<400> 14
   accaaggtcg tggacgccga ctttagc 27
<210> 15
   <211> 27
   <212> DNA
   <213> artificial
<220>
   <223> PCR primer
<400> 15
   gctaaagtcg gcgtccacga ccttggt 27
<210> 16
   <211> 27
   <212> DNA
   <213> artificial
<220>
   <223> PCR primer
<400> 16
   accaaggtcg tgacggccga ctttagc 27
<210> 17
   <211> 27
   <212> DNA
   <213> artificial
<220>
   <223> PCR primer
<400> 17
   gctaaagtcg gccgtcacga ccttggt 27
<210> 18
   <211> 27
   <212> DNA
   <213> artificial
<220>
   <223> PCR primer
<400> 18
   accaaggtcg tgtgggccga ctttagc 27
<210> 19
   <211> 27
   <212> DNA
   <213> artificial
<220>
   <223> PCR primer
<400> 19
   gctaaagtcg gcccacacga ccttggt 27
<210> 20
   <211> 27
   <212> DNA
   <213> artificial
<220>
   <223> PCR primer
<400> 20
   accaaggtcg tgtacgccga ctttagc 27
<210> 21
   <211> 27
   <212> DNA
   <213> artificial
<220>
   <223> PCR primer
<400> 21
   gctaaagtcg gcgtacacga ccttggt 27
<210> 22
   <211> 27
   <212> DNA
   <213> artificial
<220>
   <223> PCR primer
<400> 22
   accaaggtcg tgttcgccga ctttagc 27
<210> 23
   <211> 27
   <212> DNA
   <213> artificial
<220>
   <223> PCR primer
<400> 23
   gctaaagtcg gcgaacacga ccttggt 27
<210> 24
   <211> 27
   <212> DNA
   <213> artificial
<220>
   <223> PCR primer
<400> 24
   accaaggtcg tgtgcgccga ctttagc 27
<210> 25
   <211> 27
   <212> DNA
   <213> artificial
<220>
   <223> PCR primer
<400> 25
   gctaaagtcg gcgcacacga ccttggt 27
<210> 26
   <211> 27
   <212> DNA
   <213> artificial
<220>
   <223> PCR primer
<400> 26
   accaaggtcg tgaacgccga ctttagc 27
<210> 27
   <211> 27
   <212> DNA
   <213> artificial
<220>
   <223> PCR primer
<400> 27
   gctaaagtcg gcgttcacga ccttggt 27
<210> 28
   <211> 27
   <212> DNA
   <213> artificial
<220>
   <223> PCR primer
<400> 28
   accaaggtcg tgcaggccga ctttagc 27
<210> 29
   <211> 27
   <212> DNA
   <213> artificial
<220>
   <223> PCR primer
<400> 29
   gctaaagtcg gcctgcacga ccttggt 27
<210> 30
   <211> 27
   <212> DNA
   <213> artificial
<220>
   <223> PCR primer
<400> 30
   accaaggtcg tgcacgccga ctttagc 27
<210> 31
   <211> 27
   <212> DNA
   <213> artificial
<220>
   <223> PCR primer
<400> 31
   gctaaagtcg gcgtgcacga ccttggt 27
<210> 32
   <211> 27
   <212> DNA
   <213> artificial
<220>
   <223> PCR primer
<400> 32
   accaaggtcg tgaaggccga ctttagc 27
<210> 33
   <211> 27
   <212> DNA
   <213> artificial
<220>
   <223> PCR primer
<400> 33
   gctaaagtcg gccttcacga ccttggt 27
<210> 34
   <211> 27
   <212> DNA
   <213> artificial
<220>
   <223> PCR primer
<400> 34
   accaaggtcg tgatcgccga ctttagc 27
<210> 35
   <211> 27
   <212> DNA
   <213> artificial
<220>
   <223> PCR primer
<400> 35
   gctaaagtcg gcgatcacga ccttggt 27
<210> 36
   <211> 27
   <212> DNA
   <213> artificial
<220>
   <223> PCR primer
<400> 36
   accaaggtcg tgggcgccga ctttagc 27
<210> 37
   <211> 27
   <212> DNA
   <213> artificial
<220>
   <223> PCR primer
<400> 37
   gctaaagtcg gcgcccacga ccttggt 27
<210> 38
   <211> 27
   <212> DNA
   <213> artificial
<220>
   <223> PCR primer
<400> 38
   accaaggtcg tggccgccga ctttagc 27
<210> 39
   <211> 27
   <212> DNA
   <213> artificial
<220>
   <223> PCR primer
<400> 39
   gctaaagtcg gcggccacga ccttggt 27
<210> 40
   <211> 27
   <212> DNA
   <213> artificial
<220>
   <223> PCR primer
<400> 40
   accaaggtcg tggtcgccga ctttagc 27
<210> 41
   <211> 27
   <212> DNA
   <213> artificial
<220>
   <223> PCR primer
<400> 41
   gctaaagtcg gcgaccacga ccttggt 27
<210> 42
   <211> 27
   <212> DNA
   <213> artificial
<220>
   <223> PCR primer
<400> 42
   accaaggtcg tgctcgccga ctttagc 27
<210> 43
   <211> 27
   <212> DNA
   <213> artificial
<220>
   <223> PCR primer
<400> 43
   gctaaagtcg gcgagcacga ccttggt 27
<210> 44
   <211> 27
   <212> DNA
   <213> artificial
<220>
   <223> PCR primer
<400> 44
   accaaggtcg tgagcgccga ctttagc 27
<210> 45
   <211> 27
   <212> DNA
   <213> artificial
<220>
   <223> PCR primer
<400> 45
   gctaaagtcg gcgctcacga ccttggt 27
<210> 46
   <211> 27
   <212> DNA
   <213> artificial
<220>
   <223> PCR primer
<400> 46
   atcctgggcg cgttcgaggg cgtcggc 27
<210> 47
   <211> 27
   <212> DNA
   <213> artificial
<220>
   <223> PCR primer
<400> 47
   gccgacgccc tcgaacgcgc ccaggat 27
<210> 48
   <211> 27
   <212> DNA
   <213> artificial
<220>
   <223> PCR primer
<400> 48
   atcctgggcg cgatcgaggg cgtcggc 27
<210> 49
   <211> 27
   <212> DNA
   <213> artificial
<220>
   <223> PCR primer
<400> 49
   gccgacgccc tcgatcgcgc ccaggat 27
<210> 50
   <211> 27
   <212> DNA
   <213> Artificial Sequence
<220>
   <223> PCR primer
<400> 50
   atcctgggcg cggccgaggg cgtcggc 27
<210> 51
   <211> 27
   <212> DNA
   <213> Artificial Sequence
<220>
   <223> PCR Primer
<400> 51
   gccgacgccc tcggccgcgc ccaggat 27
<210> 52
   <211> 23
   <212> DNA
   <213> Artificial Sequence
<220>
   <223> PCR Primer
<400> 52
   tcctgggcgc gagcgagggc gtc 23
<210> 53
   <211> 23
   <212> DNA
   <213> Artificial Sequence
<220>
   <223> PCR Primer
<400> 53
   gacgccctcg ctcgcgccca gga 23
<210> 54
   <211> 27
   <212> DNA
   <213> Artificial Sequence
<220>
   <223> PCR Primer
<400> 54
   gtcgtcatgg tcagccgtcg cgaggag 27
<210> 55
   <211> 27
   <212> DNA
   <213> Artificial Sequence
<220>
   <223> PCR Primer
<400> 55
   ctcctcgcga cggctgacca tgacgac 27
<210> 56
   <211> 27
   <212> DNA
   <213> Artificial Sequence
<220>
   <223> PCR Primer
<400> 56
   gtcgtcatgg tcgtccgtcg cgaggag 27
<210> 57
   <211> 27
   <212> DNA
   <213> Artificial Sequence
<220>
   <223> PCR primer
<400> 57
   ctcctcgcga cggacgacca tgacgac 27
<210> 58
   <211> 27
   <212> DNA
   <213> Artificial Sequence
<220>
   <223> PCR Primer
<400> 58
   gtcgtcatgg tcatccgtcg cgaggag 27
<210> 59
   <211> 27
   <212> DNA
   <213> Artificial Sequence
<220>
   <223> PCR Primer
<400> 59
   ctcctcgcga cggatgacca tgacgac 27
<210> 60
   <211> 27
   <212> DNA
   <213> Artificial Sequence
<220>
   <223> PCR Primer
<400> 60
   gtcgtcatgg tctgccgtcg cgaggag 27
<210> 61
   <211> 27
   <212> DNA
   <213> Artificial Sequence
<220>
   <223> PCR Primer
<400> 61
   ctcctcgcga cggcagacca tgacgac 27
<210> 62
   <211> 27
   <212> DNA
   <213> Artificial Sequence
<220>
   <223> PCR Primer
<400> 62
   gtcgtcatgg tcaagcgtcg cgaggag 27
<210> 63
   <211> 27
   <212> DNA
   <213> Artificial Sequence
<220>
   <223> PCR Primer
<400> 63
   ctcctcgcga cgcttgacca tgacgac 27
<210> 64
   <211> 27
   <212> DNA
   <213> Artificial Sequence
<220>
   <223> PCR Primer
<400> 64
   gtcgtcatgg tctaccgtcg cgaggag 27
<210> 65
   <211> 27
   <212> DNA
   <213> Artificial Sequence
<220>
   <223> PCR Primer
<400> 65
   ctcctcgcga cggtagacca tgacgac 27
<210> 66
   <211> 27
   <212> DNA
   <213> Artificial Sequence
<220>
   <223> PCR Primer
<400> 66
   gtcgtcatgg tcttccgtcg cgaggag 27
<210> 67
   <211> 27
   <212> DNA
   <213> Artificial Sequence
<220>
   <223> PCR Primer
<400> 67
   ctcctcgcga cggaagacca tgacgac 27
<210> 68
   <211> 27
   <212> DNA
   <213> Artificial Sequence
<220>
   <223> PCR Primer
<400> 68
   gtcgtcatgg tccgccgtcg cgaggag 27
<210> 69
   <211> 27
   <212> DNA
   <213> Artificial Sequence
<220>
   <223> PCR Primer
<400> 69
   ctcctcgcga cggcggacca tgacgac 27